# EUROPEAN PATENT SPECIFICATION

(11) **EP 4 076 444 B1**
(45) Date of publication and mention of the grant of the patent: **25.11.2026**
(21) Application number: 20904204.3
(22) Date of filing: 15.12.2020
(51) Int. Cl.: C07D 519/00, C07D 403/06, C07D 403/12, C07D 413/06, C07D 417/06, C07D 417/12, C07D 471/04, C07D 487/04, C07D 231/56, A61P 31/00, A61K 31/427, A61K 31/416

(54) **INDAZOLE DERIVATIVES AND METHODS OF USE THEREOF FOR THE TREATMENT OF HERPES VIRUSES**
INDAZOLDERIVATE UND VERFAHREN ZUR VERWENDUNG DAVON FÜR DIE BEHANDLUNG VON HERPESVIREN
DÉRIVÉS D'INDAZOLE ET LEURS MÉTHODES D'UTILISATION DANS LE TRAITEMENT DE L'HERPÈS VIRUS

(30) Priority: 18.12.2019 US 201962949897 P
(43) Date of publication of application: 26.10.2022
(73) Proprietor: Merck Sharp & Dohme LLC, Rahway, New Jersey 07065 (US)
(72) Inventor: COOKE, Andrew John, Broughty Ferry Scotland DD5 3JA (GB); COX, Christopher, D., North Wales, Pennsylvania 19454-2505 (US); EDMONDSON, Scott, D., Concord, Massachusetts 01742 (US); JAYNE, Charles Lee, Kenilworth, New Jersey 07033 (US); NAIR, Anilkumar, Kenilworth, New Jersey 07033 (US); SCHUBERT, Jeffrey, W., APoint, Pennsylvania 19486 (US); SKUDLAREK, Jason, W., West Point, Pennsylvania 19486 (US); TELLERS, David, M., West Point, Pennsylvania 19486 (US); YANG, De-Yi, Rahway, New Jersey 07065-0907 (US)
(74) Representative: Merck Sharp & Dohme LLC
(86) International application number: PCT/US2020/065020
(87) International publication number: WO 2021/126804

(56) References cited:
- WO-A1-2008/003396
- WO-A1-2009/057733
- WO-A1-2011/050245
- WO-A1-2012/031197
- WO-A1-93/18765
- CN-A- 103 626 705
- CN-A- 105 753 841
- US-A1- 2004 235 931
- US-A1- 2011 034 497
- US-A1- 2016 168 090
- SINDHUJA ELANGOVAN ET AL: "Direct Synthesis of Amides from Coupling of Alcohols and Amines Catalyzed by Ruthenium(II) Thiocarboxamide Complexes under Aerobic Conditions", ORGANOMETALLICS, vol. 33, no. 16, 13 August 2014 (2014-08-13), pages 4269 - 4278, XP055838547, ISSN: 0276-7333, DOI: 10.1021/om500556b
- VALERIJA KARALUKA ET AL: "B(OCH 2 CF 3 ) 3 -mediated direct amidation of pharmaceutically relevant building blocks in cyclopentyl methyl ether", ORGANIC & BIOMOLECULAR CHEMISTRY, vol. 13, no. 44, September 2015 (2015-09-01), pages 10888 - 10894, XP055423540, ISSN: 1477-0520, DOI: 10.1039/C5OB01801C
- SARWAT CHOWDHURY ET AL: "Discovery and optimization of indoles and 7-azaindoles as Rho kinase (ROCK) inhibitors (part-I)", BIOORGANIC & MEDICINAL CHEMISTRY LETTERS, ELSEVIER, AMSTERDAM NL, vol. 21, no. 23, 20 September 2011 (2011-09-20), pages 7107 - 7112, XP028334849, ISSN: 0960-894X, [retrieved on 20110924], DOI: 10.1016/J.BMCL.2011.09.083
- HAMPTON SESSIONS E ET AL: "Discovery and optimization of indole and 7-azaindoles as Rho kinase (ROCK) inhibitors (Part-II)", BIOORGANIC & MEDICINAL CHEMISTRY LETTERS, vol. 21, no. 23, October 2011 (2011-10-01), pages 7113 - 7118, XP028334850, ISSN: 0960-894X, [retrieved on 20111001], DOI: 10.1016/J.BMCL.2011.09.084
- CATHERINE JOHNSON M ET AL: "Novel isoquinolone PDK1 inhibitors discovered through fragment-based lead discovery", JOURNAL OF COMPUTER-AIDED MOLECULAR DESIGN, KLUWER ACADEMIC PUBLISHERS, DO, vol. 25, no. 7, 22 July 2011 (2011-07-22), pages 689 - 698, XP019939182, ISSN: 1573-4951, DOI: 10.1007/S10822-011-9456-7
- DEL POZO CARLOS ET AL: "Amide Bond Formation with a New Fluorous Carbodiimide:? Separation by Reverse Fluorous Solid-Phase Extraction", ORGANIC LETTERS, vol. 9, no. 21, 21 September 2007 (2007-09-21), US, pages 4167 - 4170, XP093088484, ISSN: 1523-7060, DOI: 10.1021/ol701631m
- DATABASE REGISTRY [online] CHEMICAL ABSTRACTS SERVICE, COLUMBUS, OHIO, US; 29 June 2015 (2015-06-29), XP002810564, retrieved from STN Database accession no. 1790822-11-5
- DATABASE REGISTRY [online] CHEMICAL ABSTRACTS SERVICE, COLUMBUS, OHIO, US; 28 June 2015 (2015-06-28), XP002810565, Database accession no. 1790524-36-5
- DATABASE REGISTRY [online] CHEMICAL ABSTRACTS SERVICE, COLUMBUS, OHIO, US; 24 June 2015 (2015-06-24), XP002810566, Database accession no. 1788004-10-3
- HUAI-QIANG JU ET AL: "Synthesis and in vitro anti-HSV-1 activity of a novel Hsp90 inhibitor BJ-B11", BIOORGANIC & MEDICINAL CHEMISTRY LETTERS, ELSEVIER, AMSTERDAM NL, vol. 21, no. 6, 21 January 2011 (2011-01-21), pages 1675 - 1677, XP028169552, ISSN: 0960-894X, [retrieved on 20110127], DOI: 10.1016/J.BMCL.2011.01.098
- DATABASE REGISTRY [online] Chemical Abstract Service, Columbus, Ohio, US; 19 May 2019 (2019-05-19), XP093229400, retrieved from https://stn.org/stn/# Database accession no. 2310847-24-4
- DATABASE REGISTRY [online] Chemical Abstract Service, Columbus, Ohio, US; 13 September 2009 (2009-09-13), XP093229404, retrieved from https://stn.org/stn/# Database accession no. 1183469-19-3
- DATABASE REGISTRY [online] Chemical Abstract Service, Columbus, Ohio, US; 26 October 2005 (2005-10-26), XP093229405, retrieved from https://stn.org/stn/# Database accession no. 866132-48-1
- DATABASE REGISTRY [online] Chemical Abstract Service, Columbus, Ohio, US; 26 August 2004 (2004-08-26), XP093229406, retrieved from https://stn.org/stn/# Database accession no. 732997-51-2
- SINDHUJA ELANGOVAN, RAMESH RENGAN, BALAJI SUNDARRAMAN, LIU YU: "Direct Synthesis of Amides from Coupling of Alcohols and Amines Catalyzed by Ruthenium(II) Thiocarboxamide Complexes under Aerobic Conditions", ORGANOMETALLICS, vol. 33, no. 16, 25 August 2014 (2014-08-25), pages 4269 - 4278, XP055838547, ISSN: 0276-7333, DOI: 10.1021/om500556b
- DATABASE PUBCHEM COMPOUND 5 November 2019 (2019-11-05), ANONYMOUS: "1H-INDOLE-3-CARBOXYLIC ACID BENZYLAMIDE", XP055838551, retrieved from PUBCHEM Database accession no. 347769999
- CHOWDHURY ET AL.: "Discovery and optimization of indoles and 7-azaindoles as Rho kinase (ROCK) inhibitors (part-I", BIOORGANIC AND MEDICINAL CHEMISTRY LETTERS, vol. 21, 2011, pages 7107 - 7112, XP028334849, DOI: 10.1016/j.bmcl.2011.09.083

## Description

### FIELD OF THE INVENTION

The present invention relates to novel Indazole Derivatives, compositions comprising at least one Indazole Derivative, and methods of using the Indazole Derivatives for treating or preventing herpesvirus infection in a patient.

### BACKGROUND OF THE INVENTION

Human herpes viruses (*Herpesviridae*) are responsible for causing a wide variety of diseases in humans. Infection with herpes viruses can occur early in life and by adulthood over 95% of the population is infected by at least one herpes virus. These viruses establish a persistent life-long infection through viral latency in neuronal, lymphoid, or myeloid cells. Recurrent episodes of herpes virus disease can be triggered by numerous stimuli, including concurrent viral infections, stress, fatigue, allergies, pregnancy, sunlight or fever. Herpes virus infection in immune competent individuals generally causes mild self-limiting disease, such as: oral (HSV-1) and genital (HSV-2) ulcers, chicken pox (VZV), flu-like syndrome (CMV) and mononucleosis (EBV). In immunocompromised individuals however, primary infection with, or reactivation of an existing herpes virus infection is a major cause of disease and death. Key at-risk immunocompromised populations include patients undergoing solid organ or stem cell transplants, individuals with HIV/AIDS, and ICU patients.

Herpesviridae comprise a diverse family of double-stranded DNA viruses that are classified into three subfamilies (*i.e.,* α, β, and γ) based upon biological characteristics such as cell tropism, diseases caused, viral life-cycle, and site of viral persistence and latency. The family consists of eight members: Herpes Simplex Virus type 1 and 2 (HSV-1, HSV-2), Varicella Zoster Virus (VZV), Epstein-Barr virus (EBV), Cytomegalovirus (CMV), and human herpes viruses 6-8 (HHV6-8).

A-herpes viruses include herpes simplex virus types 1 and 2 (HSV1 and HSV2) and varicella-zoster virus (VZV). HSV1 causes orofacial lesions, commonly known as fever blisters or cold sores. Approximately 30% of the United States population suffers from recurrent episodes of HSV1. HSV2, which is less common than HSV1, causes genital lesions. Primary infection with VZV causes varicella, commonly known as chicken pox. Reactivation of latent VZV manifests as herpes zoster or shingles. Cytomegalovirus (CMV) is a prototypical β herpes virus. Seroprevalance to CMV in the adult population is ~60%, but certain endemic areas of the world have rates closer to 100%. CMV represents the leading viral cause of morbidity and mortality in at-risk immunocompromised patients. EBV, a γ herpes virus, causes infectious mononucleosis and is responsible for lymphoid cancers such as Burkitt's and Hodgkin's lymphoma.

Presently, there is no cure for herpes. Medicines have been developed that can prevent or shorten outbreaks, but there is a need for improved therapies for treating herpes virus infection and inhibiting viral replication. The current standard of care for immunocompromised patients at risk for herpes virus disease is pre-emptive treatment with high-dose nucleoside/nucleotide analog drugs such as acyclovir, (val)ganciclovir, and cidofovir, all of which target the viral DNA polymerase. In general, current treatments are virus specific (not broad spectrum) and in the case of (val)ganciclovir and cidofovir cannot be administered prophylactically due to dose-related toxicities including bone marrow suppression and renal toxicity. Although efficacious in many settings, the current nucleos(t)ide drugs are also limited by drug-resistant viral variants and existing cross-resistant variants which may lead to treatment failure. Therefore, there is an urgent medical need for improved, well-tolerated anti-herpes agents.

WO 2012/031197 A1 relates to compounds and compositions for the inhibition of NAMPT.

HUAI-QIANG JU ET AL, "Synthesis and in vitro anti-HSV-1 activity of a novel Hsp90 inhibitor BJ-B11", BIOORGANIC & MEDICINAL CHEMISTRY LETTERS, relates to an Hsp90 inhibitor.

US 2004/235931 A1 relates inhibition of herpes virus replication.

US 2011/034497 A1 relates to inhibitors of the WNT signal pathway.

CN 105 753 841 A relates to N-indazole substituted thiourea derivatives.

CN 103 626 705 A relates to 1-(3-benzoylaminobenzyl)-1H-indazole-3-carboxamide compounds.

WO 93/18765 A1 relates to indole derivatives with antiviral activity.

WO 2008/003396 A1 relates to indazole derivatives for treating hsp90-induced diseases.

### SUMMARY OF THE INVENTION

The present invention provides Compounds of Formula 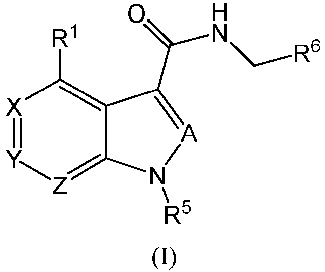 or a pharmaceutically acceptable salt thereof, wherein:
A is -N- or -C(R⁸)-;
X is -N- or -C(R²)-;
Y is -C(R³)-;
Z is -N- or -C(R⁴)-, such that only one of X and Z can be -N-;
R¹ is selected from H or methyl;
R² is selected from H, 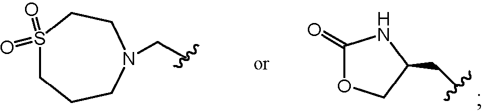
R³ is selected from 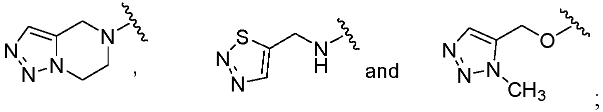
R⁴ is selected from H, -Cl or -CN;
R⁵ is selected from H, C₃-C₇ cycloalkyl, -OH, 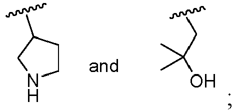
R⁶ is selected from 4-chlorophenyl and 4-cyanophenyl; and
R⁸ is H or C₁-C₆ alkyl.

The Compounds of Formula (I) (also referred to herein as the "Indazole Derivatives"), and pharmaceutically acceptable salts thereof can be useful, for example, for inhibiting herpesvirus viral replication or activity, and for treating or preventing herpesvirus infection in a patient. Without being bound by any specific theory, it is believed that the Indazole Derivatives inhibit herpesvirus viral replication by inhibiting herpesvirus polymerase.

Accordingly, the present invention provides Indazole Derivative for use in methods for treating or preventing herpesvirus infection in a patient.

The details of the invention are set forth in the accompanying detailed description below.

### DETAILED DESCRIPTION OF THE INVENTION

The present invention relates to novel Indazole Derivatives, compositions comprising at least one Indazole Derivative, and methods of using the Indazole Derivatives for treating or preventing herpesvirus infection in a patient.

In the following pages, any reference to methods of treatment by therapy should be understood as referring to the compounds or compositions described therein for use in such methods of treatment.

### Definitions and Abbreviations

The terms used herein have their ordinary meaning and the meaning of such terms is independent at each occurrence thereof. That notwithstanding and except where stated otherwise, the following definitions apply throughout the specification and claims. Chemical names, common names, and chemical structures may be used interchangeably to describe the same structure. If a chemical compound is referred to using both a chemical structure and a chemical name and an ambiguity exists between the structure and the name, the structure predominates. These definitions apply regardless of whether a term is used by itself or in combination with other terms, unless otherwise indicated. Hence, the definition of "alkyl" applies to "alkyl" as well as the "alkyl" portions of "hydroxyalkyl," "haloalkyl," "-O-alkyl," etc...

As used herein, and throughout this disclosure, the following terms, unless otherwise indicated, shall be understood to have the following meanings:
A "patient" is a human or non-human mammal. In one embodiment, a patient is a human.

The term "effective amount" as used herein, refers to an amount of Indazole Derivative and/or an additional therapeutic agent, or a composition thereof that is effective in producing the desired therapeutic, ameliorative, inhibitory or preventative effect when administered to a patient suffering from a viral infection or virus-related disorder. In the combination therapies of the present invention, an effective amount can refer to each individual agent or to the combination as a whole, wherein the amounts of all agents administered are together effective, but wherein the component agent of the combination may not be present individually in an effective amount.

The term "preventing," as used herein with respect to an herpesvirus viral infection or herpesvirus-virus related disorder, refers to reducing the likelihood of herpesvirus infection.

The term "alkyl," as used herein, refers to an aliphatic hydrocarbon group having one of its hydrogen atoms replaced with a bond. An alkyl group may be straight or branched and contain from about 1 to about 20 carbon atoms. In one embodiment, an alkyl group contains from about 1 to about 12 carbon atoms. In different embodiments, an alkyl group contains from 1 to 6 carbon atoms (C₁-C₆ alkyl) or from about 1 to about 4 carbon atoms (C₁-C₄ alkyl). Non-limiting examples of alkyl groups include methyl, ethyl, n-propyl, isopropyl, n-butyl, sec-butyl, isobutyl, tert-butyl, n-pentyl, neopentyl, isopentyl, n-hexyl, isohexyl and neohexyl. An alkyl group may be unsubstituted or substituted by one or more substituents which may be the same or different, each substituent being independently selected from the group consisting of halo, alkenyl, alkynyl, aryl, cycloalkyl, cyano, hydroxy, -O-alkyl, -O-aryl, -alkylene-O-alkyl, alkylthio, -NH₂, -NH(alkyl), -N(alkyl)₂, NH(cycloalkyl), -O-C(O)-alkyl, -O-C(O)-aryl, -O-C(O)-cycloalkyl, -C(O)OH and -C(O)O-alkyl. In one embodiment, an alkyl group is linear. In another embodiment, an alkyl group is branched. Unless otherwise indicated, an alkyl group is unsubstituted.

The term "alkenyl," as used herein, refers to an aliphatic hydrocarbon group containing at least one carbon-carbon double bond and having one of its hydrogen atoms replaced with a bond. An alkenyl group may be straight or branched and contain from about 2 to about 15 carbon atoms. In one embodiment, an alkenyl group contains from about 2 to about 12 carbon atoms. In another embodiment, an alkenyl group contains from about 2 to about 6 carbon atoms. Non-limiting examples of alkenyl groups include ethenyl, propenyl, n-butenyl, 3-methylbut-2-enyl, n-pentenyl, octenyl and decenyl. An alkenyl group may be unsubstituted or substituted by one or more substituents which may be the same or different, each substituent being independently selected from the group consisting of halo, alkenyl, alkynyl, aryl, cycloalkyl, cyano, hydroxy, - O-alkyl, -O-aryl, -alkylene-O-alkyl, alkylthio, -NH₂, -NH(alkyl), -N(alkyl)₂, -NH(cycloalkyl), - O-C(O)-alkyl, -O-C(O)-aryl, -O-C(O)-cycloalkyl, -C(O)OH and -C(O)O-alkyl. The term "C₂-C₆ alkenyl" refers to an alkenyl group having from 2 to 6 carbon atoms. Unless otherwise indicated, an alkenyl group is unsubstituted.

The term "alkynyl," as used herein, refers to an aliphatic hydrocarbon group containing at least one carbon-carbon triple bond and having one of its hydrogen atoms replaced with a bond. An alkynyl group may be straight or branched and contain from about 2 to about 15 carbon atoms. In one embodiment, an alkynyl group contains from about 2 to about 12 carbon atoms. In another embodiment, an alkynyl group contains from about 2 to about 6 carbon atoms. Non-limiting examples of alkynyl groups include ethynyl, propynyl, 2-butynyl and 3-methylbutynyl. An alkynyl group may be unsubstituted or substituted by one or more substituents which may be the same or different, each substituent being independently selected from the group consisting of halo, alkenyl, alkynyl, aryl, cycloalkyl, cyano, hydroxy, -O-alkyl, -O-aryl, -alkylene-O-alkyl, alkylthio, -NH₂, -NH(alkyl), -N(alkyl)₂, -NH(cycloalkyl), -O-C(O)-alkyl, -O-C(O)-aryl, -O-C(O)-cycloalkyl, -C(O)OH and -C(O)O-alkyl. The term "C₂-C₆ alkynyl" refers to an alkynyl group having from 2 to 6 carbon atoms. Unless otherwise indicated, an alkynyl group is unsubstituted.

The term "alkylene," as used herein, refers to an alkyl group, as defined above, wherein one of the alkyl group's hydrogen atoms has been replaced with a bond. Non-limiting examples of alkylene groups include -CH₂-, -CH₂CH₂-, -CH₂CH₂CH₂-, -CH₂CH₂CH₂CH₂-, - CH(CH₃)CH₂CH₂-, -CH(CH₃)- and -CH₂CH(CH₃)CH₂-. In one embodiment, an alkylene group has from 1 to about 6 carbon atoms. In another embodiment, an alkylene group is branched. In another embodiment, an alkylene group is linear. In one embodiment, an alkylene group is - CH₂-. The term "C₁-C₆ alkylene" refers to an alkylene group having from 1 to 6 carbon atoms.

The term "aryl," as used herein, refers to an aromatic monocyclic or multicyclic ring system comprising from about 6 to about 14 carbon atoms. In one embodiment, an aryl group contains from about 6 to about 10 carbon atoms. An aryl group can be optionally substituted with one or more "ring system substituents" which may be the same or different, and are as defined herein below. In one embodiment, an aryl group can be optionally fused to a cycloalkyl or cycloalkanoyl group. Non-limiting examples of aryl groups include phenyl and naphthyl. In one embodiment, an aryl group is phenyl. In another embodiment, an aryl group is napthalene. Unless otherwise indicated, an alkyl group is unsubstituted.

The term "cycloalkyl," as used herein, refers to a non-aromatic mono- or multicyclic ring system comprising from about 3 to about 10 ring carbon atoms. In one embodiment, a cycloalkyl contains from about 5 to about 10 ring carbon atoms. In another embodiment, a cycloalkyl contains from about 3 to about 7 ring atoms. In another embodiment, a cycloalkyl contains from about 5 to about 6 ring atoms. Non-limiting examples of monocyclic cycloalkyls include cyclopropyl, cyclobutyl, cyclopentyl, cyclohexyl, cycloheptyl and cyclooctyl. Non-limiting examples of multicyclic cycloalkyls include 1-decalinyl, norbornyl and adamantyl. A cycloalkyl group can be optionally substituted with one or more "ring system substituents" which may be the same or different, and are as defined herein below. Unless otherwise indicated, an alkynyl group is unsubstituted. In one embodiment, a cycloalkyl group is unsubstituted. The term "3 to 7-membered cycloalkyl" refers to a cycloalkyl group having from 3 to 7 ring carbon atoms. A ring carbon atom of a cycloalkyl group may be functionalized as a carbonyl group. An illustrative example of such a cycloalkyl group (also referred to herein as a "cycloalkanoyl" group) includes, but is not limited to, cyclobutanoyl:

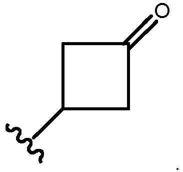

The term "cycloalkenyl," as used herein, refers to a non-aromatic mono- or multicyclic ring system comprising from about 4 to about 10 ring carbon atoms and containing at least one endocyclic double bond. In one embodiment, a cycloalkenyl contains from about 4 to about 7 ring carbon atoms. In another embodiment, a cycloalkenyl contains 5 or 6 ring atoms. Non-limiting examples of monocyclic cycloalkenyls include cyclopentenyl, cyclohexenyl, cyclohepta-1,3-dienyl. A cycloalkenyl group can be optionally substituted with one or more "ring system substituents" which may be the same or different, and are as defined herein below. A ring carbon atom of a cycloalkyl group may be functionalized as a carbonyl group. In one embodiment, a cycloalkenyl group is cyclopentenyl. In another embodiment, a cycloalkenyl group is cyclohexenyl. The term "4 to 6-membered cycloalkenyl" refers to a cycloalkenyl group having from 4 to 6 ring carbon atoms.

The term "halo," as used herein, means -F, -Cl, -Br or -I.

The term "haloalkyl," as used herein, refers to an alkyl group as defined above, wherein one or more of the alkyl group's hydrogen atoms has been replaced with a halogen. In one embodiment, a haloalkyl group has from 1 to 6 carbon atoms. In another embodiment, a haloalkyl group is substituted with from 1 to 3 F atoms. Non-limiting examples of haloalkyl groups include -CH₂F, -CHF₂, -CF₃, -CH₂Cl and -CCl₃.

The term "C₁-C₆ haloalkyl" refers to a haloalkyl group having from 1 to 6 carbon atoms.

The term "hydroxyalkyl," as used herein, refers to an alkyl group as defined above, wherein one or more of the alkyl group's hydrogen atoms has been replaced with an -OH group. In one embodiment, a hydroxyalkyl group has from 1 to 6 carbon atoms. Non-limiting examples of hydroxyalkyl groups include -CH₂OH, -CH₂CH₂OH, -CH₂CH₂CH₂OH and - CH₂CH(OH)CH₃. The term "C₁-C₆ hydroxyalkyl" refers to a hydroxyalkyl group having from 1 to 6 carbon atoms.

The term "heteroaryl," as used herein, refers to an aromatic monocyclic or multicyclic ring system comprising about 5 to about 14 ring atoms, wherein from 1 to 4 of the ring atoms is independently O, N or S and the remaining ring atoms are carbon atoms. In one embodiment, a heteroaryl group has 5 to 10 ring atoms. In another embodiment, a heteroaryl group is monocyclic and has 5 or 6 ring atoms. In another embodiment, a heteroaryl group is bicyclic and had 9 or 10 ring atoms. A heteroaryl group can be optionally substituted by one or more "ring system substituents" which may be the same or different, and are as defined herein below. A heteroaryl group is joined via a ring carbon atom, and any nitrogen atom of a heteroaryl can be optionally oxidized to the corresponding N-oxide. The term "heteroaryl" also encompasses a heteroaryl group, as defined above, which is fused to a benzene ring. Non-limiting examples of heteroaryls include pyridyl, pyrazinyl, furanyl, thienyl, pyrimidinyl, pyridone (including N-substituted pyridones), isoxazolyl, isothiazolyl, oxazolyl, oxadiazolyl, thiazolyl, pyrazolyl, furazanyl, pyrrolyl, triazolyl, 1,2,4-thiadiazolyl, pyrazinyl, pyridazinyl, quinoxalinyl, phthalazinyl, oxindolyl, imidazo[1,2-a]pyridinyl, imidazo[2,1-b]thiazolyl, benzofurazanyl, indolyl, azaindolyl, benzimidazolyl, benzothienyl, quinolinyl, imidazolyl, benzimidazolyl, thienopyridyl, quinazolinyl, thienopyrimidyl, pyrrolopyridyl, imidazopyridyl, isoquinolinyl, benzoazaindolyl, 1,2,4-triazinyl, benzothiazolyl, and all isomeric forms thereof. The term "heteroaryl" also refers to partially saturated heteroaryl moieties such as, for example, tetrahydroisoquinolyl, tetrahydroquinolyl. In one embodiment, a heteroaryl group is a 5-membered heteroaryl. In another embodiment, a heteroaryl group is a 6-membered heteroaryl. In another embodiment, a "9- or 10-membered bicyclic heteroaryl" group comprises a 5- to 6-membered heterocycloalkyl group fused to a benzene ring, such as:

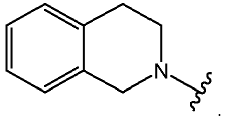

In still another embodiment, a "9- or 10-membered bicyclic heteroaryl" group comprises a 5- to 6-membered heteroaryl group fused to a cycloalkyl ring or a heterocycloalkyl ring, such as:

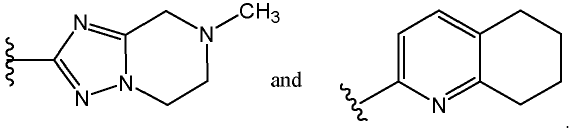

The term "heteroarylene," as used herein, refers to a bivalent group derived from an heteroaryl group, as defined above, by removal of a hydrogen atom from a ring carbon or ring heteroatom of a heteroaryl group. A heteroarylene group can be derived from a monocyclic or multicyclic ring system comprising about 5 to about 14 ring atoms, wherein from 1 to 4 of the ring atoms are each independently O, N or S and the remaining ring atoms are carbon atoms. A heteroarylene group can be optionally substituted by one or more "ring system substituents" which may be the same or different, and are as defined herein below. A heteroarylene group is joined via a ring carbon atom or by a nitrogen atom with an open valence, and any nitrogen atom of a heteroarylene can be optionally oxidized to the corresponding N-oxide. The term "heteroarylene" also encompasses a heteroarylene group, as defined above, which is fused to a benzene ring. Non-limiting examples of heteroarylenes include pyridylene, pyrazinylene, furanylene, thienylene, pyrimidinylene, pyridonylene (including those derived from N-substituted pyridonyls), isoxazolylene, isothiazolylene, oxazolylene, oxadiazolylene, thiazolylene, pyrazolylene, thiophenylene, furazanylene, pyrrolylene, triazolylene, 1,2,4-thiadiazolylene, pyrazinylene, pyridazinylene, quinoxalinylene, phthalazinylene, oxindolylene, imidazo[1,2-a]pyridinylene, imidazo[2,1-b]thiazolylene, benzofurazanylene, indolylene, azaindolylene, benzimidazolylene, benzothienylene, quinolinylene, imidazolylene, benzimidazolylene, thienopyridylene, quinazolinylene, thienopyrimidylene, pyrrolopyridylene, imidazopyridylene, isoquinolinylene, benzoazaindolylene, 1,2,4-triazinylene, benzothiazolylene, and all isomeric forms thereof. The term "heteroarylene" also refers to partially saturated heteroarylene moieties such as, for example, tetrahydroisoquinolylene, tetrahydroquinolylene. A heteroarylene group is divalent and unless specified ohterwise, either available bond on a heteroarylene ring can connect to either group flanking the heteroarylene group. For example, the group "A-heteroarylene-B," wherein the heteroarylene group is: 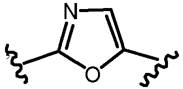 is understood to represent both:

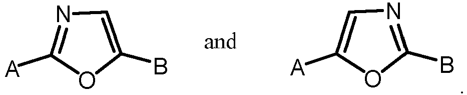

In one embodiment, a heteroarylene group is a monocyclic heteroarylene group or a bicyclic heteroarylene group. In another embodiment, a heteroarylene group is a monocyclic heteroarylene group. In another embodiment, a heteroarylene group is a bicyclic heteroarylene group. In still another embodiment, a heteroarylene group has from about 5 to about 10 ring atoms. In another embodiment, a heteroarylene group is monocyclic and has 5 or 6 ring atoms. In another embodiment, a heteroarylene group is bicyclic and has 9 or 10 ring atoms. In another embodiment, a heteroarylene group is a 5-membered monocyclic heteroarylene. In another embodiment, a heteroarylene group is a 6-membered monocyclic heteroarylene. In another embodiment, a bicyclic heteroarylene group comprises a 5- or 6-membered monocyclic heteroarylene group fused to a benzene ring. In still another embodiment, a heteroaryl group comprises a 5- to 6-membered monocyclic heteroarylene group fused to a cycloalkyl ring or a heterocycloalkyl ring.

The term "heterocycloalkyl," as used herein, refers to a non-aromatic saturated monocyclic or multicyclic ring system comprising 3 to about 11 ring atoms, wherein from 1 to 4 of the ring atoms are independently O, S, N or Si, and the remainder of the ring atoms are carbon atoms. A heterocycloalkyl group can be joined via a ring carbon, ring silicon atom or ring nitrogen atom. In one embodiment, a heterocycloalkyl group is monocyclic and has from about 3 to about 7 ring atoms. In another embodiment, a heterocycloalkyl group is monocyclic has from about 4 to about 7 ring atoms. In another embodiment, a heterocycloalkyl group is bicyclic and has from about 7 to about 11 ring atoms. In still another embodiment, a heterocycloalkyl group is monocyclic and has 5 or 6 ring atoms. In one embodiment, a heterocycloalkyl group is monocyclic. In another embodiment, a heterocycloalkyl group is bicyclic. There are no adjacent oxygen and/or sulfur atoms present in the ring system. Any -NH group in a heterocycloalkyl ring may exist protected such as, for example, as an -N(BOC), -N(CBz), -N(Tos) group; such protected heterocycloalkyl groups are considered part of this invention. A heterocycloalkyl group can be optionally substituted by one or more "ring system substituents" which may be the same or different, and are as defined herein below. The nitrogen or sulfur atom of the heterocycloalkyl can be optionally oxidized to the corresponding N-oxide, S-oxide or S,S-dioxide. Non-limiting examples of monocyclic heterocycloalkyl rings include oxetanyl, piperidyl, pyrrolidinyl, piperazinyl, morpholinyl, thiomorpholinyl, thiazolidinyl, 1,4-dioxanyl, tetrahydrofuranyl, tetrahydrothiophenyl, delta-lactam, delta-lactone, silacyclopentane, silapyrrolidine, and all isomers thereof. Non-limiting illustrative examples of a silyl-containing heterocycloalkyl group include:

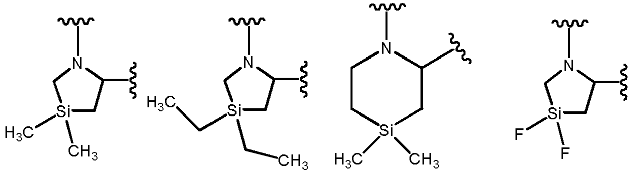

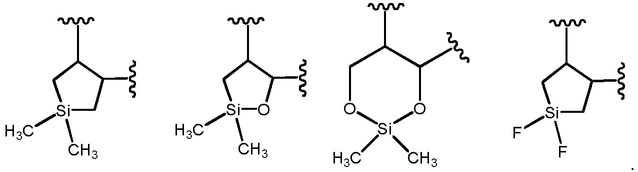

A ring carbon atom of a heterocycloalkyl group may be functionalized as a carbonyl group. Illustrative examples of such a heterocycloalkyl group include, but are not limited to:

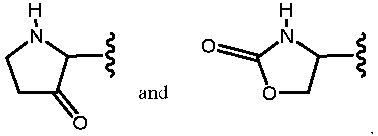

A ring sulfur atom of a heterocycloalkyl group may also be functionalized as a sulfonyl group. An example of such a heterocycloalkyl group is:

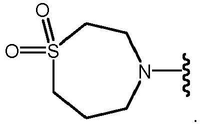

In one embodiment, a heterocycloalkyl group is a 5-membered monocyclic heterocycloalkyl. In another embodiment, a heterocycloalkyl group is a 6-membered monocyclic heterocycloalkyl. The term "5- to 7-membered monocyclic cycloalkyl" refers to a monocyclic heterocycloalkyl group having from 5 to 5 ring atoms. The term "4 to 6-membered monocyclic cycloalkyl" refers to a monocyclic heterocycloalkyl group having from 4 to 6 ring atoms. The term "9 to 10-membered bicyclic heterocycloalkyl" refers to a bicyclic heterocycloalkyl group having from 9 to 10 ring atoms.

The term "heterocycloalkenyl," as used herein, refers to a heterocycloalkyl group, as defined above, wherein the heterocycloalkyl group contains from 4 to 10 ring atoms, and at least one endocyclic carbon-carbon or carbon-nitrogen double bond. A heterocycloalkenyl group can be joined via a ring carbon or ring nitrogen atom. In one embodiment, a heterocycloalkenyl group has from 4 to 6 ring atoms. In another embodiment, a heterocycloalkenyl group is monocyclic and has 5 or 6 ring atoms. In another embodiment, a heterocycloalkenyl group is bicyclic. A heterocycloalkenyl group can optionally substituted by one or more ring system substituents, wherein "ring system substituent" is as defined above. The nitrogen or sulfur atom of the heterocycloalkenyl can be optionally oxidized to the corresponding N-oxide, S-oxide or S,S-dioxide. A ring carbon atom of a heterocycloalkenyl group may be functionalized as a carbonyl group. Non-limiting examples of heterocycloalkenyl groups include 1,2,3,4-tetrahydropyridinyl, 1,2-dihydropyridinyl, 1,4-dihydropyridinyl, 1,2,3,6-tetrahydropyridinyl, 1,4,5,6-tetrahydropyrimidinyl, 2-pyrrolinyl, 3-pyrrolinyl, 2-imidazolinyl, 2-pyrazolinyl, dihydroimidazolyl, dihydrooxazolyl, dihydrooxadiazolyl, dihydrothiazolyl, 3,4-dihydro-2H-pyranyl, dihydrofuranyl, fluoro-substituted dihydrofuranyl, 7-oxabicyclo[2.2.1]heptenyl, dihydrothiophenyl, dihydrothiopyranyl. In one embodiment, a heterocycloalkenyl group is a 5-membered heterocycloalkenyl. In another embodiment, a heterocycloalkenyl group is a 6-membered heterocycloalkenyl. The term "4 to 6-membered heterocycloalkenyl" refers to a heterocycloalkenyl group having from 4 to 6 ring atoms.

The term "substituted" means that one or more hydrogens on the designated atom is replaced with a selection from the indicated group, provided that the designated atom's normal valency under the existing circumstances is not exceeded, and that the substitution results in a stable compound. Combinations of substituents and/or variables are permissible only if such combinations result in stable compounds. By "stable compound' or "stable structure" is meant a compound that is sufficiently robust to survive isolation to a useful degree of purity from a reaction mixture, and formulation into an efficacious therapeutic agent.

The term "in substantially purified form," as used herein, refers to the physical state of a compound after the compound is isolated from a synthetic process (*e.g*., from a reaction mixture), a natural source, or a combination thereof. The term "in substantially purified form," also refers to the physical state of a compound after the compound is obtained from a purification process or processes described herein or well-known to the skilled artisan (*e.g*., chromatography, recrystallization), in sufficient purity to be characterizable by standard analytical techniques described herein or well-known to the skilled artisan.

It should also be noted that any carbon as well as heteroatom with unsatisfied valences in the text, schemes, examples and tables herein is assumed to have the sufficient number of hydrogen atom(s) to satisfy the valences.

When a functional group in a compound is termed "protected", this means that the group is in modified form to preclude undesired side reactions at the protected site when the compound is subjected to a reaction. Suitable protecting groups will be recognized by those with ordinary skill in the art as well as by reference to standard textbooks such as, for example, T. W. Greene et al, Protective Groups in Organic Synthesis (1991), Wiley, New York.

Examples of "ring system substituents" include, but are not limited to, alkyl, alkenyl, alkynyl, aryl, heteroaryl, -alkylene-aryl, -arylene-alkyl, -alkylene-heteroaryl,-alkenylene-heteroaryl, -alkynylene-heteroaryl, -OH, hydroxyalkyl, haloalkyl, -O-alkyl, -O-haloalkyl, -alkylene-O-alkyl, -O-aryl, -O-alkylene-aryl, acyl, - C(O)-aryl, halo, -NO₂, -CN, -SF₅, -C(O)OH, -C(O)O-alkyl, -C(O)O-aryl, -C(O)O-alkylene-aryl, - S(O)-alkyl, -S(O)₂-alkyl, -S(O)-aryl, -S(O)₂-aryl, -S(O)-heteroaryl, -S(O)z-heteroaryl, -S-alkyl, -S-aryl, -S-heteroaryl, -S-alkylene-aryl, -S-alkyleneheteroaryl, -S(O)₂-alkylene-aryl, -S(O)₂-alkylene-heteroaryl, -Si(alkyl)₂, -Si(aryl)₂, Si(heteroaryl)₂ -Si(alkyl)(aryl), - Si(alkyl)(cycloalkyl), -Si(alkyl)(heteroaryl), cycloalkyl, heterocycloalkyl, -O-C(O)-alkyl, -O-C(O)-aryl, -O-C(O)-cycloalkyl, -C(=N-CN)-NH₂, -C(=NH)-NH₂, -C(=NH)-NH(alkyl), - N(Y¹)(Y²), -alkylene-N(Y¹)(Y²), -C(O)N(Y¹)(Y²) and -S(O)₂N(Y¹)(Y²), wherein Y¹ and Y² can be the same or different and are independently selected from the group consisting of hydrogen, alkyl, aryl, cycloalkyl, and -alkylene-aryl. "Ring system substituent" may also mean a single moiety which simultaneously replaces two available hydrogens on two adjacent carbon atoms (one H on each carbon) on a ring system. Examples of such moiety are methylenedioxy, ethylenedioxy, -C(CH₃)₂- which form moieties such as,
for example:

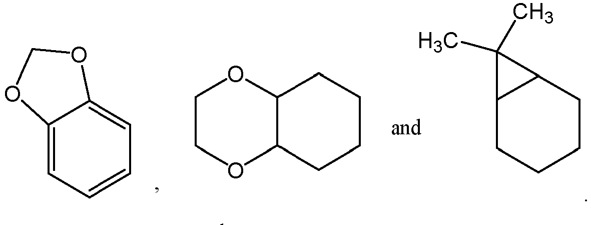

When any substituent or variable (*e.g*., R¹, m, etc.) occurs more than one time in any constituent or in Formula (I), its definition on each occurrence is independent of its definition at every other occurrence, unless otherwise indicated.

As used herein, the term "composition" is intended to encompass a product comprising the specified ingredients in the specified amounts, as well as any product which results from combination of the specified ingredients in the specified amounts.

Prodrugs and solvates of the compounds of the invention are also disclosed herein, but are not part of the claimed invention. A discussion of prodrugs is provided in T. Higuchi and V. Stella, Pro-drugs as Novel Delivery Systems (1987) 14 of the A.C.S. Symposium Series, and in Bioreversible Carriers in Drug Design, (1987) Edward B. Roche, ed., American Pharmaceutical Association and Pergamon Press. The term "prodrug" means a compound (*e.g.,* a drug precursor) that is transformed *in vivo* to provide an Indazole Derivative or a pharmaceutically acceptable salt or solvate of the compound. The transformation may occur by various mechanisms (*e.g*., by metabolic or chemical processes), such as, for example, through hydrolysis in blood.

For example, if an Indazole Derivative or a pharmaceutically acceptable salt, hydrate or solvate of the compound contains a carboxylic acid functional group, a prodrug can comprise an ester formed by the replacement of the hydrogen atom of the acid group with a group such as, for example, (C₁-C₈)alkyl, (C₂-C₁₂)alkanoyloxymethyl, 1-(alkanoyloxy)ethyl having from 4 to 9 carbon atoms, 1-methyl-1-(alkanoyloxy)-ethyl having from 5 to 10 carbon atoms, alkoxycarbonyloxymethyl having from 3 to 6 carbon atoms, 1-(alkoxycarbonyloxy)ethyl having from 4 to 6 carbon atoms, 1-methyl-1-(alkoxycarbonyloxy)ethyl having from 5 to 8 carbon atoms, N-(alkoxycarbonyl)aminomethyl having from 3 to 9 carbon atoms, 1-(N-(alkoxycarbonyl)amino)ethyl having from 4 to 10 carbon atoms, 3-phthalidyl, 4-crotonolactonyl, gamma-butyrolacton-4-yl, di-N,N-(C₁-C₂)alkylamino(C₂-C₃)alkyl (such as β-dimethylaminoethyl), carbamoyl-(C₁-C₂)alkyl, N,N-di (C₁-C₂)alkylcarbamoyl-(C₁-C₂)alkyl and piperidino-, pyrrolidino- or morpholino(C₂-C₃)alkyl.

Similarly, if an Indazole Derivative contains an alcohol functional group, a prodrug can be formed by the replacement of the hydrogen atom of the alcohol group with a group such as, for example, (C₁-C₆)alkanoyloxymethyl, 1-((C₁-C₆)alkanoyloxy)ethyl, 1-methyl-1-((C₁-C₆)alkanoyloxy)ethyl, (C₁-C₆)alkoxycarbonyloxymethyl, N-(C₁-C₆)alkoxycarbonylaminomethyl, succinoyl, (C₁-C₆)alkanoyl, α-amino(C₁-C₄)alkyl, α-amino(C₁-C₄)alkylene-aryl, arylacyl and α-aminoacyl, or α-aminoacyl-α-aminoacyl, where each α-aminoacyl group is independently selected from the naturally occurring L-amino acids, -P(O)(OH)₂, -P(O)(O(C₁-C₆)alkyl)₂ or glycosyl (the radical resulting from the removal of a hydroxyl group of the hemiacetal form of a carbohydrate).

If an Indazole Derivative incorporates an amine functional group, a prodrug can be formed by the replacement of a hydrogen atom in the amine group with a group such as, for example, R-carbonyl-, RO-carbonyl-, NRR'-carbonyl- wherein R and R' are each independently (C₁-C₁₀)alkyl, (C₃-C₇) cycloalkyl, benzyl, a natural α-aminoacyl, -C(OH)C(O)OY¹ wherein Y¹ is H, (C₁-C₆)alkyl or benzyl, -C(OY²)Y³ wherein Y² is (C₁-C₄) alkyl and Y³ is (C₁-C₆)alkyl; carboxy (C₁-C₆)alkyl; amino(C₁-C₄)alkyl or mono-N- or di-N,N-(C₁-C₆)alkylaminoalkyl; -C(Y⁴)Y⁵ wherein Y⁴ is H or methyl and Y⁵ is mono-N- or di-N,N-(C₁-C₆)alkylamino morpholino; piperidin-1-yl or pyrrolidin-1-yl.

Pharmaceutically acceptable esters of the present compounds include the following groups: (1) carboxylic acid esters obtained by esterification of the hydroxy group of a hydroxyl compound, in which the non-carbonyl moiety of the carboxylic acid portion of the ester grouping is selected from straight or branched chain alkyl (*e.g*., methyl, ethyl, n-propyl, isopropyl, t-butyl, sec-butyl or n-butyl), alkoxyalkyl (*e.g.,* methoxymethyl), aralkyl (*e.g.,* benzyl), aryloxyalkyl (for example, phenoxymethyl), aryl (*e.g.,* phenyl optionally substituted with, for example, halogen, C₁₋₄alkyl, -O-(C₁₋₄alkyl) or amino); (2) sulfonate esters, such as alkyl- or aralkylsulfonyl (for example, methanesulfonyl); (3) amino acid esters (*e.g*., L-valyl or L-isoleucyl); (4) phosphonate esters and (5) mono-, di- or triphosphate esters. The phosphate esters may be further esterified by, for example, a C₁₋₂₀ alcohol or reactive derivative thereof, or by a 2,3-di (C₆₋₂₄)acyl glycerol.

One or more compounds of the invention may exist in unsolvated as well as solvated forms with pharmaceutically acceptable solvents such as water, ethanol, and it is intended that the invention embrace both solvated and unsolvated forms. "Solvate" means a physical association of a compound of this invention with one or more solvent molecules. This physical association involves varying degrees of ionic and covalent bonding, including hydrogen bonding. In certain instances the solvate will be capable of isolation, for example when one or more solvent molecules are incorporated in the crystal lattice of the crystalline solid. "Solvate" encompasses both solution-phase and isolatable solvates. Non-limiting examples of solvates include ethanolates, methanolates. A "hydrate" is a solvate wherein the solvent molecule is water.

One or more compounds of the invention may optionally be converted to a solvate. Preparation of solvates is generally known. Thus, for example, M. Caira et al, J. Pharmaceutical Sci., 93(3), 601-611 (2004) describe the preparation of the solvates of the antifungal fluconazole in ethyl acetate as well as from water. Similar preparations of solvates, hemisolvate, hydrates are described by E. C. van Tonder et al, AAPS PharmSciTechours. , 5(1), article 12 (2004); and A. L. Bingham et al, Chem. Commun., 603-604 (2001). A typical, non-limiting, process involves dissolving the inventive compound in desired amounts of the desired solvent (organic or water or mixtures thereof) at a higher than room temperature, and cooling the solution at a rate sufficient to form crystals which are then isolated by standard methods. Analytical techniques such as, for example IR spectroscopy, show the presence of the solvent (or water) in the crystals as a solvate (or hydrate).

The Indazole Derivatives can form salts which are also within the scope of this invention. The term "salt(s)", as employed herein, denotes acidic salts formed with inorganic and/or organic acids, as well as basic salts formed with inorganic and/or organic bases. In addition, when an Indazole Derivative contains both a basic moiety, such as, but not limited to a pyridine or imidazole, and an acidic moiety, such as, but not limited to a carboxylic acid, zwitterions ("inner salts") may be formed and are included within the term "salt(s)" as used herein. In one embodiment, the salt is a pharmaceutically acceptable (*i.e.,* non-toxic, physiologically acceptable) salt. In another embodiment, the salt is other than a pharmaceutically acceptable salt. Salts of the Compounds of Formula (I) may be formed, for example, by reacting an Indazole Derivative with an amount of acid or base, such as an equivalent amount, in a medium such as one in which the salt precipitates or in an aqueous medium followed by lyophilization.

Exemplary acid addition salts include acetates, ascorbates, benzoates, benzenesulfonates, bisulfates, borates, butyrates, citrates, camphorates, camphorsulfonates, fumarates, hydrochlorides, hydrobromides, hydroiodides, lactates, maleates, methanesulfonates, naphthalenesulfonates, nitrates, oxalates, phosphates, propionates, salicylates, succinates, sulfates, tartarates, thiocyanates, toluenesulfonates (also known as tosylates). Additionally, acids which are generally considered suitable for the formation of pharmaceutically useful salts from basic pharmaceutical compounds are discussed, for example, by P. Stahl et al, Camille G. (eds.) Handbook of Pharmaceutical Salts. Properties, Selection and Use. (2002) Zurich: Wiley-VCH; S. Berge et al, Journal of Pharmaceutical Sciences (1977) 66(1) 1-19; P. Gould, International J. of Pharmaceutics (1986) 33 201-217; Anderson et al, The Practice of Medicinal Chemistry (1996), Academic Press, New York; and in The Orange Book (Food & Drug Administration, Washington, D.C. on their website).

Exemplary basic salts include ammonium salts, alkali metal salts such as sodium, lithium, and potassium salts, alkaline earth metal salts such as calcium and magnesium salts, salts with organic bases (for example, organic amines) such as dicyclohexylamine, t-butyl amine, choline, and salts with amino acids such as arginine, lysine. Basic nitrogen-containing groups may be quarternized with agents such as lower alkyl halides (*e.g*., methyl, ethyl, and butyl chlorides, bromides and iodides), dialkyl sulfates (*e.g*., dimethyl, diethyl, and dibutyl sulfates), long chain halides (*e.g.,* decyl, lauryl, and stearyl chlorides, bromides and iodides), aralkyl halides (*e.g.,* benzyl and phenethyl bromides), and others.

All such acid salts and base salts are intended to be pharmaceutically acceptable salts within the scope of the invention and all acid and base salts are considered equivalent to the free forms of the corresponding compounds for purposes of the invention.

Diastereomeric mixtures can be separated into their individual diastereomers on the basis of their physical chemical differences by methods well-known to those skilled in the art, such as, for example, by chromatography and/or fractional crystallization. Enantiomers can be separated by converting the enantiomeric mixture into a diastereomeric mixture by reaction with an appropriate optically active compound (*e.g*., chiral auxiliary such as a chiral alcohol or Mosher's acid chloride), separating the diastereomers and converting (*e.g*., hydrolyzing) the individual diastereomers to the corresponding pure enantiomers. Sterochemically pure compounds may also be prepared by using chiral starting materials or by employing salt resolution techniques. Also, some of the Indazole Derivatives may be atropisomers (*e.g*., substituted biaryls), and are considered as part of this invention. Enantiomers can also be directly separated using chiral chromatographic techniques.

It is also possible that the Indazole Derivatives may exist in different tautomeric forms, and all such forms are embraced within the scope of the invention. For example, all keto-enol and imine-enamine forms of the compounds are included in the invention.

Individual stereoisomers of the compounds of the invention may, for example, be substantially free of other isomers, or may be admixed, for example, as racemates or with all other, or other selected, stereoisomers. The chiral centers of the present invention can have the S or R configuration as defined by the *IUPAC* 1974 Recommendations. The use of the terms "salt", "solvate", is intended to apply equally to the salt, solvate, of enantiomers, stereoisomers, rotamers, tautomers, positional isomers, racemates of the inventive compounds.

In the Compounds of Formula (I), the atoms may exhibit their natural isotopic abundances, or one or more of the atoms may be artificially enriched in a particular isotope having the same atomic number, but an atomic mass or mass number different from the atomic mass or mass number predominantly found in nature. The present invention is meant to include all suitable isotopic variations of the compounds of generic Formula I. For example, different isotopic forms of hydrogen (H) include protium (¹H), and deuterium (²H). Protium is the predominant hydrogen isotope found in nature. Enriching for deuterium may afford certain therapeutic advantages, such as increasing *in vivo* half-life or reducing dosage requirements, or may provide a compound useful as a standard for characterization of biological samples. Isotopically-enriched Compounds of Formula (I) can be prepared without undue experimentation by conventional techniques well known to those skilled in the art or by processes analogous to those described in the Schemes and Examples herein using appropriate isotopically-enriched reagents and/or intermediates. In one embodiment, a Compound of Formula (I) has one or more of its hydrogen atoms replaced with deuterium.

The following abbreviations are used below and have the following meanings: Ac is acyl; BOC or Boc is *tert*-butyloxycarbonyl; Celite is diatomaceous earth; DCE is dichloroethane; DCM is dichloromethane; DIEA is diisopropylethylamine; DME is dimethoxyethane; DMF is N,N-dimethylformamide; dppf is diphenylphosphinoferrocene; DMSO s dimethylsulfoxide; Et₃N is triethylamine; EtOAc is ethyl acetate; HPLC is high performance liquid chromatography; Ir{dF(CF₃)ppy}₂(dtbpy)]PF₆ is [4,4'-*Bis*(1,1-dimethylethyl)-2,2'-bipyridine-*N*1*,N*1']*bis*[3,5-difluoro-2-[5-(trifluoromethyl)-2-pyridinyl-*N*]phenyl-C]Iridium(III) hexafluorophosphate; LCMS is liquid chromatography/mass spectrometry; Me is methyl; MeOH is methanol; MS is mass spectrometry; TFA is trifluoroacetic acid; THF is tetrahydrofuran; and TLC is thin-layer chromatography.

### The Compounds of Formula (I)

The present invention provides Indazole Derivatives of Formula (I): 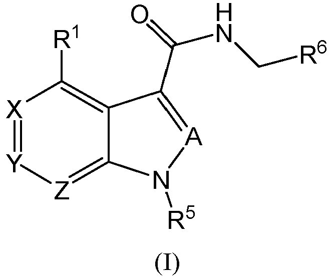 and pharmaceutically acceptable salts thereof, wherein A, A', R², R³, R⁴ and R⁵ are defined in the claims for the Compounds of Formula (I).

In one embodiment, A is -N-.

In another embodiment, A is -C(R⁸)-.

In one embodiment, X is -N-.

In one embodiment, Z is -N-.

In another embodiment, none of X, Y and Z are -N-.

In one embodiment, R¹ is H.

In another embodiment, R¹ is methyl.

In one embodiment, X is -C(R²)-.

In another embodiment, X is -C(R²)-, and R² is:

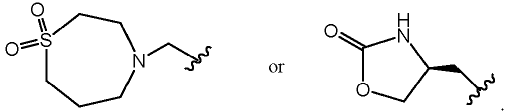

In one embodiment, Z is -C(R⁴)-.

In another embodiment, Z is -C(R⁴)-, and R⁴ is -Cl or -CN.

In one embodiment, R⁶ is 4-chlorophenyl.

In another embodiment, R⁶ is 4-cyanophenyl.

In one embodiment, the compounds of formula (I) are compounds of formula (Ia): 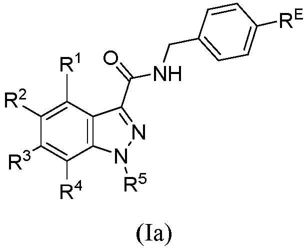 or a pharmaceutically acceptable salt thereof,
wherein R^{E} is Cl or -CN and the remaining substituents are as defined in the claims.

In one embodiment, for the compounds of formula (Ia), R¹ is H.

In another embodiment, for the compounds of formula (Ia), R¹ is methyl.

In one embodiment, for the compounds of formula (Ia), R² is 5- to 7-membered monocyclic heterocycloalkyl.

In another embodiment, for the compounds of formula (Ia), R² is:

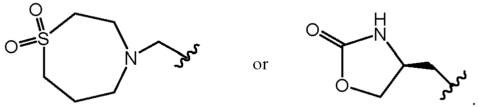

In one embodiment, for the compounds of formula (Ia), R³ is selected from 9- or 10-membered bicyclic heterocycloalkyl, -NH-CH₂-(5- or 6-membered monocyclic heteroaryl), and - O-CH₂-(5- or 6-membered monocyclic heteroaryl).

In another embodiment, for the compounds of formula (Ia), R³ is:

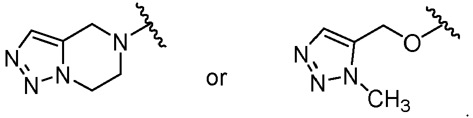

In one embodiment, for the compounds of formula (Ia), R⁴ is selected from H, -CH₃, and Cl.

In one embodiment, for the compounds of formula (Ia), R⁵ is selected from H,

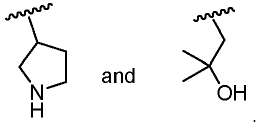

In one embodiment, for the compounds of formula (Ia), R^{E} is Cl.

In another embodiment, for the compounds of formula (Ia), R^{E} is -CN.

Other embodiments of the present invention include the following:
(a) A pharmaceutical composition comprising an effective amount of a Compound of Formula (I) or a pharmaceutically acceptable salt thereof, and a pharmaceutically acceptable carrier.
(b) The pharmaceutical composition of (a), further comprising a second therapeutic agent selected from the group consisting of anti-herpes agents and immunomodulators.
(c) The pharmaceutical composition of (b), wherein the anti-herpes agent is selected from the group consisting of herpesvirus polymerase inhibitors, and CMV terminase inhibitors.
(d) A pharmaceutical combination that is (i) a Compound of Formula (I), and (ii) a second therapeutic agent selected from the group consisting of anti-herpes agents and immunomodulators; wherein the Compound of Formula (I), and the second therapeutic agent are each employed in an amount that renders the combination effective for inhibiting herpesvirus replication, or for treating herpesvirus infection and/or reducing the likelihood or severity of symptoms of herpesvirus infection.
(e) The combination of (d), wherein the anti-herpes agent is selected from the group consisting of herpesvirus polymerase inhibitors, and CMV terminase inhibitors.
(f) A Compound of Formula (I) for use in a method of inhibiting herpesvirus replication in a subject.
(g) A Compound of Formula (I) for use in a method of treating herpesvirus infection and/or reducing the likelihood or severity of symptoms of herpesvirus infection in a subject.
(h) The compound for use according to (g), wherein the Compound of Formula (I) is administered in combination with an effective amount of at least one second therapeutic agent selected from the group consisting of anti-herpes agents and immunomodulators.
(i) The compounds for use according to (h), wherein the anti-herpes agent is selected from the group consisting of herpesvirus polymerase inhibitors, and CMV terminase inhibitors.
(j) A pharmaceutical composition for use in a method of inhibiting herpesvirus replication in a subject in need thereof which comprises administering to the subject the pharmaceutical composition of (a), (b) or (c) or the combination of (d) or (e).
(k) A pharmaceutical composition or combination for use in a method of treating herpesvirus infection and/or reducing the likelihood or severity of symptoms of herpesvirus infection in a subject in need thereof which comprises administering to the subject the pharmaceutical composition of (a), (b) or (c) or the combination of (d) or (e).

Additional embodiments of the invention include the pharmaceutical compositions, combinations and methods set forth in (a)-(k) above and the uses set forth in the preceding paragraph, wherein the compound of the present invention employed therein is a compound of one of the embodiments, aspects, classes, sub-classes, or features of the compounds described above. In all of these embodiments, the compound may optionally be used in the form of a pharmaceutically acceptable salt or hydrate as appropriate.

It is further to be understood that the embodiments of compositions and methods provided as (a) through (k) above are understood to include all embodiments of the compounds, including such embodiments as result from combinations of embodiments.

Non-limiting examples of the Compounds of Formula (I) include compounds **1-32,** as set forth in the Examples below, and pharmaceutically acceptable salts thereof.

### Methods For Making the Compounds of Formula (I)

The Compounds of Formula (I) may be prepared from known or readily prepared starting materials, following methods known to one skilled in the art of organic synthesis. Methods useful for making the Compounds of Formula (I) are set forth in the Examples below Alternative synthetic pathways and analogous structures will be apparent to those skilled in the art of organic synthesis.

One skilled in the art of organic synthesis will recognize that the synthesis of multicyclic heterocycle cores contained in Compounds of Formula (I) may require protection of certain functional groups (*i.e.,* derivatization for the purpose of chemical compatibility with a particular reaction condition). Suitable protecting groups for the various functional groups of these Compounds and methods for their installation and removal are well known in the art of organic chemistry. A summary of many of these methods can be found in Greene et al., Protective Groups in Organic Synthesis, Wiley-Interscience, New York, (1999).

One skilled in the art of organic synthesis will also recognize that one route for the synthesis of the multicyclic heterocycle cores of the Compounds of Formula (I) may be more desirable depending on the choice of appendage substituents.

Additionally, one skilled in the art will recognize that in some cases the order of reactions may differ from that presented herein to avoid functional group incompatibilities and thus adjust the synthetic route accordingly.

The preparation of multicyclic intermediates useful for making the multicyclic heterocycle cores of the Compounds of Formula (I) have been described in the literature and in compendia such as "Comprehensive Heterocyclic Chemistry" editions I, II and III, published by Elsevier and edited by A.R. Katritzky & R. JK Taylor. Manipulation of the required substitution patterns have also been described in the available chemical literature as summarized in compendia such as "Comprehensive Organic Chemistry" published by Elsevier and edited by DH R. Barton and W. D. Ollis; "Comprehensive Organic Functional Group Transformations" edited by edited by A.R. Katritzky & R. JK Taylor and "Comprehensive Organic Transformation" published by Wily-CVH and edited by R. C. Larock.

The starting materials used and the intermediates prepared using the methods set forth in the Examples below may be isolated and purified if desired using conventional techniques, including but not limited to filtration, distillation, crystallization, chromatography and alike. Such materials can be characterized using conventional means, including physical constants and spectral data.

One skilled in the art will be aware of standard formulation techniques as set forth in the open literature as well as in textbooks such as Zheng, "Formulation and Analytical Development for Low-dose Oral Drug Products," Wiley, 2009, ISBN.

### EXAMPLES

### General Methods

Solvents, reagents, and intermediates that are commercially available were used as received. Reagents and intermediates that are not commercially available were prepared in the manner as described below. ¹H NMR spectra are reported as ppm downfield from Me4Si with number of protons, multiplicities, and coupling constants in Hertz indicated parenthetically. Where LC/MS data are presented, the retention time and observed parent ion are given. Flash column chromatography was performed using pre-packed normal phase silica or bulk silica, and using a gradient elution of hexanes/ethyl acetate, from 100% hexanes to 100% ethyl acetate.

### Example 1

### Preparation of Compound 1

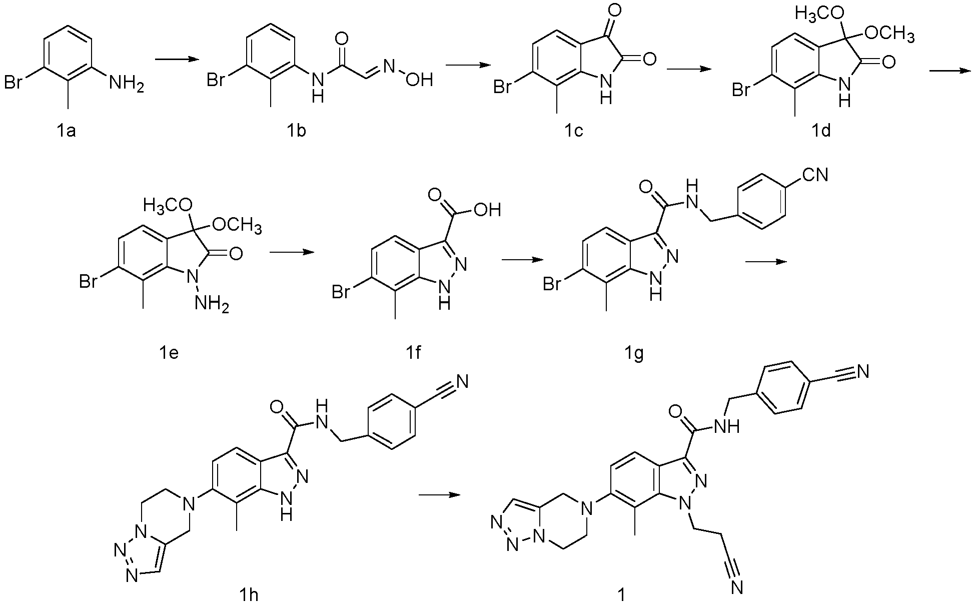

### Step A - synthesis of compound 1b

A solution of 3-bromo-2-methylaniline (6.00 g, 32.2 mmol) in 5 mL of 36% HCl was added to a mixture of hydroxylamine hydrochloride (7.84 g, 113 mmol), 2,2,2-tribromoacetaldehyde (14.84 g, 52.9 mmol), and sodium sulfate (33 g, 232 mmol) in water (186 mL). The resulting reaction was heated to 70 °C. and allowed to stir at this temperature for 2 hours. The reaction mixture was then filtered, and the collected solid was dried *in vacuo* to provide intermediate compound **1b**, which was used without further purification. MS: *m*/*z =* 257.0 [M+H].

### Step B - synthesis of compound 1c

Sulfuric acid (40 mL, 1135 mmol) was heated to 55 °C in a round bottom flask, and compound **1b** (7.2 g, 28.0 mmol) was added portion-wise over about 30 minutes while keeping the reaction temperature at 55°C. The reaction mixture was then heated to 70°C, and allowed to stir at this temperature for 30 minutes, then was allowed to cool to room temperature. The reaction mixture was slowly added to 300 grams of ice and allowed to stir for 1 hour, then filtered. The collected solid, compound **1c**, was used without further purification. MS: *m*/*z* = 242.1 [M+H].

### Step C - synthesis of compound 1d

To a solution of compound **1c** (2.5 g, 10.41 mmol) in MeOH (20 mL) was added trimethoxymethane (1.367 mL, 12.50 mmol), and p-toluenesulfonic acid (0.198 g, 1.041 mmol). The resulting reaction was heated to reflux and allowed to stir at this temperature for 6 hours. The reaction mixture was then basified with 1N NaOH and concentrated *in vacuo.* The residue obtained was purified by silica gel chromatography (120 gram ISCO Redisep Gold column, eluent 0-50% EtOAc/Hexanes) to provide compound **1d** as a solid. MS: *m*/*z* = 240.1 [M+H].

### Step D - synthesis of compound 1e

A solution of compound **1d** (1.40 g, 4.89 mmol) in THF (20 mL) was cooled to 0 °C and treated with 60% sodium hydride dispersion in mineral oil (0.215 g, 5.38 mmol). The resulting reaction was allowed to stir at 0 °C for 10 minutes, then a solution of (aminooxy)diphenylphosphine oxide (1.369 g, 5.87 mmol) in THF (20 mL) was added. The resulting reaction was allowed to stir at room temperature for 1 hour, filtered through a plug of Celite, and washed with ether (200 mL). The organic layer was collected, concentrated *in vacuo,* and purified using a 120 gram ISCO Redisep Gold column, eluting with 0-60% EtOAc/Hexanes containing 0.1% Et₃N to provide compound **1e** as a solid. MS: *m*/*z* = 257.0 [M+H].

### Step E - synthesis of compound 1f

To a suspension of compound **1e** (1.3 g, 4.32 mmol) in water (200 mL) was added concentrated sulfuric acid (14 mL, 252 mmol) dropwise, and the resulting reaction was allowed to stir at room temperature for 1 hour. The reaction mixture was then heated to 85 °C, and allowed to stir at this temperature for 3 hours. The reaction mixture was allowed to cool to room temperature and diluted with EtOAc (350 mL). The organic layer was collected, dried over magnesium sulfate, filtered, and concentrated *in vacuo* to provide compound **1f**, which was used without further purification. MS: *m*/*z* = 255.2 [M+H].

### Step F - synthesis of compound 1g

To a solution of (benzotriazol-1-yloxy)tris(dimethylamino)phosphonium hexafluorophosphate (4.96 g, 11.21 mmol), and **1f** (2.20 g, 8.63 mmol) in DMF (28.8 mL) was added DIEA (3.77 mL, 21.56 mmol). The resulting reaction was allowed to stir at room temperature for 10 minutes, then 4-(aminomethyl)benzonitrile (1.482 g, 11.21 mmol) was added. The resulting reaction was allowed to stir at room temperature under nitrogen for 2 hours, and poured into ice water (100 mL), and the resulting solution was allowed to stir for 30 minutes, then filtered. The collected solid was washed with water (2x50 mL), and dried *in vacuo* to provide compound **1g**, which was used without further purification. MS: *m*/*z* = 369.3 [M+H].

### Step G- synthesis of compound 1h

To a 40 mL vial containing compound **1g** (1.460 g, 3.95 mmol), 4,5,6,7-tetrahydro-[1,2,3]triazolo[1,5-a]pyrazine (736 mg, 5.93 mmol), and (2-dicyclohexylphosphino-2',6'-diisopropyl-1,1'-biphenyl)[2-(2-aminoethyl)phenyl)]palladium(II) (288 mg, 0.395 mmol) was added THF (20 mL). The vial was capped and degassed, 1.0 M lithium bis(trimethylsilyl)amide in THF (11.9 mL, 11.86 mmol) was added, and the reaction was degassed again. The resulting reaction was heated to 50 °C, and allowed to stir at this temperature for 2 hours. The reaction mixture was allowed to cool to room temperature, then 10 mL of water was added. The resulting solution was extracted with ethyl acetate (3x10 mL), dried over sodium sulfate, filtered through Celite, and concentrated *in vacuo.* The residue obtained was dissolved in DCM:MeOH (8:1), and purified via silica gel chromatography using a 120 gram ISCO Redisep Gold column, and eluting with 40-100% EtOAc/Hexanes to provide compound **1h**. MS: *m*/*z* = 413.4 [M+H].

### Step H- synthesis of compound 1

To a solution of compound **1h** (550 mg, 1.3 mmol) in DMF (1.3 mL) was added 3-bromopropanenitrile (330 µL, 4.0 mmol), and potassium carbonate (920 mg, 6.7 mmol). The resulting reaction was heated to 60 °C, and allowed to stir at this temperature for 2 hours. The resulting reaction was filtered, and concentrated *in vacuo,* and the resulting residue was purified by silica gel chromatography, eluting with a gradient of 3:1 ethyl acetate:ethanol:hexanes - 40: 100 to 100:0, to provide compound **1.** MS: *m*/*z* = 466.4 [M+H]. ¹H NMR (500 MHz, DMSO-*d*₆) δ 9.04 (t, 1H), 8.03 (d, *J =* 8.7 Hz, 1H), 7.80 (d, *J* = 8.2 Hz, 2H), 7.63 (s, 1H), 7.53 (d, *J =* 8.2 Hz, 2H), 7.25 (d, *J =* 8.8 Hz, 1H), 4.93 (t, *J* = 6.5 Hz, 2H), 4.57 (d, *J* = 6.2 Hz, 2H), 4.52 (t, *J =* 5.4 Hz, 2H), 4.29 (s, 2H), 3.48 (t, *J* = 5.4 Hz, 2H), 3.24 (t, *J* = 6.4 Hz, 2H), 2.70 (s, 3H).

The following compounds of the present invention were made using the methods described in the Example above, substituting the appropriate reactants and/or reagents:

| **Compound** | **Structure** | **MS [M+H]** |
|---|---|---|
| **4** | 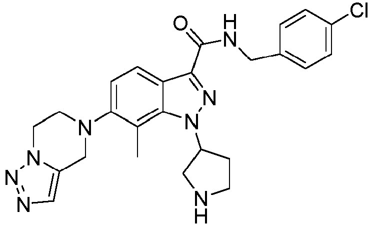 | 491.3 |
| **5** | 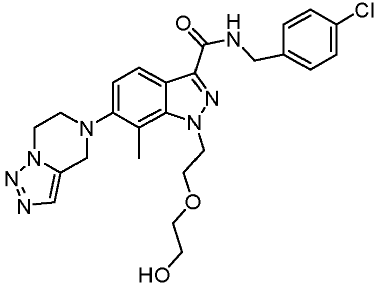 | 510.4 |
| **6** | 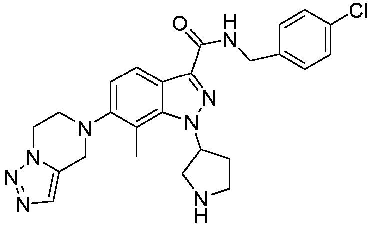 | 491.4 |
| **14** | 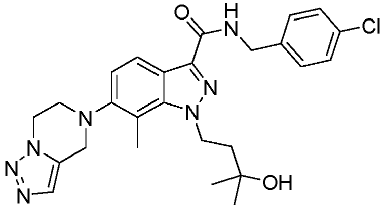 | 494.4 |
| **27** | 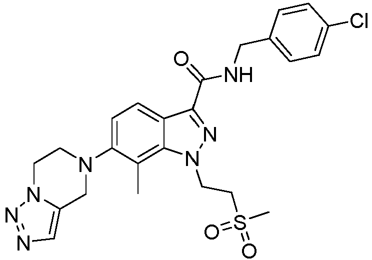 | 526.2 |
| **32** | 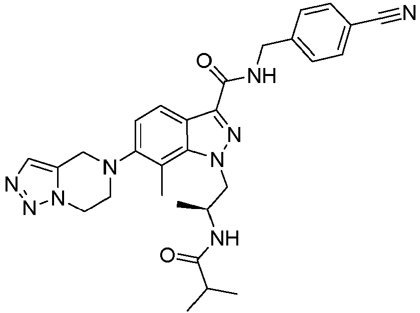 | 540.7 |

### Example 2

### Preparation of Compound 2

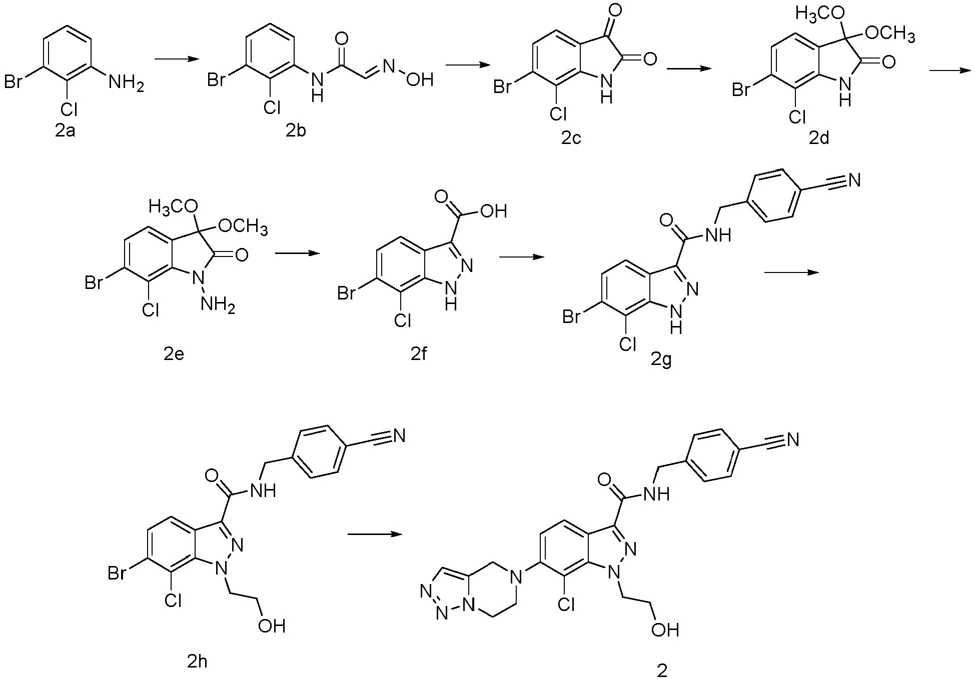

### Steps A-E - synthesis of compound 2f

Compound 2f was prepared using the methods described in Example 1, steps A-E, substituting the appropriate reactants and/or reagents. MS: *m*/*z* = 275.0 [M+H].

### Step F - synthesis of compound 2g

Compound 2g was using the method described in Example 1, step F, substituting the appropriate reactants and/or reagents. MS: *m*/*z* = 391.2 [M+H].

### Step G - synthesis of compound 2h

Compound 2h was using the method described in Example 1,step G substituting the appropriate reactants and/or reagents. MS: *m*/*z* =435.2 [M+H].

### Step H - synthesis of compound 2

Compound **2** was prepared using the method described in Example 1, step H, substituting the appropriate reactants and/or reagents. MS: *m*/*z* = 477.5 [M+H]. ¹H NMR (500 MHz, DMSO-*d*₆) δ 9.13 (t, *J* = 6.3 Hz, 1H), 8.14 (d, *J* = 8.7 Hz, 1H), 7.80 (d, *J* = 8.3 Hz, 2H), 7.65 (s, 1H), 7.53 (d, *J* = 8.2 Hz, 2H), 7.29 (d, *J* = 8.7 Hz, 1H), 4.94 (t, *J* = 5.7 Hz, 1H), 4.85 (t, *J* = 6.0 Hz, 2H), 4.58 - 4.50 (m, 4H), 4.46 (s, 2H), 3.88 (q, *J* = 5.9 Hz, 2H), 3.63 (t, *J* = 5.3 Hz, 2H).

### Example 3

### Preparation of Compound 3

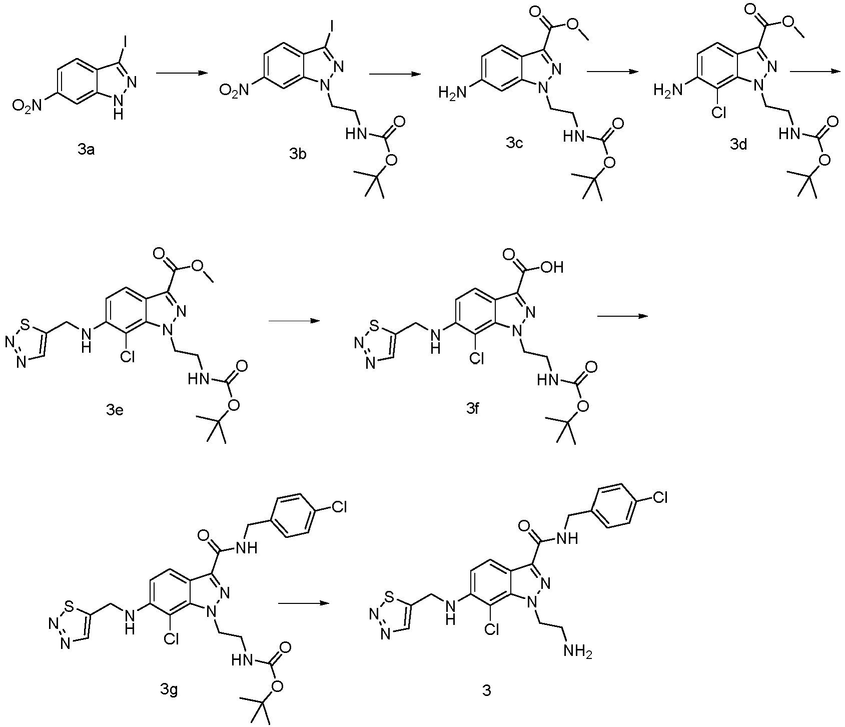

### Step A - synthesis of compound 3b

Compound 3b was prepared using the method described in Example 1, step H, substituting the appropriate reactants and/or reagents. MS: *m*/*z 333.0* = [M-BOC+H].

### Step B - synthesis of compound 3c

To a high-pressure reaction vessel was added compound **3b** (3.5 g, 8.1 mmol), methanol (90 mL), and DMSO (20 mL) followed by triethylamine (4.5 mL, 32.4 mmol). After degassing for 10 minutes, palladium(II) acetate (0.36 g, 1.62 mmol), and 1,3-bis(diphenylphosphino)propane (0.67 g, 1.6 mmol) were added, and the reaction mixture was again degassed for 10 minutes using nitrogen. The vessel was then charged with 45 psi CO gas, sealed, heated to 80 °C, and allowed to stir at this temperature for 16 hours. The reaction mixture was then concentrated *in vacuo,* and the residue obtained was purified by silica gel chromatography eluting with 65-85% EtOAc/petroleum ether to provide compound **3c** as a solid. MS: *m*/*z* = 335.2 [M+H].

### Step C - synthesis of compound 3d

To a solution of compound **3c** (450 mg, 1.3 mmol) in acetonitrile (10 mL), and DCM (10 mL) was added N-chlorosuccinimide (270 mg, 2.0 mmol). The resulting reaction was allowed to stir for 4 hours at room temperature, then was concentrated *in vacuo.* The resulting residue was diluted with water and DCM. The resulting solution was extracted with DCM and washed with brine. The combined organic extracts were dried over sodium sulfate, concentrated *in vacuo,* and the resulting residue was purified by silica gel chromatography, eluting with at 30-40% EtOAc/petroleum ether to provide compound **3d.** MS: *m*/*z* = 369.2 [M+H].

### Step D - synthesis of compound 3e

To a solution of compound 3**d** (200 mg, 0.54 mmol) in DCE (5 mL) was added 1,2,3-thiadiazole-5-carbaldehyde (186 mg, 1.6 mmol), followed by sodium triacetoxyborohydride (345 mg, 1.6 mmol). The reaction mixture was heated to 100°C, and allowed to stir at this temperature for 16 hours. The reaction mixture was diluted with DCM and water, extracted with DCM, and washed with brine. The combined organic extracts were dried over sodium sulfate, filtered, concentrated *in vacuo.* The residue obtained was dissolved in MeOH (5 mL), then sodium borohydride (41.0 mg, 1.1 mmol) was added. The resulting reaction was allowed to stir at room temperature for 2 hours, then the reaction mixture was concentrated *in vacuo,* diluted with DCM, and washed with brine. The combined organic extracts were dried over sodium sulfate, concentrated *in vacuo,* and the residue obtained was purified by silica gel chromatography eluting with 50% EtOAc/petroleum ether to provide compound **3e** as a solid. MS: *m*/*z* = 467.0 [M+H].

### Step E - synthesis of compound 3f

To a solution of compound 3**e** (50 mg, 0.1 mmol) in THF (2 mL) was added a solution of LiOH (7.7 mg, 0.3 mmol) in water (0.5 mL). The resulting reaction was allowed to stir at room temperature for 2 hours, then concentrated *in vacuo.* The resulting residue was acidified with 1.5N HCl and then extracted with ethyl acetate. The organic extract was dried over sodium sulfate, filtered, and concentrated *in vacuo* to provide compound **3f** as a solid. MS: *m*/*z =* 451.2 [M+H].

### Step F - synthesis of compound 3g

Compound 3g was prepared using the method described in Example 1, step F, substituting the appropriate reactants and/or reagents. MS: *m*/*z* = 577.0 [M+H].

### Step G - synthesis of compound 3

To a solution of compound 3**g** (25 mg, 0.04 mmol) in DCM (2 mL) at 0°C, was added TFA (10.0 µL) 0.1 mmol). The resulting reaction was allowed to stir at room temperature for 2 hours, then was concentrated *in vacuo,* and the residue obtained was triturated with diethyl ether to provide compound **3** as a solid, which was used without further purification. MS: *m*/*z* = 476.2 [M+H]. ¹H NMR (500 MHz, DMSO-*d*₆) δ 8.94 (t, *J=* 6 Hz, 1H), 8.90 (s, 1H), 7.92 (d, J=8.8 Hz, 1H), 7.41 - 7.35 (m, 4H), 6.95 (d, *J=* 8.8 Hz, 1H). 6.86 (t, *J=* 6 Hz, 1H), 5.01 (d, *J*= 6 Hz, 2H), 4.94 (t, *J=* 6 Hz, 2H), 4.48 (d, *J=* 6 Hz, 2H), 3.40 (s, 2H).

### Example 4

### Preparation of Compound 7

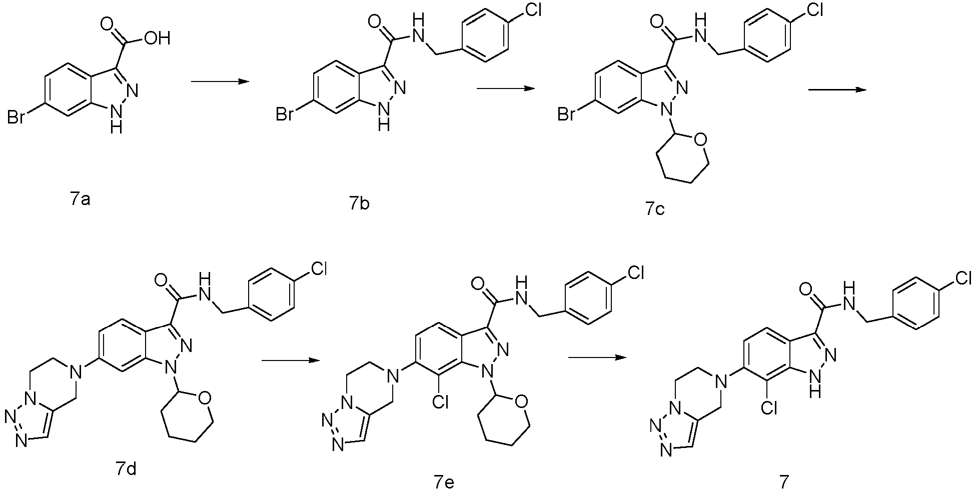

### Step A - synthesis of compound 7b

Compound 7b was prepared using the method described in Example 1, step F, substituting the appropriate reactants and/or reagents. MS: *m*/*z* 366.0 = [M+H].

### Step B - synthesis of compound 7c

To a 500 mL round bottom flask was added **7b** (6.9 g, 18.9 mmol), p-toluenesulfonic acid (0.360 g, 1.892 mmol), and THF (95 mL), followed by 3,4-dihydro-2H-pyran (17.3 mL, 190 mmol). The sample was heated to reflux for 1 hour, then concentrated *in vacuo.* The resulting residue was purified by silica gel chromatography using a 120 gram ISCO Redisep Gold column eluting with 0-45% 3:1 EtOAc:EtOH in hexanes to provide compound 7c as a solid. MS: *m*/*z* 450.1 = [M+H].

### Step C - synthesis of compound 7d

Compound 7d was prepared using the method described in Example 1, step G, substituting the appropriate reactants and/or reagents. MS: *m*/*z* 492.2 = [M+H].

### Step D - synthesis of compound 7e

Compound 7e was prepared using the method described in Example 3, step C, substituting the appropriate reactants and/or reagents. MS: *m*/*z* 526.3 = [M+H].

### Step E - synthesis of compound 7

To a solution of compound **7e** (48.6 mg, 0.09 mmol) in DCM (0.9 mL), was added 4 M hydrochloric acid (0.23 mL, 0.9 mmol), and the reaction was allowed to stir at room temperature for about 15 hours. The sample was concentrated *in vacuo,* and the resulting residue was purified using preparative HPLC (reverse-phase C-18), eluting with acetonitrile/water + 0.1% formic acid 5-95%. The sample was freeze-dried to provide compound **7** as a solid. MS: *m*/*z* 442.1 = [M+H]. ¹H NMR (500 MHz, Methanol-*d*₄) δ 8.16 (d, *J* = 8.7 Hz, 1H), 7.66 (s, 1H), 7.40 (d, *J* = 8.5 Hz, 2H), 7.35 (d, *J* = 8.5 Hz, 2H), 7.27 (d, *J* = 8.7 Hz, 1H), 4.63-4.60 (m, 4H), 4.55 (s, 2H), 3.79 - 3.73 (m, 2H).

### Example 5

### Preparation of Compound 8

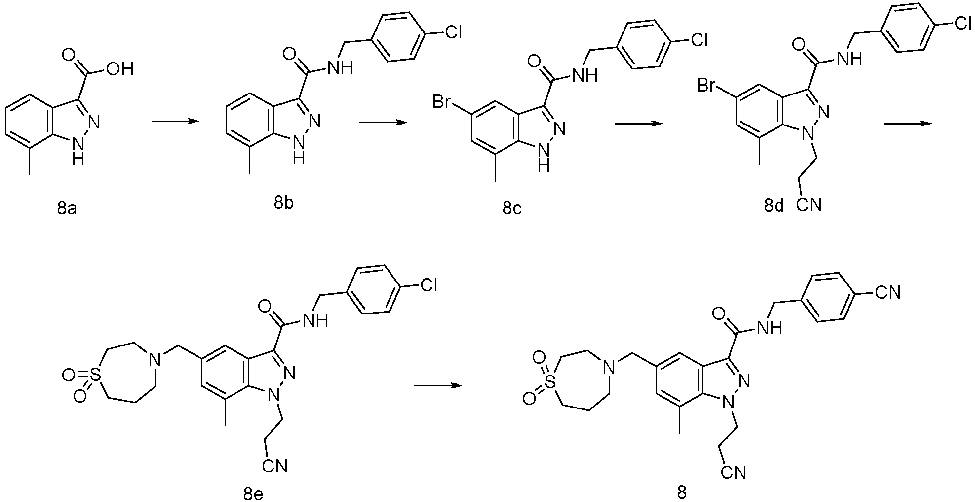

### Step A - synthesis of compound 8b

Compound 8b was made using the method described in Example 1, step F, substituting the appropriate reactants and/or reagents. MS: *m*/*z* 300.1 = [M+H].

### Step B - synthesis of compound 8c

To a solution of compound **8b** (500 mg, 1.7 mmol) in DMF (17 mL) was added N-bromosuccinimide (300 mg, 1.7 mmol), and the resulting reaction was allowed to stir at room temperature for 1 hour, then heated to 50 °C, and allowed to stir at this temperature for 15 hours. The reaction mixture was concentrated *in vacuo,* and the resulting residue was purified using preparative HPLC (reverse-phase C-18), eluting with acetonitrile/water + 0.1% formic acid 5-95%, and freeze-dried to provide compound **8c** as a solid. MS: *m*/*z* 380.0 = [M+H].

### Step C - synthesis of compound 8d

Compound 8d was made using the method described in Example 1, step H, substituting the appropriate reactants and/or reagents. MS: *m*/*z* 433.0 = [M+H].

### Step D - synthesis of compound 8e

To a 5 mL microwave-safe vial was added compound **8d** (45.8 mg, 0.11 mmol), chloro[(di(1-adamantyl)-n-butylphosphine)-2-(2-aminobiphenyl)]palladium(II) (7.1 mg, 11 µmol), and 4-((trifluoroboranyl)methyl)-1,4-thiazepane-1,1-dioxide, potassium salt (114 mg, 0.424 mmol). To the resulting mixture was then added dioxane (1.0 mL), cesium carbonate (104 mg, 0.318 mmol), and water (0.5 mL). The resulting reaction was sealed, purged with nitrogen gas, and heated to 110°C for 20 hours. The reaction mixture was then filtered, and purified using preparative HPLC (reverse-phase C-18), eluting with acetonitrile/water + 0.1% formic acid 5-95%, and freeze-dried to provide compound **8e** as a solid. MS: *m*/*z* 514.2 = [M+H].

### Step E - synthesis of compound 8

To a 5 mL microwave-safe vial was added compound **8e** (20.0 mg, 0.039 mmol), zinc cyanide (5.5 mg, 0.047 mmol), chloro(2-dicyclohexylphosphino-2',4',6'-triisopropyl-1,1'-biphenyl)[2-(2'-amino-1,1'-biphenyl)]palladium(II) (4.6 mg, 5.8 µmol), and potassium phosphate (12.4 mg, 0.058 mmol). The mixture was purged three times with nitrogen, and suspended in dry DMF (1.0 mL). The resulting reaction was allowed to stir at 50 °C for 2 hours, then filtered, purified using preparative HPLC (reverse-phase C-18), eluting with acetonitrile/water + 0.1% formic acid 5-95%, and freeze-dried to provide compound **8** as a solid. MS: *m*/*z* 505.4 = [M+H]. ¹H NMR (500 MHz, DMSO-*d*₆) δ 9.02 (t, *J* = 6.3 Hz, 1H), 7.96 (s, 1H), 7.81 (d, *J* = 8.3 Hz, 2H), 7.55 (d, *J =* 8.3 Hz, 2H), 7.28 (s, 1H), 4.90 (t, *J* = 6.5 Hz, 2H), 4.58 (d, *J* = 6.3 Hz, 2H), 3.31 (s, 2H), 3.30 - 3.23 (m, 4H), 3.23 - 3.17 (m, 2H), 2.92 - 2.87 (m, 2H), 2.85 - 2.79 (m, 2H), 2.76 (s, 2H), 1.92 (p, *J* = 6.1 Hz, 2H).

### Example 6

### Preparation of Intermediate Compound Int-1

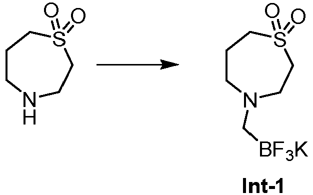

To a 100 mL round bottom flask was added 1,4-thiazepane-1,1-dioxide, HCl (5.00 g, 27.0 mmol), potassium (bromomethyl)trifluoroborate (6.00 g, 29.9 mmol), and THF (43 mL). The resulting reaction was heated to reflux and allowed to stir at this temperature for about 15 hours. The reaction mixture was then cooled, diluted with 100 mL acetone, and potassium carbonate (4.10 g, 29.9 mmol) was added in a single portion. The resulting solution was allowed to stir at room temperature for 2 hours, then filtered through Celite, and the filtrate was concentrated *in vacuo* to provide intermediate compound **Int-1,** which was used without further purification.

### Example 7

### Preparation of Compound 10

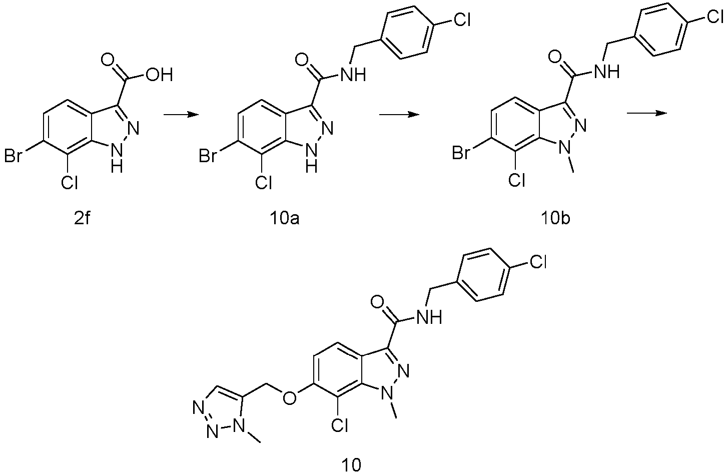

### Step A - synthesis of compound 10a

Compound 10a was made using the method described in Example 2, step F, substituting the appropriate reactants and/or reagents. MS: *m*/*z* 400.2 = [M+H].

### Step B - synthesis of compound 10b

Compound 10b was made using the method described in Example 2, step G, substituting the appropriate reactants and/or reagents. 414.2 = [M+H].

### Step C - synthesis of compound 10

To a 5 mL microwave-safe vial was added compound **10b** (80 mg, 0.19 mmol), (1-methyl-1H-1,2,3-triazol-5-yl)methanol (43.8 mg, 0.39 mmol), cesium carbonate (252 mg, 0.78 mmol), and methanesulfonato(2-(di-t-butylphosphino)-3-methoxy-6-methyl-2',4',6'-tri-i-propyl-1,1'-biphenyl)(2'-amino-1,1'-biphenyl-2-yl)palladium(II) (16.2 mg, 0.019 mmol). The vial was sealed and degassed with nitrogen. To this mixture was added toluene (2 mL), and the reaction was heated to 100 °C for 3 hours. The reaction mixture was cooled to room temperature, diluted with EtOAc (100 mL), and washed with water (100 mL). The collected organic phase was dried over magnesium sulfate, filtered, concentrated *in* vacuo, and the resulting residue was purified by silica gel chromatography using a 12 gram ISCO Redisep Gold column, and eluted with 0-100% EtOAc in hexanes to provide compound **10** as a solid. MS: *m*/*z* 445.4 = [M+H]. ¹H NMR (500 MHz, Chloroform-d) δ 8.32 (d, *J* = 8.9 Hz, 1H), 7.74 (s, 1H), 7.33 (s, 4H), 7.09 (d, *J* = 8.9 Hz, 1H), 5.29 (s, 2H), 4.65 (d, *J* = 6.1 Hz, 2H), 4.38 (s, 3H), 4.23 (s, 3H).

### Example 8

### Preparation of Compound 11

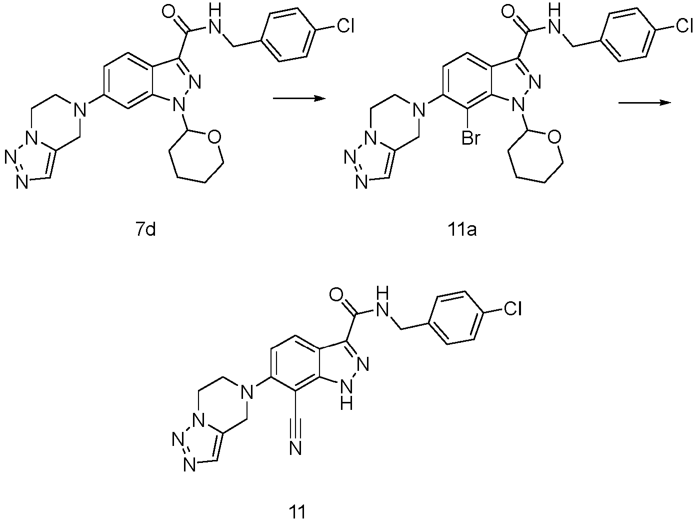

### Step A - synthesis of compound 11a

Compound 11a was made using the method described in Example 4, step D, substituting the appropriate reactants and/or reagents. MS: *m*/*z* 572.1 = [M+H].

### Step B - synthesis of compound 11

To compound **11a** (100 mg, 0.18 mmol), and copper(I) cyanide (20.4 mg, 0.23 mmol) was added DMF (2.5 mL), and the resulting reaction was heated to 150 °C and allowed to stir at this temperature for 10 hours. The reaction mixture was diluted with 50 mL EtOAc, and washed with 10 mL saturated ammonium chloride solution. The organic layer was separated, dried over magnesium sulfate, filtered, and concentrated *in vacuo.* The resulting residue was purified using preparative HPLC (reverse-phase C-18), eluting with acetonitrile/water + 0.1% formic acid 5-95%, and freeze-dried to provide compound **11** as a solid. MS: *m*/*z* 433.2 = [M+H]. ¹H NMR (500 MHz, DMSO-*d*₆) δ 8.30 (d, *J* = 8.7 Hz, 1H), 7.68 (s, 1H), 7.38 (s, 4H), 7.19 (d, *J* = 9.8 Hz, 1H), 4.76 (s, 2H), 4.59 - 4.54 (m, 2H), 4.46 (d, *J* = 6.3 Hz, 2H), 4.00 - 3.97 (m, 2H).

### Example 9

### Preparation of Compound 12

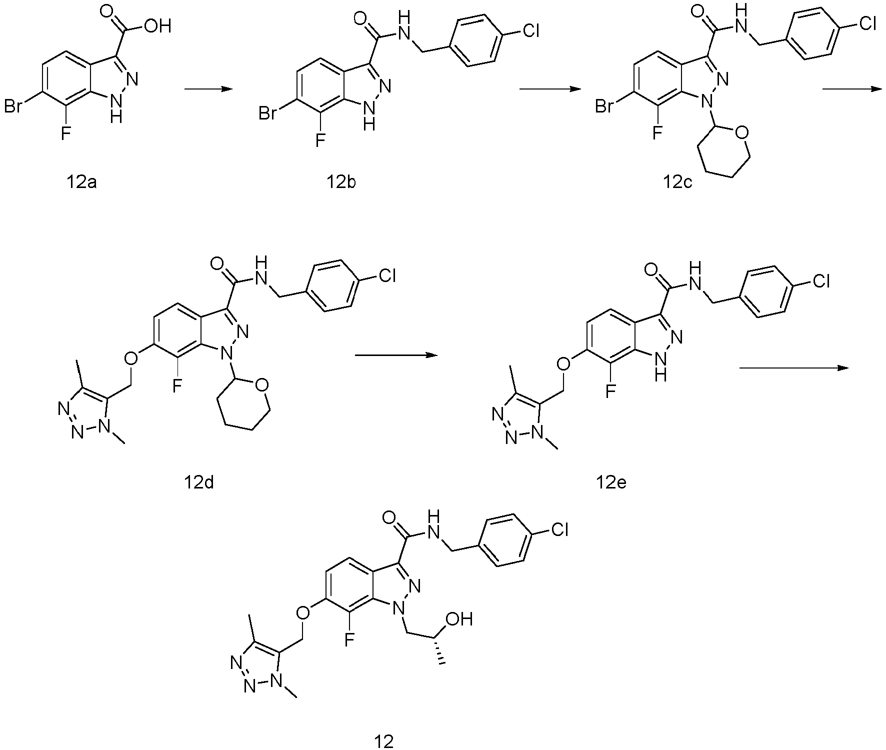

### Step A - synthesis of compound 12b

Compound 12b was made using the method described in Example 1, step F, substituting the appropriate reactants and/or reagents. MS: *m*/*z* 382.3 = [M+H].

### Step B - synthesis of compound 12c

Compound 12c was made using the method described in Example 4, step B, substituting the appropriate reactants and/or reagents. MS: *m*/*z* 466.2 = [M+H].

### Step C - synthesis of compound 12d

Compound 12d was made using the method described in Example 7, step C, substituting the appropriate reactants and/or reagents. MS: *m*/*z* 513.4 = [M+H].

### Step D - synthesis of compound 12e

Compound 12e was made using the method described in Example 4, step E, substituting the appropriate reactants and/or reagents. MS: *m*/*z* 429.3 = [M+H].

### Step E - synthesis of compound 12

To a solution of compound **12e** (30 mg, 0.07 mmol), and (R)-(+)-propylene oxide (12 mg, 0.21 mmol) in DMF (230 µl) was added cesium carbonate (40 mg, 0.12 mmol), and the resulting reaction was capped, heated to 80 °C and allowed to stir at this temperature for about 15 hours. The reaction mixture was diluted with 1 mL DMF, filtered, and purified using preparative HPLC (reverse-phase C-18), eluting with acetonitrile/water + 0.1% TFA 10-55%. Fractions containing product were combined, adjusted to basic pH with saturated sodium bicarbonate, and extracted with 2 x 5 mL EtOAc. The combined organic extracts were dried over magnesium sulfate, filtered, concentrated, and dried *in vacuo* to provide compound 12 as a solid. MS: *m*/*z* 487.3 = [M+H]. ¹H NMR (500 MHz, Chloroform-d) δ 8.14 (d, *J =* 8.8 Hz, 1H), 7.34 (s, 4H), 7.13 - 7.03 (m, 1H), 5.21 (s, 2H), 4.65 (d, *J* = 6.1 Hz, 2H), 4.55 - 4.48 (m, 1H), 4.46 - 4.38 (m, 1H), 4.36 - 4.26 (m, 1H), 4.16 (s, 3H), 2.28 (s, 3H), 1.29 (d, *J* = 6.3 Hz, 3H).

### Example 10

### Preparation of Compound 13

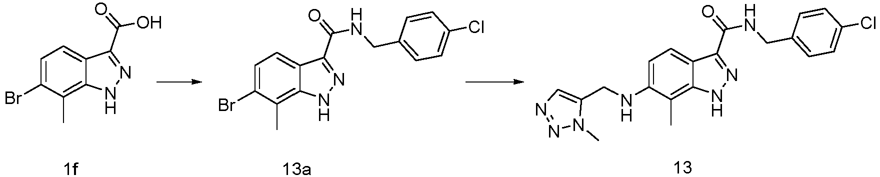

### Step A - synthesis of compound 13a

Compound 13a was made using the method described in Example 1, step F, substituting the appropriate reactants and/or reagents. MS: *m*/*z* 380.1 = [M+H].

### Step B - synthesis of compound 13

To a 5 mL microwave-safe vial was added compound **13a** (100 mg, 0.26 mmol), chloro(2-dicyclohexylphosphino-2',6'-diisopropoxy-1,1'-biphenyl)[2-(2'-amino-1,1'-biphenyl)] palladium(II) (38.5 mg, 0.053 mmol), (1-methyl-1H-1,2,3-triazol-5-yl)methanamine hydrochloride (58.9 mg, 0.40 mmol), THF (1.3 mL), and 2 M sodium tert-butoxide (530 µl, 1.1 mmol). The vial was sealed and heated to 50 °C for about 15 hours, with stirring. The reaction mixture was diluted with 10 mL EtOAc, and washed with 10 mL saturated ammonium chloride. The collected organic phase was dried over sodium sulfate, filtered, and concentrated *in vacuo,* and the resulting residue was purified using preparative HPLC (reverse-phase C-18), eluting with acetonitrile/water + 0.1% TFA 5-95% to provide compound **13.** MS: *m*/*z* 410.1 = [M+H]. ¹H NMR (500 MHz, DMSO-*d*₆) δ 8.83 (t, *J* = 6.2 Hz, 1H), 7.74 (d, *J* = 8.8 Hz, 1H), 7.53 (s, 1H), 7.40 - 7.28 (m, 4H), 6.76 (d, *J* = 8.9 Hz, 1H), 5.72 (t, *J* = 6.0 Hz, 1H), 4.54 (d, *J* = 6.0 Hz, 2H), 4.43 (d, *J=* 6.4 Hz, 2H), 4.03 (s, 3H), 2.30 (s, 3H).

### Example 11

### Preparation of Compound 15

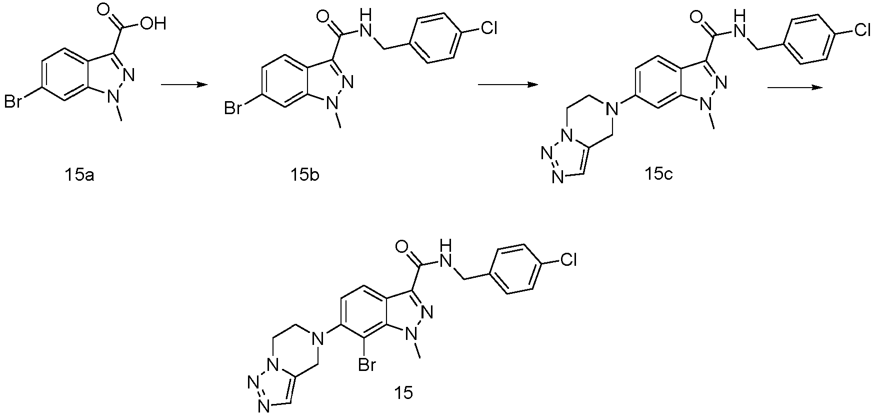

### Step A - synthesis of compound 15b

Compound 15b was made using the method described in Example 1, step F, substituting the appropriate reactants and/or reagents. MS: *m*/*z* 380.1 = [M+H].

### Step B - synthesis of compound 15c

Compound 15c was made using the method described in Example 1, step G, substituting the appropriate reactants and/or reagents. MS: *m*/*z* 422.3 = [M+H].

### Step C - synthesis of compound 15

To a solution of compound **15c** (100 mg, 0.24 mmol) in chloroform (2.4 mL) was added bromine (36.6 µl, 0.71 mmol). The resulting reaction was allowed to stir at room temperature for 2 hours, then quenched with 1 mL saturated sodium thiosulfate. The organic layer was separated and concentrated under a stream of nitrogen. The resulting residue was purified by silica gel chromatography using a 12 gram ISCO Redisep Gold column, and eluted with 0-50% EtOAc in hexanes to provide compound 15 as a solid. MS: *m*/*z* 502.1 = [M+H]. ¹H NMR (500 MHz, Chloroform-*d*) δ 8.38 (d, *J* = 8.6 Hz, 1H), 7.58 (s, 1H), 7.32 (s, 4H), 7.13 (d, *J* = 8.6 Hz, 1H), 4.69 - 4.60 (m, 4H), 4.45-4.42 (m, 5H), 3.65 - 3.58 (m, 2H).

### Example 12

### Preparation of Compound 16

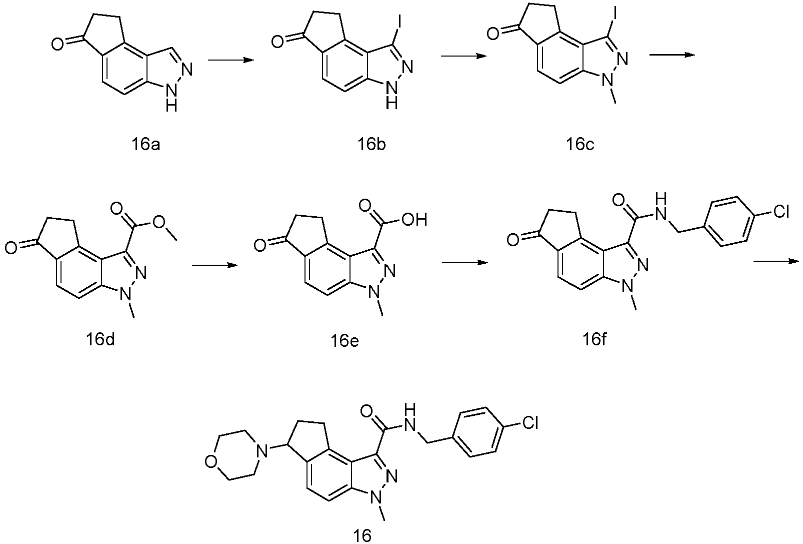

### Step A - synthesis of compound 16b

To a solution of compound **16a** (90 mg, 0.52 mmol, prepared as described in US Patent No. 7,923,568) in DMF (5 mL) was added N-iodosuccinimide (129 mg, 0.58 mmol). The resulting reaction was allowed to stir at room temperature for 2 hours. The reaction mixture was then diluted with 50 mL water, resulting in formation of a precipitate. The reaction mixture was filtered, and the collected solid was dried *in vacuo* to provide compound **16b,** which was used without further purification. MS: *m*/*z* 299.0 = [M+H].

### Step B - synthesis of compound 16c

Compound 16c was made using the methods described in Example 1, substituting the appropriate reactants and/or reagents. MS: *m*/*z* 313.0 = [M+H].

### Step C - synthesis of compound 16d

Compound 16d was made using the method described in Example 3, step B, substituting the appropriate reactants and/or reagents. MS: *m*/*z* 245.1 = [M+H].

### Step D - synthesis of compound 16e

Compound 16e was made using the method described in Example 3, step E, substituting the appropriate reactants and/or reagents. MS: *m*/*z* 231.1 = [M+H].

### Step E - synthesis of compound 16f

To a solution of compound **16e** (200 mg, 0.87 mmol) in DMF (5 mL) was added 1-propanephosphonic anhydride (691 mg, 2.2 mmol), triethylamine (0.36 mL, 2.6 mmol), and (4-chlorophenyl)methanamine (148 mg, 1.0 mmol). The resulting reaction was allowed to stir at room temperature for 16 hours, diluted with 50 mL water, and extracted with ethyl acetate (2 x 50 mL). The combined organic extracts were dried over sodium sulfate, filtered, concentrated *in vacuo,* and resulting residue was purified by silica gel chromatography eluting with 0-20% EtOAc in petroleum ether to provide compound **16f** as a solid. MS: *m*/*z* 400.1 = [M+H].

### Step F - synthesis of compound 16

To a solution of compound **16f** (50 mg, 0.14 mmol) in MeOH (5 mL) was added acetic acid (0.040 mL, 0.71 mmol), morpholine (0.018 mL, 0.21 mmol), and sodium cyanoborohydride (27 mg, 0.42 mmol). The resulting reaction was heated to 95 °C and allowed to stir at this temperature for 16 hours, then the reaction mixture was concentrated *in vacuo,* and the resulting residue was diluted with 10 mL water and extracted with ethyl acetate (2 x 10 mL). The combined organic extracts were washed with 5 mL saturated sodium bicarbonate, dried over sodium sulfate, filtered, and concentrated *in vacuo.* The residue obtained was purified using preparative HPLC (reverse-phase C-18), eluting with acetonitrile/water + 0.1% formic acid 5-100%. The racemic product was resolved using chiral supercritical fluid chromatography, using a Lux C3 column, and eluted with 40% MeOH in CO₂ with 20 mM ammonia added, to provide compound **16.** MS: *m*/*z* 426.4 = [M+H]. ¹H NMR (500 MHz, methanol-*d*₄) δ 7.54 (d, *J* = 8.7 Hz, 1H), 7.46 (d, *J* =8.4 Hz, 1H), 7.40 - 7.33 (m, 4H), 4.44 - 4.41 (m, 1H), 4.14 (s, 3H), 3.71-3.67 (m, 4H), 3.45- 3.40 (m, 2H), 2.61-2.57(m, 2H), 2.48-2.43 (m, 2H), 2.30-2.29 (m, 1H), 2.19-2.17 (m, 2H).

The following compound of the present invention was made using methodology described in this Example substituting the appropriate reactants and/or reagents: 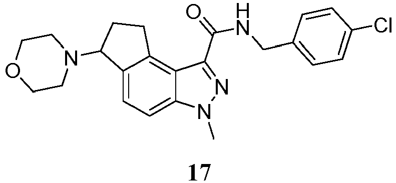 MS [M+H] = 426.4

### Example 13

### Preparation of Compound 18

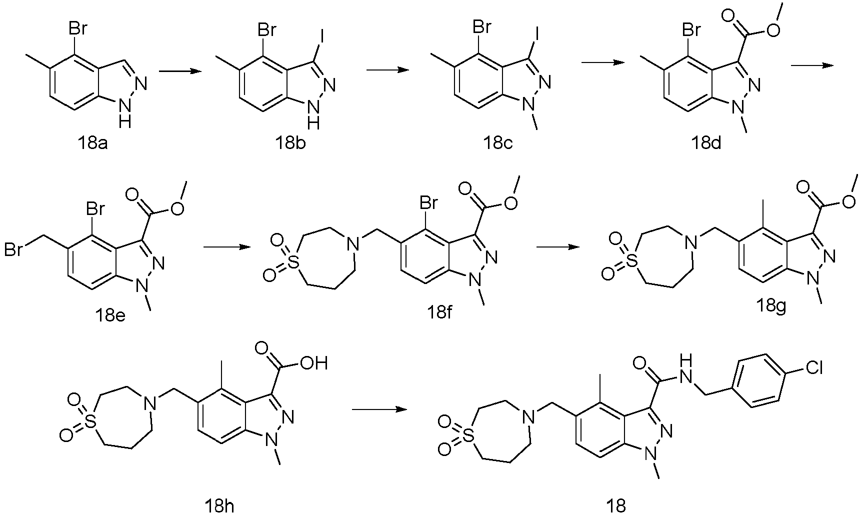

### Step A - synthesis of compound 18b

To a solution of compound **18a** (1.0 g, 4.7 mmol) in DMF (10 mL) was added potassium hydroxide, and the resulting reaction was allowed to stir at room temperature for 10 minutes. Iodine (1.443 g, 5.69 mmol) was added, and the reaction was allowed to stir at room temperature for 4 hours. The reaction mixture was quenched with saturated sodium metabisulfite (30 mL), and further diluted with water (30 mL). The resulting solution was allowed to stir for 15 minutes, then the pH was adjusted to pH 4 using 1.5N HCl. The acidified solution was filtered, and the collected solid was washed with water, and dried *in vacuo* to provide compound **18b** as a solid. MS: *m*/*z* 335.0 = [M+H].

### Step B - synthesis of compound 18c

To a solution of compound **18b** (1.5g, 4.5 mmol) in DMF (10 mL) was added cesium carbonate (1.53 g, 4.7 mmol). After 10 minutes, methyl iodide (0.28 mL, 4.5 mmol) was added dropwise, and the resulting reaction was allowed to stir at room temperature for 2 hours. The reaction mixture was quenched with ice-water and extracted into EtOAc (3 x 50 mL). The combined organic extracts were washed with water (3 x 30 mL), brine (50 mL), filtered, dried over sodium sulfate, and concentrated *in vacuo.* The resulting residue was purified by silica gel chromatography eluting with 0-5% EtOAc in petroleum ether to provide compound **18c** as a solid. MS: *m*/*z* 353.2 = [M+H].

### Step C - synthesis of compound 18d

Compound 118d was made using the method described in Example 3, step B, substituting the appropriate reactants and/or reagents. MS: *m*/*z* 285.2 = [M+H].

### Step D - synthesis of compound 18e

To a solution of compound **18d** (100 mg, 0.35 mmol) in carbon tetrachloride (10 mL) was added N-bromosuccinimide (69.2 mg, 0.39 mmol), and azobisisobutyronitrile (11.60 mg, 0.071 mmol). The resulting reaction was heated to 77°C, and allowed to stir at this temperature for 9 hours. The reaction mixture was then concentrated *in vacuo,* and the resulting residue was diluted with DCM (60 mL), washed with water (20 mL), brine (20 mL), dried over sodium sulfate, and concentrated *in vacuo* to provide compound **18e,** which was used without further purification. MS: *m*/*z* 363.0 = [M+H].

### Step E - synthesis of compound 18f

To a solution of compound **18e** (165 mg, 0.46 mmol) in acetonitrile (10 mL) was added DIEA (0.32 mL, 1.8 mmol), followed by 1,4-thiazepane 1,1-dioxide, HCl (72 mg, 0.39 mmol). The resulting reaction was allowed to stir at room temperature for 18 hours, then the reaction mixture was concentrated *in vacuo,* diluted with EtOAc (80 mL), and washed with water (20 mL). The aqueous layer was extracted with EtOAc (2 x 20 mL), and the combined organic extracts were washed with brine (30 mL), dried over sodium sulfate, and concentrated *in vacuo.* The resulting residue was purified by silica gel chromatography eluting with 0-85% EtOAc in petroleum ether to provide compound **18f** as a solid. MS: *m*/*z* 432.2 = [M+H].

### Step F - synthesis of compound 18g

To a microwave-safe vial was added compound **18f** (160 mg, 0.37 mmol), trimethylboroxine (93 mg, 0.74 mmol), 1M potassium phosphate (0.93 mL, 0.93 mmol), and 1,4-dioxane (6 mL). The resulting solution was sparged with nitrogen for 10 minutes. To the reaction was added [1,1'-bis(diphenylphosphino)ferrocene]dichloropalladium(II) methylene chloride complex (12.15 mg, 0.015 mmol), and the vial was re-sealed. The resulting reaction was heated to 90 °C, and allowed to stir at this temperature for 20 hours. The reaction mixture was then diluted with EtOAc (50 mL), washed sequentially with water (30 mL) and brine (20 mL), dried over sodium sulfate, filtered, and concentrated *in vacuo.* The resulting residue was purified by silica gel chromatography eluting with 0-2% MeOH in DCM to provide compound **18g** as a solid. MS: *m*/*z* 366.2 = [M+H].

### Step G - synthesis of compound 18h

Compound 18h was made using the method described in Example 3, step E, substituting the appropriate reactants and/or reagents. MS: *m*/*z* 352.2 = [M+H].

### Step H - synthesis of compound 18

Compound **18** was made using the method described in Example 12, step E, substituting the appropriate reactants and/or reagents. MS: *m*/*z* 475.2 = [M+H]. ¹H NMR (400 MHz, Methanol-*d*₄) δ 9.02 (t, *J* = 6.2 Hz, 1H), 7.49 - 7.38 (m, 6H), 4.48 (d, *J* = 6 Hz, 2H), 4.06 (s, 3H), 3.79 (s, 2H), 3.23 - 3.20 (m, 2H), 3.13 - 3.12 (m, 2H), 2.87 - 2.84 (m, 2H), 2.78-2.77 (m, 2H). 2.67 (s, 3H), 1.94 - 1.91 (m, 2H).

### Example 14

### Preparation of Compound 19

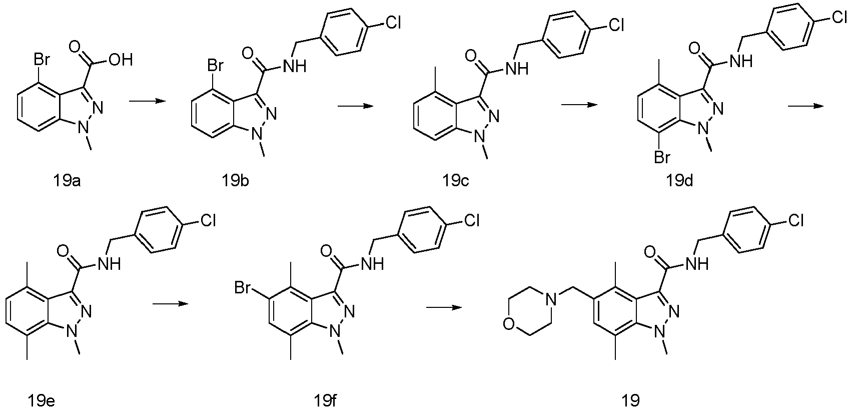

### Step A - synthesis of compound 19b

Compound 19b was made using the method described in Example 12, step E, substituting the appropriate reactants and/or reagents. MS: *m*/*z* 378.0 = [M+H].

### Step B - synthesis of compound 19c

To a solution of compound **19b** (1.30 g, 3.43 mmol), and bis(tri-t-butylphosphine) palladium(0) (175 mg, 0.34 mmol) in THF (17 mL) was added a solution of 2 M methylzinc(II) chloride in THF (2.10 mL, 4.12 mmol). The resulting reaction was allowed to stir at room temperature for 30 minutes, then diluted with water (10 mL). The organic layer was collected, dried over sodium sulfate, filtered, concentrated *in vacuo,* and the resulting residue was purified by silica gel chromatography using a 40g Redisep Gold column, and eluted with 0-40% EtOAc/hexanes to provide compound **19c** as a solid. MS: *m*/*z* 314.1 = [M+H].

### Step C - synthesis of compound 19d

To a solution of compound **19c** (1.00 g, 3.19 mmol) in chloroform (32 mL) was added bromine (0.49 mL, 9.56 mmol). The resulting reaction was allowed to stir at room temperature for 1 hour, then washed with saturated sodium thiosulfate (10 mL). The organic layer was collected, dried over sodium sulfate, filtered, and concentrated *in vacuo.* The resulting residue was purified by silica gel chromatography using a 120g Redisep Gold column, and eluted with 0-40% EtOAc/hexanes to provide compound **19d** as a solid. MS: *m*/*z* 394.0 = [M+H].

### Step D - synthesis of compound 19e

Compound 19e was made using the method described in step B, substituting the appropriate reactants and/or reagents. MS: *m*/*z* 328.2 = [M+H].

### Step E - synthesis of compound 19f

Compound 19f was made using the method described in step C above, substituting the appropriate reactants and/or reagents. MS: *m*/*z* 408.1 = [M+H].

### Step F - synthesis of compound 19

To microwave-safe vial containing a solution of compound **19f** (25 mg, 0.061 mmol), chloro[(di(1-adamantyl)-n-butylphosphine)-2-(2-aminobiphenyl)]palladium(II) (4.11 mg, 6.15 µmol), and 4-trifluoroboratomethyl-morpholine (38.2 mg, 0.184 mmol) in THF (615 µl) was added 1 M cesium carbonate (180 µl, 0.18 mmol). The vial was capped and heated to 120 °C, and the resulting reaction was allowed to stir at this temperature for 2 hours. The reaction mixture was then concentrated under a stream of nitrogen, and the resulting residue was purified using preparative HPLC (reverse-phase C-18), eluting with acetonitrile/water + 0.1% TFA 5-95% to provide compound **19.** MS: *m*/*z* 427.3 = [M+H]. ¹H NMR (700 MHz, DMSO-*d*₆) δ 9.08 (t, *J =* 6.1 Hz, 1H), 7.44 - 7.36 (m, 4H), 7.27 (s, 1H), 4.47 (d, *J* = 6.2 Hz, 2H), 4.44 (s, 2H), 4.31 (s, 3H), 3.99 - 3.91 (m, 3H), 3.70 - 3.60 (m, 2H), 3.31 - 3.18 (m, 4H), 2.73 (s, 3H), 2.63 (s, 3H).

### Example 15

### Preparation of Compound 20

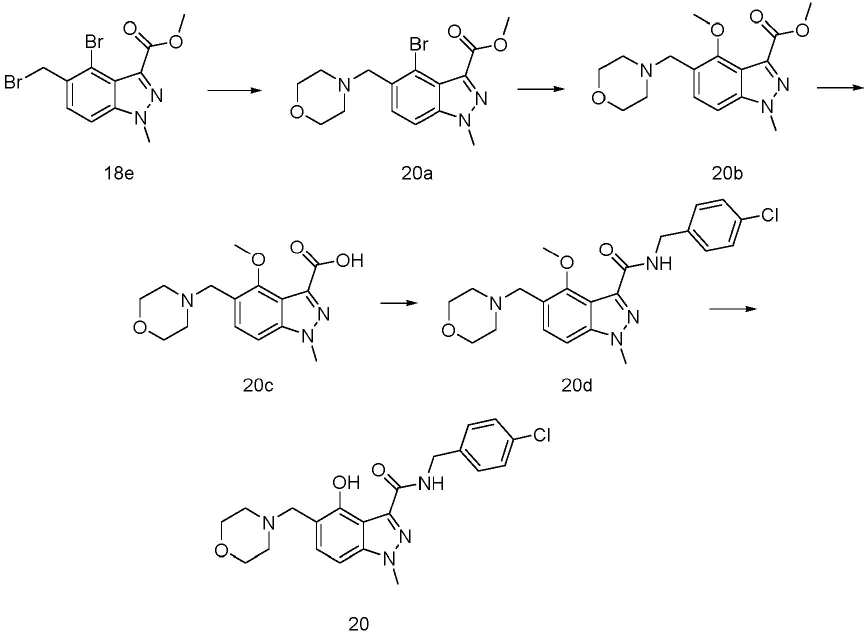

### Step A - synthesis of compound 20a

Compound 20a was made using the method described in Example 18, step E, substituting the appropriate reactants and/or reagents. MS: *m*/*z* 370.0 = [M+H].

### Step B - synthesis of compound 20b

To a solution of sodium methoxide (0.035 g, 0.65 mmol) in methanol (2 mL) was added copper(I) chloride (1.29 mg, 0.013 mmol), methyl formate (7.83 mg, 0.13 mmol), and compound **20a** (120 mg, 0.33 mmol). The resulting reaction was heated to 115°C, and allowed to stir at this temperature for 24 hours. The reaction mixture was then cooled to room temperature, diluted with water (5 mL), and extracted with dichloromethane (2 x 5 mL). The combined organic extracts were washed with brine, dried over sodium sulfate, filtered, and concentrated *in vacuo* to provide compound **20b,** which was used without further purification. MS: *m*/*z* 320.2 = [M+H].

### Step C - synthesis of compound 20c

Compound 20c was made using the method described in Example 3, step E, substituting the appropriate reactants and/or reagents. MS: *m*/*z* 306.2 = [M+H].

### Step D - synthesis of compound 20d

Compound 20d was made using the method described in Example 12, step E, substituting the appropriate reactants and/or reagents. MS: *m*/*z* 429.2 = [M+H].

### Step E - synthesis of compound 20

A solution of compound **20d** (30.0 mg, 0.070 mmol) in dichloromethane (5 mL) was cooled to 0°C, and 1M boron tribromide in dichloromethane (66 µl, 0.70 mmol) was added. The resulting reaction was allowed to stir at room temperature for 7 days, then was cooled to 0 °C, quenched with saturated sodium thiosulfate (1 mL), and extracted with dichloromethane (5 mL). The organic extract was dried over sodium sulfate, filtered, concentrated *in vacuo,* and the resulting residue was purified using preparative HPLC (reverse-phase C-18), eluting with acetonitrile/water + 0.1% TFA 5-95% to provide compound **20** as a solid. MS: *m*/*z* 415.2 = [M+H]. ¹H NMR (400 MHz, MeOD) δ 9.49 (s, 1H), 7.46 - 7.34 (m, 5H), 7.17 - 7.14 (m, 1H), 4.63 (d, *J =* 5.6 Hz, 2H), 4.48 (s, 2H), 4.13 (s, 3H), 4.06 (d, *J=*12.8 Hz, 2H), 3.78 - 3.67 (m, 2H), 3.47 - 3.43 (m, 2H), 3.28 - 3.25 (m, 2H).

### Example 16

### Preparation of Compound 21

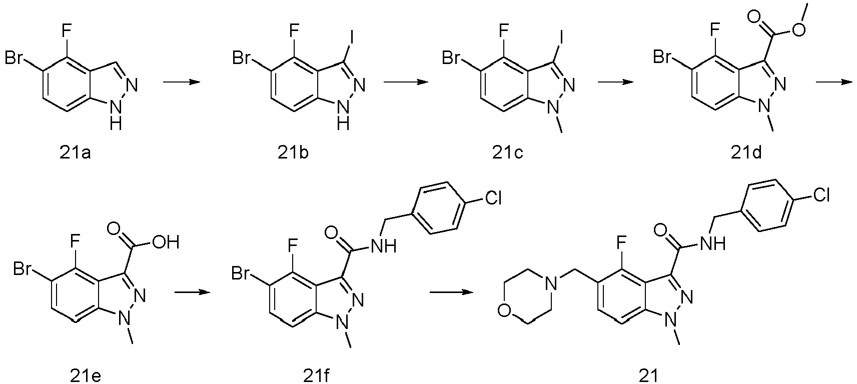

### Step A - synthesis of compound 21b

Compound 21b was made using the method described in Example 13, step A, substituting the appropriate reactants and/or reagents. MS: *m*/*z* 339.0 = [M+H].

### Step B - synthesis of compound 21c

Compound 21c was made using the method described in Example 13, step B, substituting the appropriate reactants and/or reagents. MS: *m*/*z* 354.9 = [M+H].

### Step C - synthesis of compound 21d

Compound 21d was made using the method described in Example 3, step B, substituting the appropriate reactants and/or reagents. ¹H NMR (400 MHz, Chloroform-d) δ 7.62 - 7.58 (m, 1H) 7.18 (d, *J* = 8.8 Hz, 1H), 4.18 (s, 3H), 4.06 (s, 3H).

### Step D - synthesis of compound 21e

Compound 21e was made using the method described in Example 3, step E, substituting the appropriate reactants and/or reagents. MS: *m*/*z* 273.2 = [M+H].

### Step E - synthesis of compound 21f

Compound 21f was made using the method described in Example 12, step E, substituting the appropriate reactants and/or reagents. MS: *m*/*z* 396.2 = [M+H].

### Step F - synthesis of compound 21

Compound **21** was made using the method described in Example 14, step F, substituting the appropriate reactants and/or reagents. MS: *m*/*z* 417.4 = [M+H]. 1H NMR (500 MHz, Methanol-d4) δ 7.56 - 7.49 (m, 1H), 7.42 (d, J = 8.6 Hz, 1H), 7.38 (d, J = 8.5 Hz, 2H), 7.33 (d, J = 8.5 Hz, 2H), 4.59 (s, 2H), 4.12 (s, 3H), 3.71 (d, J = 1.6 Hz, 2H), 3.69 - 3.64 (m, 4H), 2.53 - 2.49 (m, 4H).

### Example 17

### Preparation of Compound 22

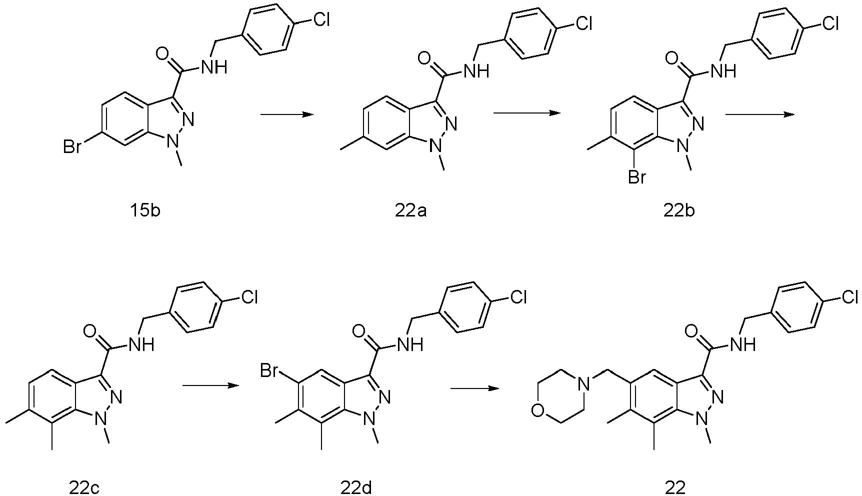

### Step A - synthesis of compound 22a

Compound 22a was made using the method described in Example 14, step B, substituting the appropriate reactants and/or reagents. MS: *m*/*z* 314.2 = [M+H].

### Step B - synthesis of compound 22b

Compound 22b was made using the method described in Example 14, step C, substituting the appropriate reactants and/or reagents. MS: *m*/*z* 394.1 = [M+H].

### Step C - synthesis of compound 22c

Compound 22c was made using the method described in Example 14, step B, substituting the appropriate reactants and/or reagents. MS: *m*/*z* 328.1 = [M+H].

### Step D - synthesis of compound 22d

Compound 22d was made using the method described in Example 14, step C, substituting the appropriate reactants and/or reagents. MS: *m*/*z* 408.1 = [M+H].

### Step E - synthesis of compound 22

Compound **22** was made using the method described in Example 14, step F, substituting the appropriate reactants and/or reagents. MS: *m*/*z* 427.3 = [M+H]. ¹H NMR (500 MHz, DMSO-*d*₆) δ 9.42 (br s, 1H), 9.02 (t, *J* = 6.2 Hz, 1H), 8.29 (s, 1H), 7.43 - 7.31 (m, 4H), 4.55 (d, *J* = 5.2 Hz, 2H), 4.45 (d, *J* = 6.2 Hz, 2H), 4.38 (s, 3H), 3.94 (d, *J* = 12.4 Hz, 2H), 3.69 - 3.57 (m, 2H), 3.30 - 3.22 (m, 4H), 2.72 (s, 3H), 2.44 (s, 3H).

### Example 18

### Preparation of Compound 23

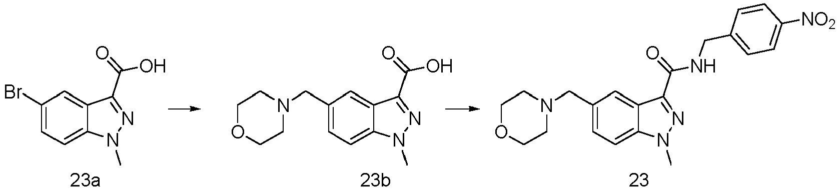

### Step A - synthesis of compound 23b

Compound 23b was made using the method described in Example 14, step F, substituting the appropriate reactants and/or reagents. MS: *m*/*z* 276.2 = [M+H].

### Step B - synthesis of compound 23

To a vial containing (4-nitrophenyl)methanamine hydrochloride (34.3 mg, 0.18 mmol) was added a solution of compound **23b** (25.0 mg, 0.09 mmol), and (benzotriazol-1-yloxy)tris(dimethylamino)phosphonium hexafluorophosphate (80.0 mg, 0.18 mmol) in dichloromethane (1.0 mL). DIEA (47.6 µL, 0.27 mmol) was added, and the resulting reaction was allowed to stir at room temperature for 6 hours. The reaction mixture was concentrated under a stream of nitrogen, and the resulting residue was purified using preparative HPLC (reverse-phase C-18), eluting with acetonitrile/water + 0.1% TFA 10-40% to provide compound **23** as a solid. MS: *m*/*z* 410.2 = [M+H]. ¹H NMR (500 MHz, DMSO-*d*₆) δ 9.24 (s, 1H), 8.38 (s, 1H), 8.21 (d, *J* = 8.7 Hz, 2H), 7.86 (d, *J* = 7.7 Hz, 1H), 7.61 (d, *J* = 8.6 Hz, 2H), 7.56 (d, *J* = 8.6 Hz, 1H), 4.61 (d, *J =* 6.2 Hz, 2H), 4.50 (s, 2H), 4.17 (s, 3H), 4.02 - 3.89 (m, 2H), 3.66 - 3.52 (m, 2H), 3.32 - 3.07 (m, 4H).

The following compounds of the present invention were made using methodology described in the Example above, substituting the appropriate reactants and/or reagents:

| **Compound** | **Structure** | **MS [M+H]** |
|---|---|---|
| **24** | 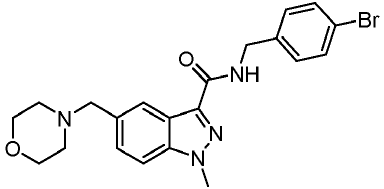 | 443.2 |
| **25** | 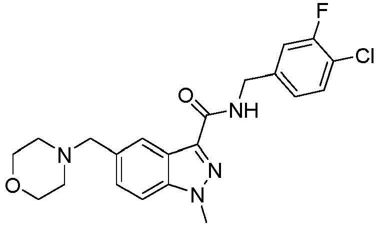 | 416.1 |
| **26** | 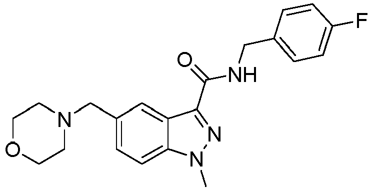 | 383.2 |

### Example 19

### Preparation of Compound 28

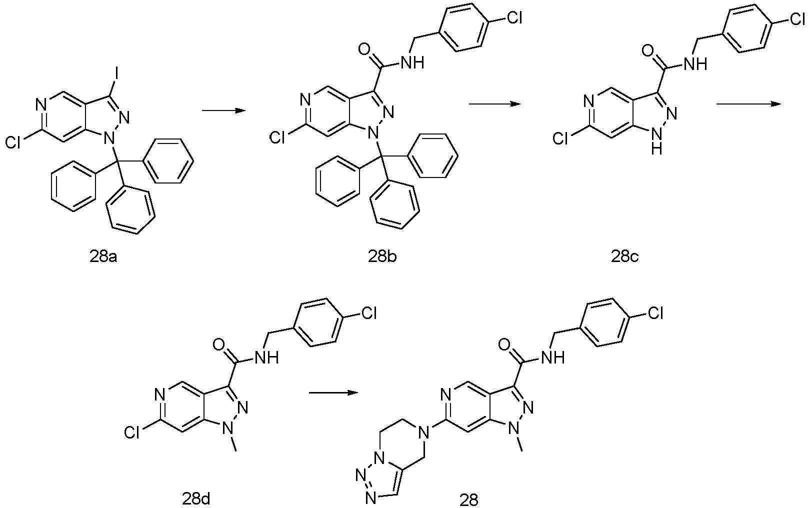

### Step A - synthesis of compound 28b

To a round bottom flask was added compound **28a** (100 mg, 0.19 mmol, prepared as described in International Publication No. WO 13/063214), methanol (2 mL), and DMSO (2 mL), followed by triethylamine (5.3 µl, 0.038 mmol). After degassing the reaction mixture for 10 minutes, palladium(II) acetate (8.61 mg, 0.038 mmol), and 1,3-bis(diphenylphosphino)propane (79 mg, 0.192 mmol) were added, and the reaction mixture was again degassed for 10 minutes with nitrogen. 4-(chlorophenyl)methanamine (81 mg, 0.58 mmol) was then added, and the reaction vessel was charged with CO gas. The resulting reaction was heated to 80 °C, and allowed to stir at this temperature for 16 hours, then the reaction mixture was diluted with water (30 mL), and extracted with EtOAc (2 x 30 mL). The combined organic extracts were dried over sodium sulfate, filtered, concentrated *in vacuo,* and the resulting residue was purified by silica gel chromatography eluting with 0-30% EtOAc/petroleum ether to provide compound **28b.** MS: *m*/*z* = 563.4 [M+H].

### Step B - synthesis of compound 28c

A solution of compound **28b** (300 mg, 0.53 mmol) in DCM (5 mL) was cooled to 0 °C, then TFA (2.46 mL, 31.9 mmol), and triethylsilane (0.21 mL, 1.33 mmol) were added. The resulting reaction was allowed to stir at room temperature for 2 hours, then was concentrated *in vacuo.* The residue obtained was washed with diethyl ether (10 mL) to provide compound **28c** as a solid, which was used without further purification. MS: *m*/*z* = 321.2 [M+H].

### Step C - synthesis of compound 28d

To a solution of compound **28c** (100 mg, 0.311 mmol) in DMF (2 mL) was added potassium carbonate (43.0 mg, 0.31 mmol). The resulting solution was allowed to stir for 10 minutes, then methyl iodide (0.019 mL, 0.31 mmol) was added. The resulting reaction was allowed to stir at room temperature for 2 hours, then was diluted with water (5 mL), upon which a precipitate formed. The solution was filtered, and the collected solid was dried *in vacuo* to provide compound **28d** as a solid that was used without further purification. MS: *m*/*z* = 335.2 [M+H].

### Step D - synthesis of compound 28

Compound **28** was made using the method described in Example 10, step B, substituting the appropriate reactants and/or reagents. MS: *m*/*z* 423.4 = [M+H]. ¹H NMR (500 MHz, DMSO-d6) δ 9.08 (t, J = 6.2 Hz, 1H), 9.03 (s, 1H), 7.69 (s, 1H), 7.41 - 7.33 (m, 4H), 7.09 (s, 1H), 4.93 (s, 2H), 4.51 (t, J = 5.3 Hz, 2H), 4.45 (d, J = 5.7 Hz, 2H), 4.23 (t, J = 5.4 Hz, 2H), 4.03 (s, 3H).

### Example 20

### Preparation of Compound 29

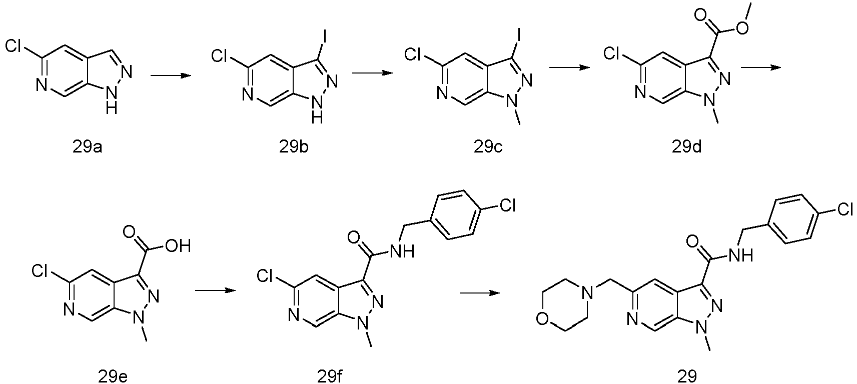

### Step A - synthesis of compound 29b

Compound 29b was made using the method described in Example 13, step A, substituting the appropriate reactants and/or reagents. MS: *m*/*z* 278.4 = [M+H].

### Step B - synthesis of compound 29c

Compound 29c was made using the method described in Example 13, step B, substituting the appropriate reactants and/or reagents. MS: *m*/*z* 294.0 = [M+H].

### Step C - synthesis of compound 29d

Compound 29d was made using the method described in Example 13, step C, substituting the appropriate reactants and/or reagents. MS: *m*/*z* 226.2 = [M+H].

### Step D - synthesis of compound 29e

Compound 29e was made using the method described in Example 3, step E, substituting the appropriate reactants and/or reagents. MS: *m*/*z* 212.2 = [M+H].

### Step E - synthesis of compound 29f

Compound 29f was made using the method described in Example 12, step E, and substituting the appropriate reactants and/or reagents. MS: *m*/*z* 335.2 = [M+H].

### Step F - synthesis of compound 29

To a pressure tube was added compound **29f** (210 mg, 0.63 mmol), tetrahydrofuran (15 mL), potassium (morpholin-4-yl)methyltrifluoroborate (195 mg, 0.94 mmol), cesium carbonate (20.4 mg, 0.063 mmol), water (1 mL), palladium(II) acetate (141 mg, 0.63 mmol) mmol), and 2-dicyclohexylphosphino-2',4',6'-triisopropylbiphenyl (59.7 mg, 0.13 mmol). The resulting reaction was sparged with nitrogen, heated to 100°C, and allowed to stir at this temperature for 48 hours. The reaction mixture was then cooled to room temperature, diluted with water (60 mL), and extracted with EtOAc (2 x 60 mL). The combined organic extracts were sequentially washed with water (50 mL) and brine (50 mL), then dried over sodium sulfate, filtered, and concentrated *in vacuo.* The residue obtained was purified using preparative HPLC (reverse-phase C-18), eluting with acetonitrile/water + 0.1% formic acid to provide compound **29** as a solid. MS: *m*/*z* 400.4 = [M+H]. ¹H NMR (400 MHz, Methanol-*d*₄) δ 9.13 (s, 1H), 8.23 (s, 1H), 7.41 - 7.34 (m, 4H), 4.62 (s, 2H), 4.30 (s, 3H), 3.91 (s, 2H), 3.76 - 3.74 (m, 4H), 2.57 - 2.55 (m, 4H).

### Example 21

### Preparation of Compound 30

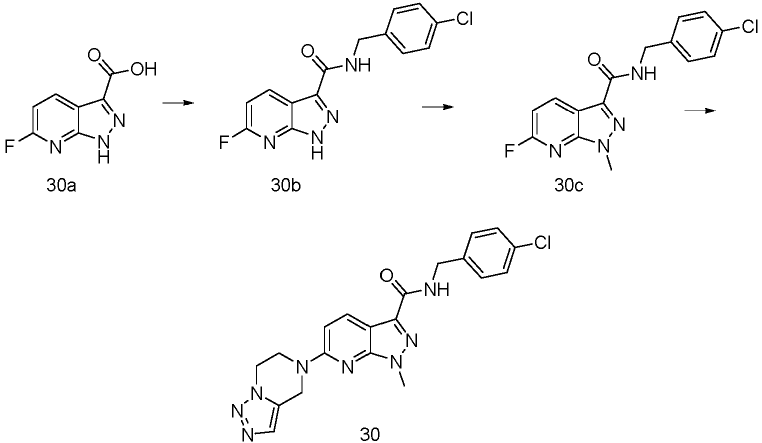

### Step A - synthesis of compound 30b

Compound 30b was made using the method described in Example 12, step E, substituting the appropriate reactants and/or reagents. MS: *m*/*z* 319.2 = [M+H].

### Step B - synthesis of compound 30c

Compound 30c was made using the method described in Example 1, step H, substituting the appropriate reactants and/or reagents. MS: *m*/*z* 319.2 = [M+H].

### Step C - synthesis of compound 30

To a microwave-safe vial was added compound **30c** (25 mg, 0.078 mmol), cesium carbonate (128 mg, 0.39 mmol), 4,5,6,7-tetrahydro-[1,2,3]triazolo[1,5-a]pyrazine hydrochloride (37.8 mg, 0.24 mmol), and DMSO (780 µL). The resulting reaction was heated to 125 °C, and allowed to stir at this temperature for about 15 hours, then the reaction mixture was filtered. The filtrate was directly purified using preparative HPLC (reverse-phase C-18), eluting with acetonitrile/water + 0.1% TFA 5-95% to provide compound **30** as an oil. MS: *m*/*z* 423.3 = [M+H]. ¹H NMR (500 MHz, DMSO-*d*₆) δ 8.98 (t, *J* = 6.2 Hz, 1H), 8.24 (d, *J* = 9.0 Hz, 1H), 7.70 (s, 1H), 7.42 - 7.29 (m, 4H), 7.12 (d, *J* = 9.0 Hz, 1H), 5.04 (s, 2H), 4.56 - 4.50 (m, 2H), 4.45 - 4.41 (m, 2H), 4.28 - 4.22 (m, 2H), 4.00 (s, 2H), 3.00 (s, 3H).

### Example 22

### Preparation of Compound 31

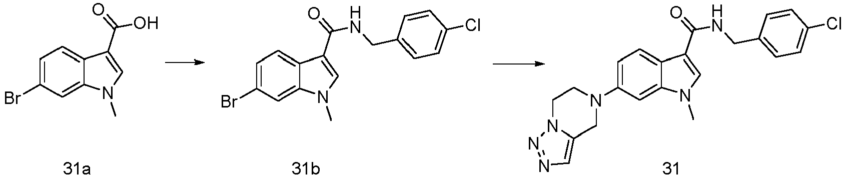

### Step A - synthesis of compound 31b

Compound 31b was made using the method described in Example 1, step F, substituting the appropriate reactants and/or reagents. MS: *m*/*z* 377.3 = [M+H].

### Step B - synthesis of compound 31

Compound 31 was made using the method described in Example 1, step G, substituting the appropriate reactants and/or reagents. MS: *m*/*z* 421.2 = [M+H]. ¹H NMR (500 MHz, Methanol-*d*₄) δ 8.03 (d, *J* = 8.6 Hz, 1H), 7.73 (s, 1H), 7.65 (s, 1H), 7.38 - 7.29 (m, 4H), 7.11 - 7.06 (m, 2H), 4.58 (s, 2H), 4.57 - 4.52 (m, 4H), 3.85 (s, 2H), 3.81 (s, 3H).

The following compound of the present invention was made using methodology described in the Example above, substituting the appropriate reactants and/or reagents:

### Example 23

### Preparation of Compound 33

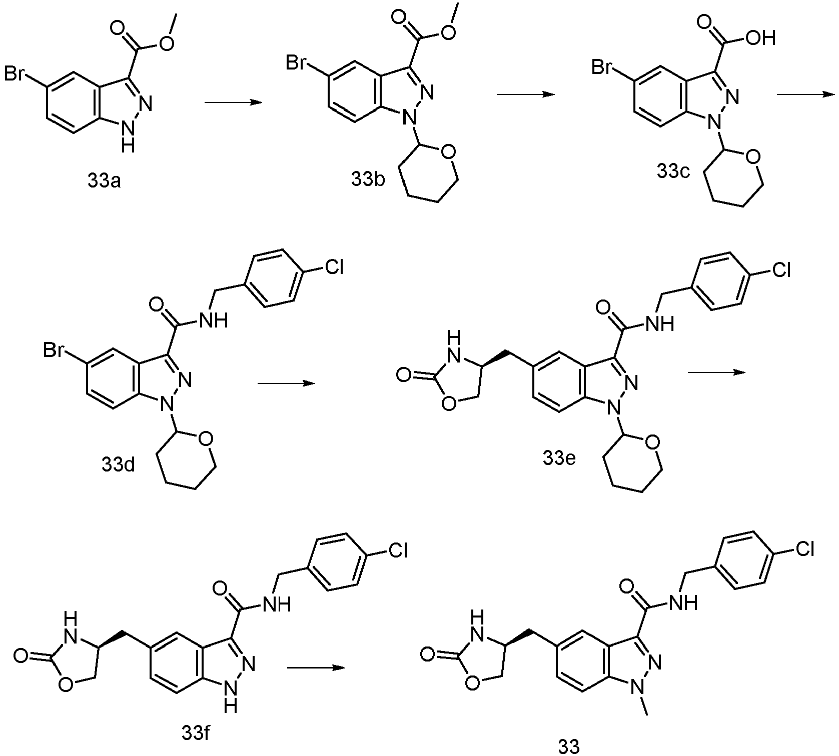

### Step A- synthesis of compound 33b

Compound 33b was made using the method described in Example 4, step B, substituting the appropriate reactants and/or reagents. MS: *m*/*z* = 339.1 [M+H].

### Step B- synthesis of compound 33c

Compound 33c was made using the method described in Example 3, step E, and substituting the appropriate reactants and/or reagents. MS: *m*/*z* = 327.0 [M+H].

### Step C- synthesis of compound 33d

Compound 33d was made using the method described in Example 1, step F, substituting the appropriate reactants and/or reagents. MS: *m*/*z* = 450.2 [M+H].

### Step D - synthesis of compound 33e

To an 8-mL vial under an argon atmosphere was added compound 33d (500 mg, 1.11 mmol), (R)-4-(bromomethyl)oxazolidin-2-one (301 mg, 1.67 mmol), nickel(II) chloride ethylene glycol dimethyl ether complex (2.45 mg, 0.011 mmol), [4,4'-*Bis*(1,1-dimethylethyl)-2,2'-*bipyridine-N1,N1']bis[3,5-difluoro-2-[5-(trifluoromethyl)-2-pyridinyl-N]phenyl-C]Iridium(III)* hexafluorophosphate, [Ir{dF(CF₃)ppy}₂(dtbpy)]PF₆ (25.00 mg, 0.022 mmol), sodium carbonate (236 mg, 2.228 mmol), 1,1,1,3,3,3-hexamethyl-2-(trimethylsilyl)trisilane (277 mg, 1.11 mmol), and 1,2-dimethoxyethane (5 mL). The resulting reaction was irradiated with blue LED light for 16 hours at ambient temperature. The reaction mixture was then quenched using saturated aqueous ammonium chloride (20 mL), and extracted with ethyl acetate (3 x 50 mL). The combined organic extracts were washed with brine (20 mL), dried over sodium sulfate, and filtered. The filtrate was concentrated *in vacuo,* and the resulting residue was purified by silica gel chromatography eluting with 50-100% EtOAc/petroleum ether to provide compound **33e** as a solid. MS: *m*/*z* = 469.7 [M+H].

### Step E - synthesis of compound 33f

A solution of compound **33e** (220 mg, 0.47 mmol) in DCM (10 mL) was cooled to 0 °C, and TFA (4 mL), and triethylsilane (70 mg, 0.60 mmol) were added. The resulting reaction was allowed to warm to room temperature, then stirred at this temperature for 3 hours, and the reaction mixture was then concentrated *in vacuo.* The resulting residue was purified by silica gel chromatography eluting with 0-10% MeOH/DCM to provide compound **33f** as a solid. MS: *m*/*z* = 385.3 [M+H].

### Step F - synthesis of compound 33

Compound **33** was made from compound 33f, using the method described in Example 1, step H substituting the appropriate reactants and/or reagents. MS: *m*/*z* 399.1 = [M+H]. ¹H NMR (400 MHz, Methanol-*d*₄) δ 8.10 (s, 1H), 7.60 (d, *J* = 8.7 Hz, 1H), 7.44 - 7.32 (m, 6H), 4.62 (s, 2H), 4.49 - 4.39 (m, 1H), 4.29 - 4.18 (m, 2H), 4.15 (s, 3H), 3.06 - 3.00 (m, 2H).

### Example 24

### Preparation of Compound 34

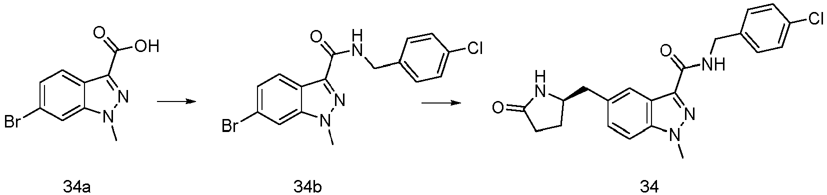

### Step A- synthesis of compound 34b

Compound 34b was made using the method described in Example 1, step F substituting the appropriate reactants and/or reagents. MS: *m*/*z* = 380.0 [M+H].

### Step B- synthesis of compound 34

Compound **34** was made using the method described in Example 23, step D substituting the appropriate reactants and/or reagents. MS: *m*/*z* = 397.1 [M+H]. ¹H NMR (600 MHz, DMSO-*d*₆) δ 8.96 (s, 1H), 7.96 (s, 1H), 7.75 (s, 1H), 7.64 (d, *J* = 8.5 Hz, 1H), 7.39 - 7.29 (m, 5H), 4.43 (d, *J* = 6.2 Hz, 2H), 4.09 (s, 3H), 3.84 - 3.72 (m, 1H), 2.95 - 2.88 (m, 1H), 2.80 - 2.70 (m, 1H), 2.02 - 1.94 (m, 2H), 1.94 - 1.86 (m, 1H), 1.70 - 1.61 (m, 1H).

### Example 25

### HSV CPE assay

Vero cells were maintained at 37°C/5% CO₂/90% relative humidity in Dulbecco's Modified Eagle's Medium with 10% fetal bovine serum, 2.0 nM L-glutamine, 100 units/mL penicillin and 100ug/mL streptomycin. For this assay, Vero cells (maintained at 37°C/5% CO₂/90% relative humidity in Dulbecco's Modified Eagle's Medium with 10% fetal bovine serum, 2.0 nM L-glutamine, 100 units/mL penicillin and 100 µg/mL streptomycin) were seeded in 96-well microtiter plates and incubated for about 15 hours. They were then infected with HSV-1, strain F, or with HSV-2, strain G, at a multiplicity of infection known to result in 85-95% loss of cell viability during the assays, in the same media with 2% fetal bovine serum. Test compounds were dissolved in DMSO, and 6 point serial 10-fold dilutions in DMSO were prepared. Test compounds were added to infected cells, and the plates were incubated at 37°C/5% CO2/90% relative humidity for 5 days. Each plate contains cell control wells (cells only), virus control wells (cells plus virus), drug colorimetric control wells (drug only), background control wells (media only) and experimental wells (drug plus cells plus virus). Cytoprotection was assessed by the addition of Celltiter^{®}96 Reagent (Promega, Madison, WI), according to manufacturer's recommendations. % cytopathic effect (CPE) reduction was calculated and IC₅₀ (concentration inhibition virus replication by 50%) was reported.

### Example 26

### Viral qPCR assays

MRC5 and Vero cells were obtained from ATCC and were maintained at 37°C/5% CO₂/90% relative humidity in Minimal Essential Medium with 10% fetal bovine serum, 2.0 nM L-glutamine, 100 units/mL penicillin and 100ug/mL streptomycin. Assay plates were prepared by dispensing compounds dissolved in DMSO into wells of 384 well collagen-coated plates using an ECHO acoustic dispenser. Each test compound was tested in a 10-point, serial 3-fold dilution. Controls included uninfected cells and infected cells treated only with DMSO. Assays were initiated by mixing selected cells, in suspension, with virus, and dispensing 50µl/well infected cells to pre-plated compounds. Plates were incubated at 37°C/5% CO₂/90% relative humidity for ~ 72 hours to permit genomic replication, and infected cells were lysed by the addition of an equal volume of lysis buffer (10mM Tris-HCl, pH8, 50mM KCl, 2mM MgCl₂, 0.45% NP-40, 0.45% Tween-20, and 100µg/mL proteinase K). An aliquot of the lysate was then transferred to a 384-well PCR plate and incubated at 56 °C for 1 hour, and then at 95 °C for 10 minutes. Levels of a viral gene were measured in 10ul qPCR assays using TaqMan^{®} Gene Expression Master Mix (Applied Biosystems) and an 7900HT Fast Real-Time PCR System with 384-Well Block Module. 7-point, serial 10-fold dilutions of a plasmid standard were run on each plate to generate a standard curve, and genome copies numbers were calculated by plotting experimental Ct onto linear regression of the standard curve. Compound effects on viral genome copy number were normalized to the window defined by the controls. Calculated % effects were fit using a 4-parameter algorithm, and EC50 was reported.
HCMV: Strain AD169 was assayed in MRC-5 cells and was used at 0.05-0.1 pfu/cell. The assays were performed in either growth media or in the same media with 50% fetal bovine serum. Primer-probe set was Thermo Fisher Assay ID = AIFATFK.
HSV-1: Strain F was assayed in Vero or MRC5 cells and was used at 0.0005-0.004 pfu/cell in growth medium. Primer-probe set was Thermo Fisher Assay ID = AIBJZIB.
HSV-2: Strain G was assayed in Vero or MRC5 cells and was used at 0.004-0.4 pfu/cell in growth medium. Primer-probe set was Thermo Fisher Assay ID = AICSXOJ.

### Example 27

### CMV and VZV polymerase assays

Human cytomegalovirus and varicella zoster virus DNA polymerases were expressed via baculovirus vector in SF21 cells and purified. Heterodimeric nucleic acid substrate used in the herpesvirus polymerase reactions were generated by annealing a 59-mer template to a 17-mer digoxigenin-labeled primer. Polymerase (HCMV final concentration of 0.2nM; VZV final concentration of 0.4nM) was combined with an inhibitor compound or DMSO in assay buffer (10 mM HEPES, pH 7.5, 25 mM KCl, 25 mM NaCl, 5 mM MgCl₂, 5% glycerol, 0.67 mg/mL bovine serum albumin, and 1mM tris(2-carboxyethyl)phosphine)), and this mixture was preincubated for 30 minutes at room temperature in 384-well microtiter plates. The polymerization reaction was initiated by the addition of template/primer substrate (final concentration: 1.6 nM) and dNTPs (final concentration: 24 nM dCTP, 24 nMdGTP, 16nM dATP, 16 nM dTTP, and 0.8nM biotin-dUTP). After a 60 minute incubation period at 37°C, the reactions were terminated using quench buffer (25 mM HEPES pH 7.5, 100 mM NaCl, 0.25% Tween-20, 12 mM EDTA, and 1mg/mL bovine serum albumin). Incorporation of biotinylated UTP was detected with 2.5-5 µg/mL anti-DIG AlphaLISA acceptor beads and 5-10 µg/mL streptavidin AlphaLISA donor beads (PerkinElmer). Compound effects were normalized to the window defined by the controls (DMSO only and pre-quenched wells) and were fit using a 4-parameter algorithm to report an IC₅₀.

Illustrative compounds of the present invention were tested in one or more of the above assays and results are provided in the table below:

| **Compound** | **CMV^{a} (IC₅₀)** | **CMV Cell^{b} (EC₅₀)** | **VZV^{a} (IC₅₀)** | **HSV1 Cell^{b} (EC₅₀)** | **HSV2 Cell^{b} CPE(EC₅₀)** |
|---|---|---|---|---|---|
| **1** | 53 nM | 120 nM | 13 nM | 1600 nM | 2500 nM |
| **2** | 50 nM | 42 nM | 16 nM | 2100 nM | 1900 nM |
| **3** | 9.6 nM | 71 nM | 2.9 nM | 85 nM* | 170 nM* |
| **4** | 37 nM | 85 nM | 16 nM | 3100 nM | 2100 nM |
| **5** | 33 nM | 97 nM | 12 nM | >40000 nM | 610 nM |
| **6** | 45 nM | 110 nM | 19 nM | >40000 nM | 3700 nM |
| **7** | 22 nM | 120 nM | 5.1 nM | 120 nM | 200 nM |
| **8** | 103 nM | 150 nM | 62 nM | 6400 nM | 22000 nM |
| **10** | 41 nM | 460 nM | 12 nM | 250 nM | 590 nM |
| **11** | 23 nM | 93 nM | 3.7 nM | 480 nM | 700 nM |
| **12** | 7.1 nM | 300 nM | 1.6 nM | N/A | N/A |
| **13** | 17 nM | 320 nM | 5.0 nM | N/A | N/A |
| **14** | 54 nM | 870 nM | 12 nM | 340 nM | 570 nM |
| **15** | 25 nM | 930 nM | 14 nM | 850 nM | 930 nM |
| **16** | 260 nM | 970 nM | 38 nM | 1500 nM | 1600 nM |
| **17** | 150 nM | 1000 nM | 14 nM | 310 nM* | 350 nM* |
| **18** | 120 nM | 2900 nM | 160 nM | 6300 nM | 6100 nM |
| **19** | 280 nM | 6100 nM | 66 nM | 1800 nM | 5300 nM |
| **20** | 390 nM | >9900 nM | 66 nM | >40000 nM | >40000 nM |
| **21** | 480 nM | 7200 nM | 180 nM | 2200 nM | 3600 nM |
| **22** | 580 nM | 4300 nM | N/A | N/A | N/A |
| **23** | 990 nM | 12000 nM | 180 nM | N/A | N/A |
| **24** | 920 nM | >9900 nM | 220 nM | N/A | N/A |
| **25** | 620 nM | 5300 nM | 140 nM | 4100 nM | 5900 nM |
| **26** | 3300 nM | N/A | 740 nM | N/A | N/A |
| **27** | 16 nM | 94 nM | 5.1 nM | 180 nM* | 150 nM* |
| **28** | 810 nM | 7500 nM | 250 nM | N/A | N/A |
| **29** | 2400 nM | N/A | 1600 nM | N/A | N/A |
| **30** | 1600 nM | N/A | 3000 nM | N/A | N/A |
| **31** | 190 nM | N/A | 120 nM | N/A | N/A |
| **32** | 4.2 nM | 60 nM | 1.8 nM | 14000 nM | 13000 nM |
| **33** | 78 nM | 160 nM | 35 nM | 200 nM* | 110 nM* |
| **34** | 67 nM | 330 nM | 24 nM | 220 nM* | 240 nM* |

| | | | | | |
|---|---|---|---|---|---|
| **N/A** = **not available** **a = data generated using the assay described in Example 27** **b = data generated using the assay described in Example 25** *** - data generated using the assay described in Example 26** | | | | | |

### Uses of the Indazole Derivatives

The Indazole Derivatives are useful in human and veterinary medicine for treating or preventing a viral infection in a patient. In one embodiment, the Indazole Derivatives can be inhibitors of viral replication. In another embodiment, the Indazole Derivatives can be inhibitors of herpesvirus replication. Accordingly, the Indazole Derivatives are useful for treating viral infections, such as herpesvirus. In accordance with the invention, the Indazole Derivatives can be administered to a patient in need of treatment or prevention of a viral infection.

Accordingly, in one embodiment, the invention provides methods for treating or preventing a viral infection in a patient comprising administering to the patient an effective amount of at least one Indazole Derivative or a pharmaceutically acceptable salt thereof.

### Treatment or Prevention of Herpesvirus Infection

The Indazole Derivatives are useful in the inhibition of herpesvirus replication, the treatment of herpesvirus infection and/or reduction of the likelihood or severity of symptoms of herpesvirus infection and the inhibition of herpesvirus viral replication and/or herpesvirus viral production in a cell-based system. For example, the Indazole Derivatives are useful in treating infection by herpesvirus after suspected past exposure to herpesvirus by such means as blood transfusion, exchange of body fluids, bites, accidental needle stick, or exposure to patient blood during surgery or other medical procedures.

Accordingly, in one embodiment, the invention provides a method for treating herpesvirus infection in a patient, the method comprising administering to the patient an effective amount of at least one Indazole Derivative or a pharmaceutically acceptable salt thereof.

In one embodiment, the herpesvirus being treated or prevented is of the family α-herpesviridae. Herpesviruses of the family α-herpesviridae include, but are not limited to, herpes simplex virus 1 (HSV-1), herpes simplex 2 (HSV-2), and varicella zoster virus (VZV).

In another embodiment, the herpesvirus being treated or prevented is of the family β-herpesviridae. Herpesviruses of the family β-herpesviridae include, but are not limited to, human cytomegalovirus (CMV), human herpesvirus 6 (HHV6), and human herpesvirus 7 (HHV7).

In another embodiment, the herpesvirus being treated or prevented is of the family γ-herpesviridae. Herpesviruses of the family γ-herpesviridae include, but are not limited to, Epstein-Barr virus (EBV), human herpesvirus 4 (HHV4), and Kaposi's sarcoma-associated herpesvirus (KHSV), also known as human herpesvirus 8 (HHV8).

In one embodiment, the herpesvirus being treated or prevented is HSV-1.

In another embodiment, the herpesvirus being treated or prevented is HSV-2.

In another embodiment, the herpesvirus being treated or prevented is VZV.

In still another embodiment, the herpesvirus being treated or prevented is CMV.

In another embodiment, the herpesvirus being treated or prevented is HHV6.

In yet another embodiment, the herpesvirus being treated or prevented is HHV7.

In another embodiment, the herpesvirus being treated or prevented is EBV.

In a further embodiment, the herpesvirus being treated or prevented is HHV4.

In another embodiment, the herpesvirus being treated or prevented is KSHV.

In a specific embodiment, the amount administered is effective to treat or prevent infection by herpesvirus in the patient. In another specific embodiment, the amount administered is effective to inhibit herpesvirus viral replication and/or viral production in the patient.

The Indazole Derivatives are also useful in the preparation and execution of screening assays for antiviral compounds. Furthermore, the Indazole Derivatives are useful in establishing or determining the binding site of other antivirals to the herpesvirus polymerase.

The compositions and combinations of the present invention can be useful for treating a patient suffering from infection related to any herpesvirus infection. Herpesvirus types may differ in their antigenicity, level of viremia, severity of disease produced, and response to therapy. See Poole et al., Clinical Therapeutics, 40:8 (2018), 1282-1298.

### Combination Therapy

In another embodiment, the present methods for treating or preventing herpesvirus infection can further comprise the administration of one or more additional therapeutic agents which are not Indazole Derivatives.

In one embodiment, the additional therapeutic agent is an antiviral agent. In another embodiment, the additional therapeutic agent is an anti-herpes agent.

Anti-herpes agents useful in the present compositions and methods include, but are not limited to, nucleoside polymerase inhibitors, such as acyclovir, valaciclovir, famciclovir, penciclovir, cidofovir, brincidofovir (CMX-001), valmanciclovir, ganciclovir, valganciclovir, and N-methanocarbathymidine (N-MCT); pyrophosphate polymerase inhibitors, such as foscarnet; CMV terminase inhibitors, such as letermovir; viral kinase inhibitors, such as maribavir; and helicase-primase inhibitors, such as pritelivir (AIC-316) and amenamevir (ASP-2151).

In another embodiment, the additional therapeutic agent is an immunomodulatory agent, such as an immunosuppressive agent. Immunosuppressant agents useful in the present compositions and methods include, but are not limited to, cytotoxic agents, such as cyclophosphamide and cyclosporin A; corticosteroids, such as hydrocortisone and dexamethasone, and non-steroidal anti-inflammatory agents (NSAID).

Accordingly, in one embodiment, the present invention provides methods for treating a herpesvirus infection in a patient, the method comprising administering to the patient: (i) at least one Indazole Derivative, or a pharmaceutically acceptable salt thereof, and (ii) at least one additional therapeutic agent that is other than an Indazole Derivative, wherein the amounts administered are together effective to treat or prevent the herpesvirus infection.

When administering a combination therapy of the invention to a patient, therapeutic agents in the combination, or a pharmaceutical composition or compositions comprising therapeutic agents, may be administered in any order such as, for example, sequentially, concurrently, together, simultaneously. The amounts of the various actives in such combination therapy may be different amounts (different dosage amounts) or same amounts (same dosage amounts). Thus, for non-limiting illustration purposes, an Indazole Derivative and an additional therapeutic agent may be present in fixed amounts (dosage amounts) in a single dosage unit (*e.g.,* a capsule, a tablet).

In one embodiment, the at least one Indazole Derivative is administered during a time when the additional therapeutic agent(s) exert their prophylactic or therapeutic effect, or *vice versa.*

In another embodiment, the at least one Indazole Derivative and the additional therapeutic agent(s) are administered in doses commonly employed when such agents are used as monotherapy for treating a herpesvirus infection.

In another embodiment, the at least one Indazole Derivative and the additional therapeutic agent(s) are administered in doses lower than the doses commonly employed when such agents are used as monotherapy for treating a herpesvirus infection.

In still another embodiment, the at least one Indazole Derivative and the additional therapeutic agent(s) act synergistically and are administered in doses lower than the doses commonly employed when such agents are used as monotherapy for treating a herpesvirus infection.

In one embodiment, the at least one Indazole Derivative and the additional therapeutic agent(s) are present in the same composition. In one embodiment, this composition is suitable for oral administration. In another embodiment, this composition is suitable for intravenous administration. In another embodiment, this composition is suitable for subcutaneous administration. In still another embodiment, this composition is suitable for parenteral administration.

The at least one Indazole Derivative and the additional therapeutic agent(s) can act additively or synergistically. A synergistic combination may allow the use of lower dosages of one or more agents and/or less frequent administration of one or more agents of a combination therapy. A lower dosage or less frequent administration of one or more agents may lower toxicity of therapy without reducing the efficacy of therapy.

In one embodiment, the administration of at least one Indazole Derivative and the additional therapeutic agent(s) may inhibit the resistance of a herpesvirus infection to these agents.

The doses and dosage regimen of the other agents used in the combination therapies of the present invention for the treatment or prevention of herpesvirus infection can be determined by the attending clinician, taking into consideration the approved doses and dosage regimen in the package insert; the age, sex and general health of the patient; and the type and severity of the viral infection or related disease or disorder. When administered in combination, the Indazole Derivative(s) and the other agent(s) can be administered simultaneously (*i.e.,* in the same composition or in separate compositions one right after the other) or sequentially. This particularly useful when the components of the combination are given on different dosing schedules, e.g., one component is administered once daily and another component is administered every six hours, or when the preferred pharmaceutical compositions are different, *e.g.,* one is a tablet and one is a capsule. A kit comprising the separate dosage forms is therefore advantageous.

In one embodiment, one or more compounds of the present invention are administered with one or more additional therapeutic agents selected from: an immunomodulator, an anti-herpes agent, a viral replication inhibitor, an antisense agent, a therapeutic vaccine, a virion production inhibitor, a viral entry inhibitor, a viral assembly inhibitor, an antibody therapy (monoclonal or polyclonal), and any agent useful for treating any type of herpesvirus infection.

### Compositions and Administration

Due to their activity, the Indazole Derivatives are useful in veterinary and human medicine. As described above, the Indazole Derivatives are useful for treating or preventing herpesvirus infection in a patient in need thereof.

Accordingly, in one embodiment, the present invention provides pharmaceutical compositions comprising an effective amount of a compound of formula(I), or a pharmaceutically acceptable salt thereof, and a pharmaceutically acceptable carrier.

In another embodiment, the present invention provides pharmaceutical compositions comprising (i) an effective amount of a compound of formula(I), or a pharmaceutically acceptable salt thereof, and a pharmaceutically acceptable carrier; and (ii) one or more additional therapeutic agents, wherein said additional therapeutic agents are selected from anti-herpes agents and immunomodulators.

When administered to a patient, the Indazole Derivatives can be administered as a component of a composition that comprises a pharmaceutically acceptable carrier or vehicle. The present invention provides pharmaceutical compositions comprising an effective amount of at least one Indazole Derivative and a pharmaceutically acceptable carrier. In the pharmaceutical compositions and methods of the present invention, the active ingredients will typically be administered in admixture with suitable carrier materials suitably selected with respect to the intended form of administration, *i.e.,* oral tablets, capsules (either solid-filled, semi-solid filled or liquid filled), powders for constitution, oral gels, elixirs, dispersible granules, syrups, suspensions, and consistent with conventional pharmaceutical practices. For example, for oral administration in the form of tablets or capsules, the active drug component may be combined with any oral non-toxic pharmaceutically acceptable inert carrier, such as lactose, starch, sucrose, cellulose, magnesium stearate, dicalcium phosphate, calcium sulfate, talc, mannitol, ethyl alcohol (liquid forms). Solid form preparations include powders, tablets, dispersible granules, capsules, cachets and suppositories. Powders and tablets may be comprised of from about 0.5 to about 95 percent inventive composition. Tablets, powders, cachets and capsules can be used as solid dosage forms suitable for oral administration.

Moreover, when desired or needed, suitable binders, lubricants, disintegrating agents and coloring agents may also be incorporated in the mixture. Suitable binders include starch, gelatin, natural sugars, corn sweeteners, natural and synthetic gums such as acacia, sodium alginate, carboxymethylcellulose, polyethylene glycol and waxes. Among the lubricants there may be mentioned for use in these dosage forms, boric acid, sodium benzoate, sodium acetate, sodium chloride. Disintegrants include starch, methylcellulose, guar gum. Sweetening and flavoring agents and preservatives may also be included where appropriate.

Liquid form preparations include solutions, suspensions and emulsions and may include water or water-propylene glycol solutions for parenteral or intravenous injection.

Also included are solid form preparations which are intended to be converted, shortly before use, to liquid form preparations for either oral or parenteral administration. Such liquid forms include solutions, suspensions and emulsions.

For preparing suppositories, a low melting wax such as a mixture of fatty acid glycerides or cocoa butter is first melted, and the active ingredient is dispersed homogeneously therein as by stirring. The molten homogeneous mixture is then poured into convenient sized molds, allowed to cool and thereby solidify.

Additionally, the compositions of the present invention may be formulated in sustained release form to provide the rate-controlled release of any one or more of the components or active ingredients to optimize therapeutic effects, *i.e.,* antiviral activity. Suitable dosage forms for sustained release include layered tablets containing layers of varying disintegration rates or controlled release polymeric matrices impregnated with the active components and shaped in tablet form or capsules containing such impregnated or encapsulated porous polymeric matrices.

In one embodiment, the one or more Indazole Derivatives are administered orally.

In another embodiment, the one or more Indazole Derivatives are administered intravenously.

In still another embodiment, the one or more Indazole Derivatives are administered sublingually.

In one embodiment, a pharmaceutical preparation comprising at least one Indazole Derivative is in unit dosage form. In such form, the preparation is subdivided into unit doses containing effective amounts of the active components.

Compositions can be prepared according to conventional mixing, granulating or coating methods, respectively, and the present compositions can contain, in one embodiment, from about 0.1% to about 99% of the Indazole Derivative(s) by weight or volume. In various embodiments, the present compositions can contain, in one embodiment, from about 1% to about 70% or from about 5% to about 60% of the Indazole Derivative(s) by weight or volume.

The amount and frequency of administration of the Indazole Derivatives will be regulated according to the judgment of the attending clinician considering such factors as age, condition and size of the patient as well as severity of the symptoms being treated. Generally, a total daily dosage of the at least one Indazole Derivative(s) alone, or when administered as combination therapy, can range from about 1 to about 2500 mg per day, although variations will necessarily occur depending on the target of therapy, the patient and the route of administration. In one embodiment, the dosage is from about 10 to about 1000 mg/day, administered in a single dose or in 2-4 divided doses. In another embodiment, the dosage is from about 1 to about 500 mg/day, administered in a single dose or in 2-4 divided doses. In still another embodiment, the dosage is from about 1 to about 100 mg/day, administered in a single dose or in 2-4 divided doses. In yet another embodiment, the dosage is from about 1 to about 50 mg/day, administered in a single dose or in 2-4 divided doses. In another embodiment, the dosage is from about 500 to about 1500 mg/day, administered in a single dose or in 2-4 divided doses. In still another embodiment, the dosage is from about 500 to about 1000 mg/day, administered in a single dose or in 2-4 divided doses. In yet another embodiment, the dosage is from about 100 to about 500 mg/day, administered in a single dose or in 2-4 divided doses.

The compositions of the invention can further comprise one or more additional therapeutic agents, selected from those listed above herein. Accordingly, in one embodiment, the present invention provides compositions comprising: (i) at least one Indazole Derivative or a pharmaceutically acceptable salt thereof; (ii) one or more additional therapeutic agents that are not an Indazole Derivative; and (iii) a pharmaceutically acceptable carrier, wherein the amounts in the composition are together effective to treat herpesvirus infection.

In one embodiment, the present invention provides compositions comprising a Compound of Formula (I) and a pharmaceutically acceptable carrier.

In another embodiment, the present invention provides compositions comprising a Compound of Formula (I), a pharmaceutically acceptable carrier, and a second therapeutic agent selected from the group consisting of anti-herpes agents and immunomodulators.

In another embodiment, the present invention provides compositions comprising a Compound of Formula (I), a pharmaceutically acceptable carrier, and two additional therapeutic agents, each of which are independently selected from the group consisting of anti-herpes agents and immunomodulators.

### Kits (not part of the claimed invention)

Also described is a kit comprising a therapeutically effective amount of at least one Indazole Derivative, or a pharmaceutically acceptable salt, solvate of said compound and a pharmaceutically acceptable carrier, vehicle or diluent.

Also described is a kit comprising an amount of at least one Indazole Derivative, or a pharmaceutically acceptable salt, solvate of said compound and an amount of at least one additional therapeutic agent listed above, wherein the amounts of the two or more active ingredients result in a desired therapeutic effect. In one embodiment, the one or more Indazole Derivatives and the one or more additional therapeutic agents are provided in the same container. In one embodiment, the one or more Indazole Derivatives and the one or more additional therapeutic agents are provided in separate containers.

## Claims

1. A compound having the formula (I): 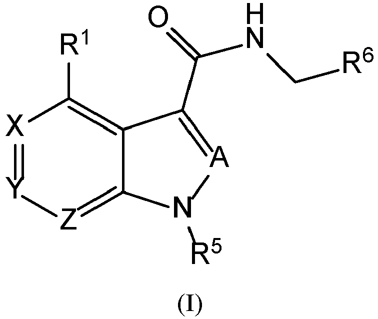 or a pharmaceutically acceptable salt thereof,
wherein:
A is -N- or -C(R⁸)-;
X is -N- or -C(R²)-;
Y is -C(R³)-;
Z is -N- or -C(R⁴)-, such that only one of X and Z can be -N-;
R¹ is selected from H or methyl;
R² is selected from H, 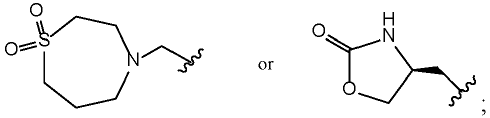
R³ is selected from 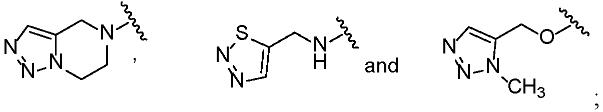
R⁴ is selected from H, -Cl or -CN;
R⁵ is selected from H, C₃-C₇ cycloalkyl, -OH, 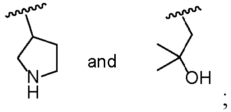
R⁶ is selected from 4-chlorophenyl and 4-cyanophenyl; and
R⁸ is H or C₁-C₆ alkyl.

2. The compound of claim 1, wherein A is -N-.

3. The compound of claim 1, wherein A is -C(R⁸)-.

4. The compound of any of claims 1-3, wherein X is -N-.

5. The compound of any of claims 1-3, wherein Z is -N-.

6. The compound of any of claims 1-3, or 5, wherein R² is:

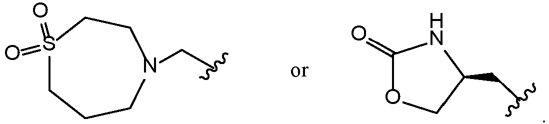

7. The compound of any of claims 1-4, or 6, wherein R⁴ is -Cl or -CN.

8. A compound, that is selected from any of the compounds numbered 1-8 and 10-34:
| Compound Number | Structure |
|---|---|
| 1 | 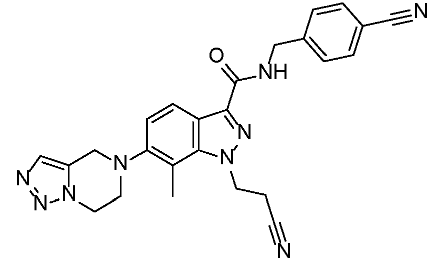 |
| 4 | 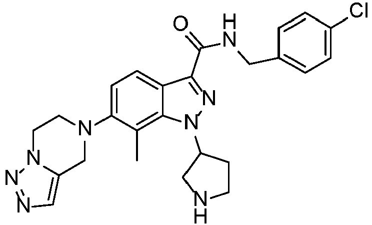 |
| 5 | 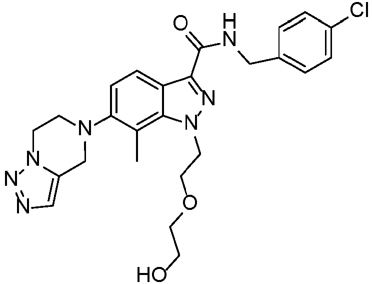 |
| 6 | 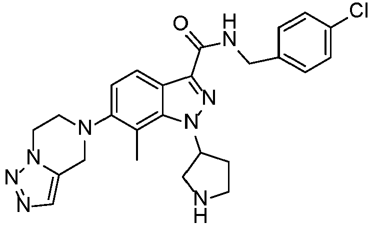 |
| 14 | 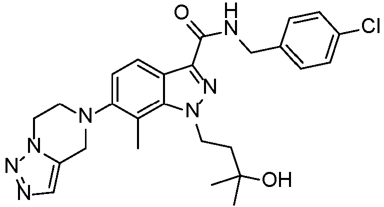 |
| 27 | 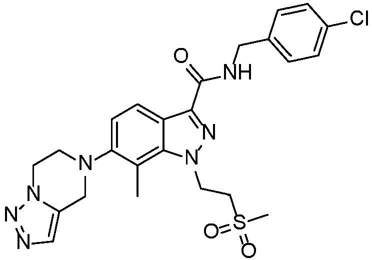 |
| 32 | 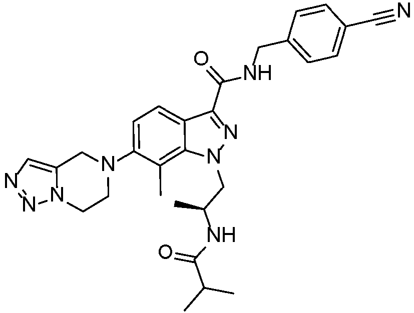 |
| 2 | 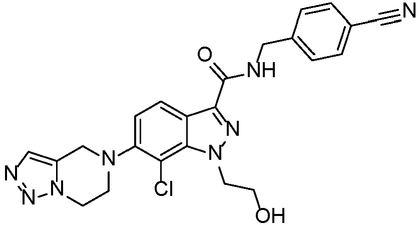 |
| 3 | 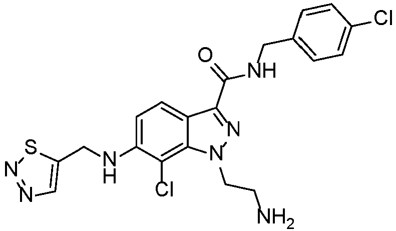 |
| 7 | 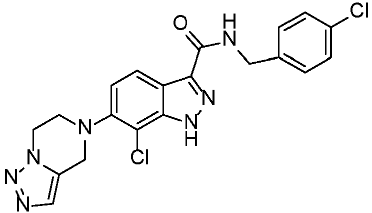 |
| 8 | 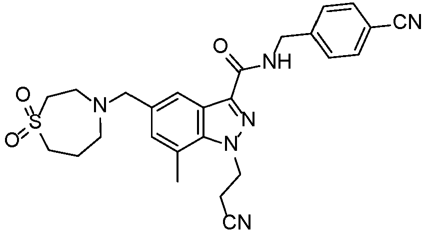 |
| 10 | 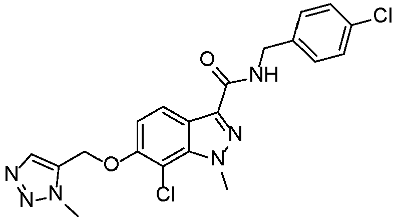 |
| 11 | 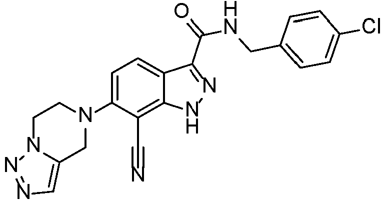 |
| 12 | 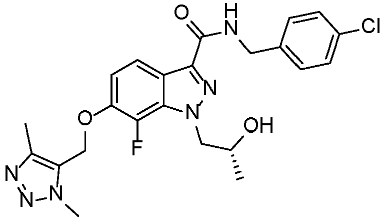 |
| 13 | 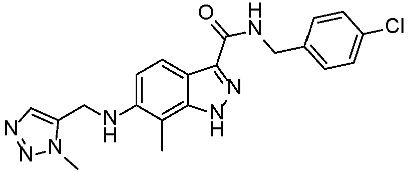 |
| 15 | 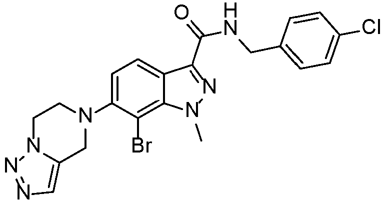 |
| 16 | 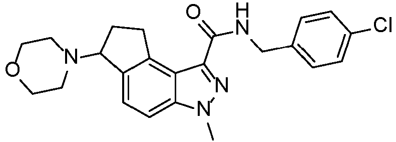 |
| 17 | 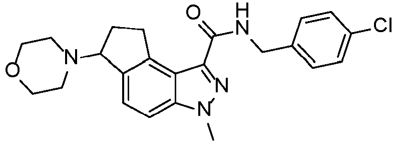 |
| 18 | 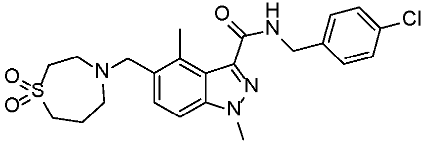 |
| 19 | 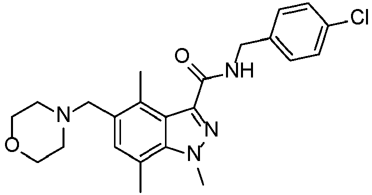 |
| 20 | 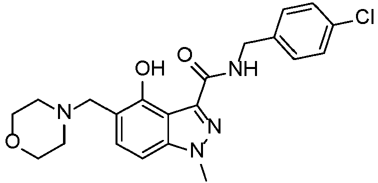 |
| 21 | 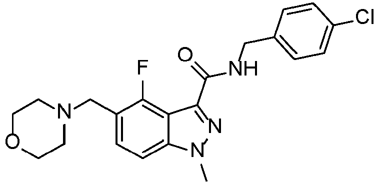 |
| 22 | 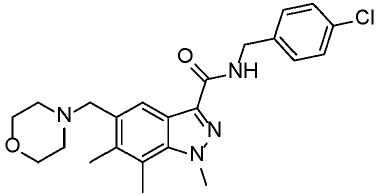 |
| 28 | 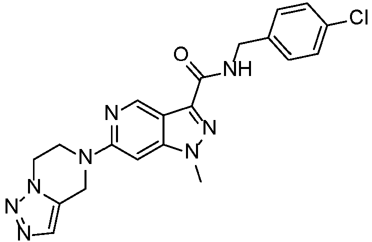 |
| 29 | 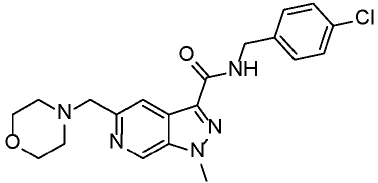 |
| 30 | 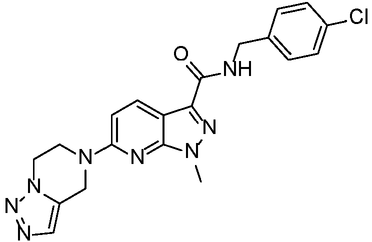 |
| 31 | 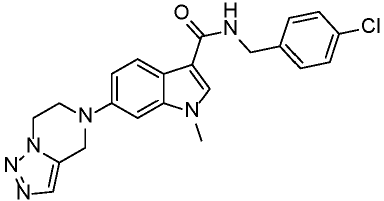 |
| 33 | 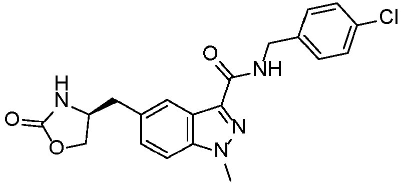 |
| 34 | 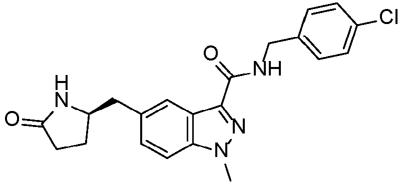 |
| 23 | 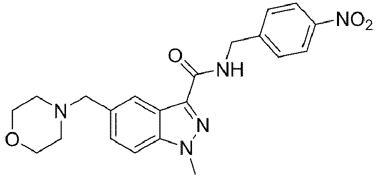 |
| 24 | 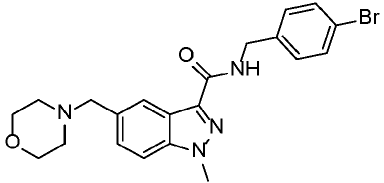 |
| 25 | 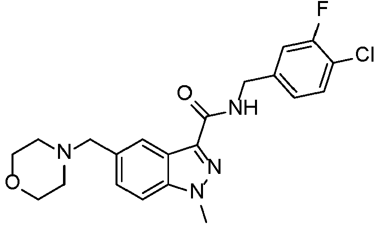 |
| 26 | 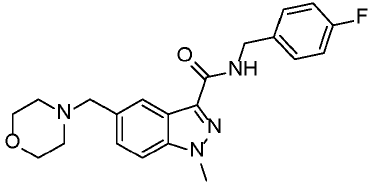 |
or a pharmaceutically acceptable salt thereof.

9. A pharmaceutical composition comprising the compound of any of claims 1-8, or a pharmaceutically acceptable salt thereof, and a pharmaceutically acceptable carrier.

10. The pharmaceutical composition according to claim 9, further comprising one or more additional therapeutic agents, wherein said additional therapeutic agents are selected from anti-herpes agents, and immunomodulators.

11. A compound according to any of claims 1-8, or a pharmaceutically acceptable salt thereof, for use in treating herpesvirus infection.

12. A combination comprising a compound according to any of claims 1-8, or a pharmaceutically acceptable salt thereof and one or more additional therapeutic agents selected from anti-herpes agents and immunomodulators, for use in treating or preventing herpesvirus infection.

13. The compound according to any of claims 1-8, or a pharmaceutically acceptable salt thereof for use in therapy.

## Patentansprüche

1. Eine Verbindung der Formel (I) 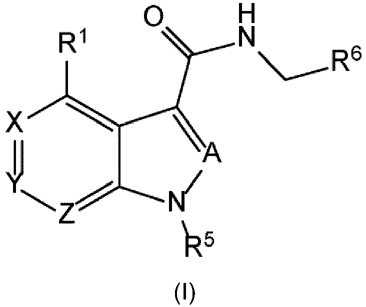 oder ein pharmazeutisch annehmbares Salz davon,
wobei:
A -N- oder -C(R⁸)- ist,
X -N- oder -C(R²)- ist,
Y -C(R³)- ist,
Z -N- oder -C(R⁴)- ist, so dass nur einer der Reste X und Z -N- sein kann,
R¹ ausgewählt ist aus H oder Methyl,
R² ausgewählt ist aus H, 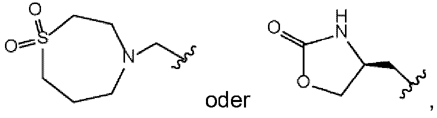
R³ ausgewählt ist aus 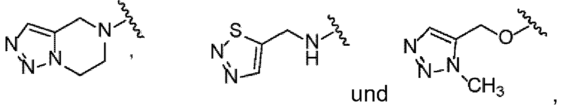
R⁴ ausgewählt ist aus H, -Cl oder -CN,
R⁵ ausgewählt ist aus H, C₃-C₇-Cycloalkyl, -OH, 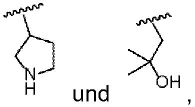
R⁶ ausgewählt ist aus 4-Chlorphenyl und 4-Cyanophenyl, und
R⁸ H oder C₁-C₆-Alkyl ist.

2. Die Verbindung nach Anspruch 1, wobei A -N- ist.

3. Die Verbindung nach Anspruch 1, wobei A -C(R⁸)- ist.

4. Die Verbindung nach einem der Ansprüche 1 - 3, wobei X -N- ist.

5. Die Verbindung nach einem der Ansprüche 1 - 3, wobei Z -N- ist.

6. Die Verbindung nach einem der Ansprüche 1 - 3 oder 5, wobei R² ist:

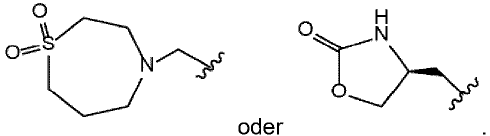

7. Die Verbindung nach einem der Ansprüche 1 - 4 oder 6, wobei R⁴ -Cl oder -CN ist.

8. Eine Verbindung, die ausgewählt ist aus einer der Verbindungen mit den Nummern 1 - 8 und 10 - 34:
| Verbindung Nummer | Struktur |
|---|---|
| 1 | 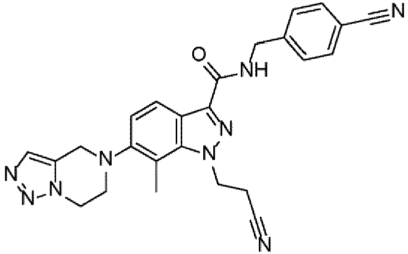 |
| 4 | 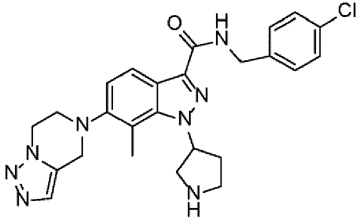 |
| 5 | 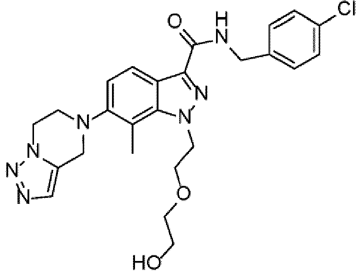 |
| 6 | 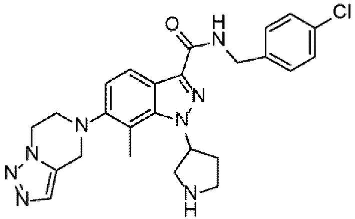 |
| 14 | 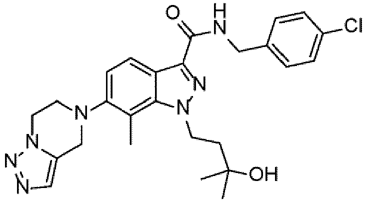 |
| 27 | 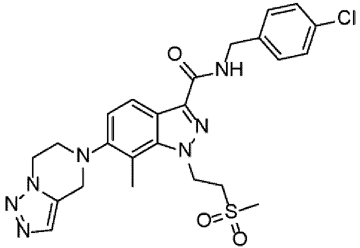 |
| 32 | 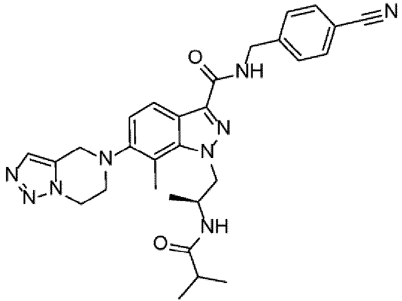 |
| 2 | 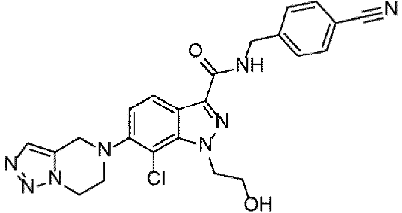 |
| 3 | 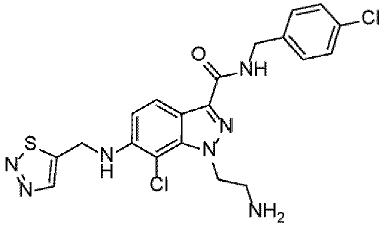 |
| 7 | 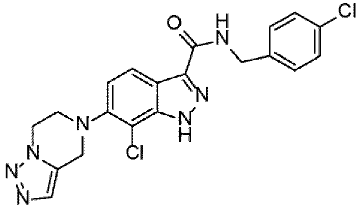 |
| 8 | 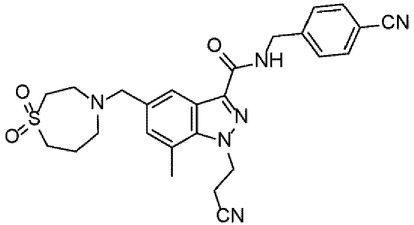 |
| 10 | 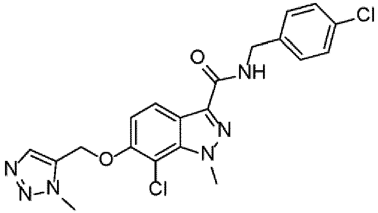 |
| 11 | 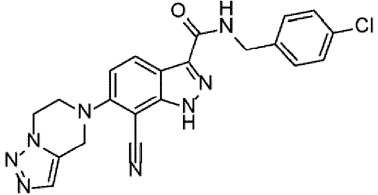 |
| 12 | 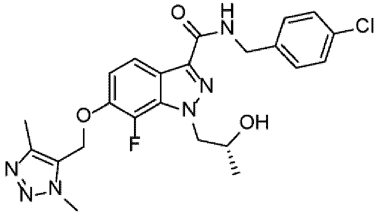 |
| 13 | 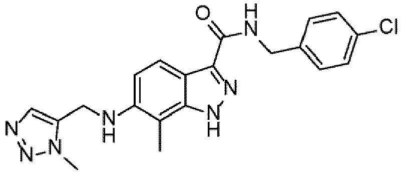 |
| 15 | 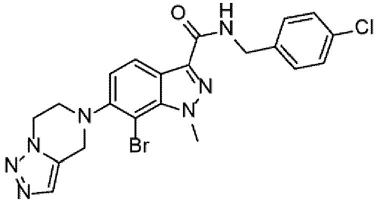 |
| 16 | 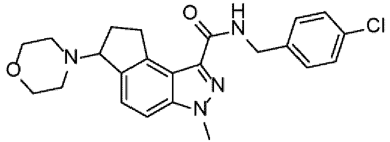 |
| 17 | 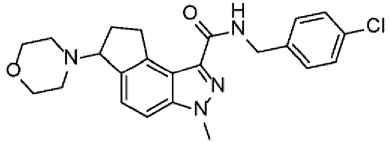 |
| 18 | 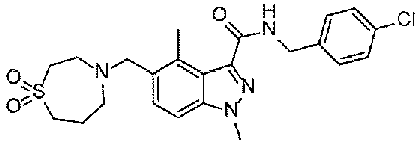 |
| 19 | 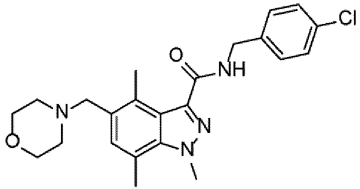 |
| 20 | 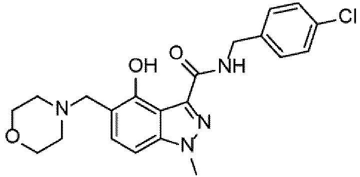 |
| 21 | 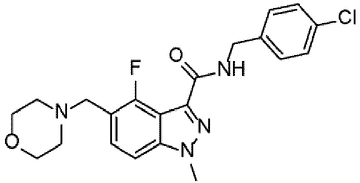 |
| 22 | 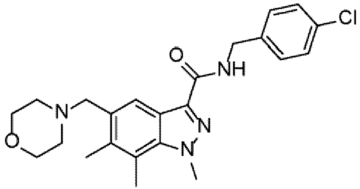 |
| 28 | 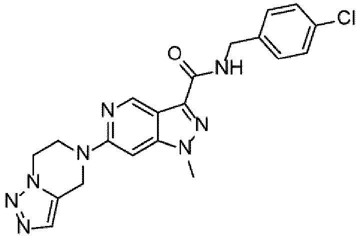 |
| 29 | 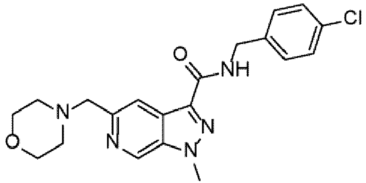 |
| 30 | 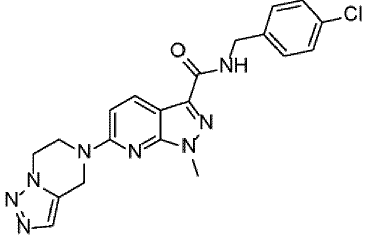 |
| 31 | 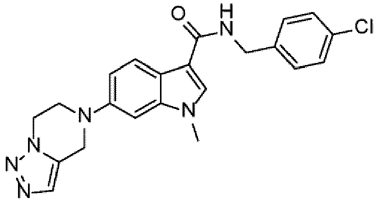 |
| 33 | 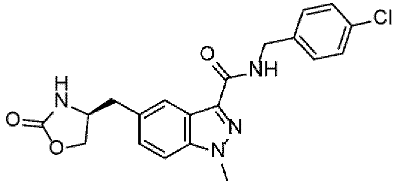 |
| 34 | 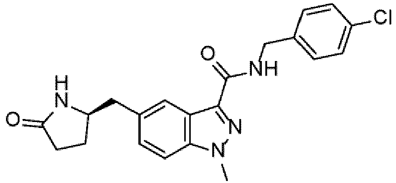 |
| 23 | 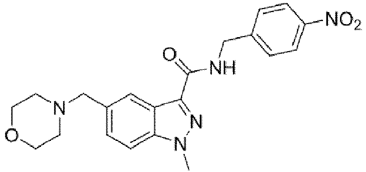 |
| 24 | 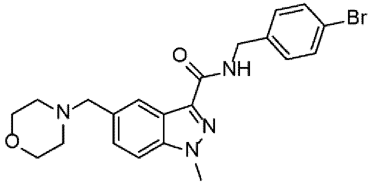 |
| 25 | 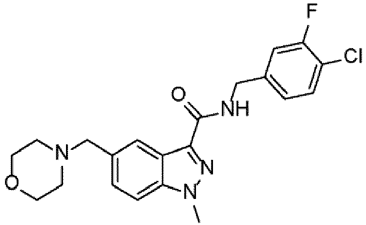 |
| 26 | 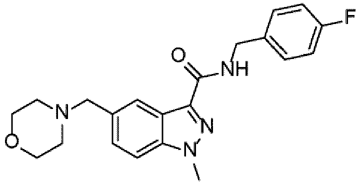 |
oder ein pharmazeutisch annehmbares Salz davon.

9. Eine pharmazeutische Zusammensetzung, die die Verbindung nach einem der Ansprüche 1 - 8 oder ein pharmazeutisch annehmbares Salz davon und einen pharmazeutisch annehmbaren Träger umfasst.

10. Die pharmazeutische Zusammensetzung nach Anspruch 9, ferner umfassend ein oder mehrere zusätzliche therapeutische Mittel, wobei die zusätzlichen therapeutischen Mittel ausgewählt sind aus Mitteln gegen Herpes und Immunmodulatoren.

11. Eine Verbindung nach einem der Ansprüche 1 - 8 oder ein pharmazeutisch annehmbares Salz davon zur Verwendung bei der Behandlung einer Herpesvirusinfektion.

12. Eine Kombination, die eine Verbindung nach einem der Ansprüche 1 - 8 oder ein pharmazeutisch annehmbares Salz davon und ein oder mehrere zusätzliche therapeutische Mittel, ausgewählt aus Mitteln gegen Herpes und Immunmodulatoren, umfasst, zur Verwendung bei der Behandlung oder Prävention einer Herpesvirusinfektion.

13. Die Verbindung nach einem der Ansprüche 1 - 8 oder ein pharmazeutisch annehmbares Salz davon zur Verwendung in der Therapie.

## Revendications

1. Composé ayant la formule (I) : 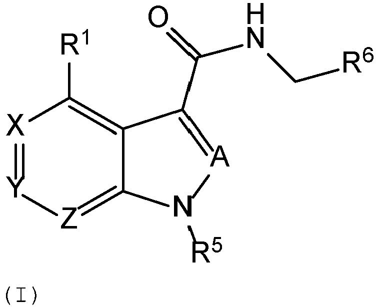 ou sel pharmaceutiquement acceptable correspondant,
dans laquelle :
A est -N- ou -C(R⁸)- ;
X est -N- ou -C(R²)- ;
Y est -C(R³)- ;
Z est -N- ou -C(R⁴)-, tel qu'un seul parmi X et Z peut être -N- ;
R¹ est choisi parmi H ou méthyle ;
R² est choisi parmi H, 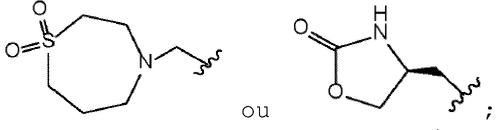
R³ est choisi parmi 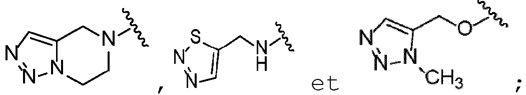
R⁴ est choisi parmi H, -Cl ou -CN ;
R⁵ est choisi parmi H, C₃-C₇ cycloalkyle, -OH, 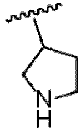 et 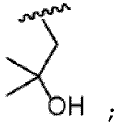
R⁶ est choisi parmi 4-chlorophényle et 4-cyanophényle ; et
R⁸ est H ou C₁-C₆ alkyle.

2. Composé selon la revendication 1, dans lequel A est -N-.

3. Composé selon la revendication 1, dans lequel A est -C(R⁸)-.

4. Composé selon l'une quelconque des revendications 1 à 3, dans lequel X est -N-.

5. Composé selon l'une quelconque des revendications 1 à 3, dans lequel Z est -N-.

6. Composé selon l'une quelconque des revendications 1 à 3, ou 5, dans lequel R² est :

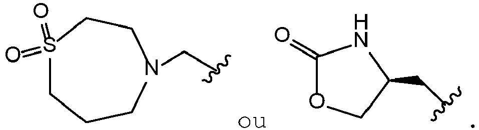

7. Composé selon l'une quelconque des revendications 1 à 4, ou 6, dans lequel R⁴ est -Cl ou -CN.

8. Composé, qui est choisi parmi l'un quelconque des composés numérotés 1-8 et 10-34 :
| Numéro de composé | Structure |
|---|---|
| 1 | 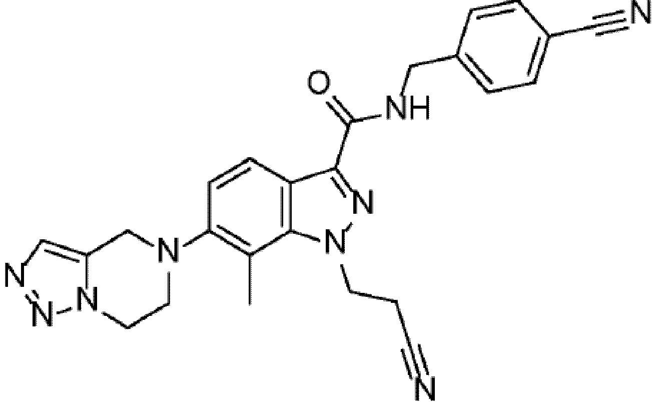 |
| 4 | 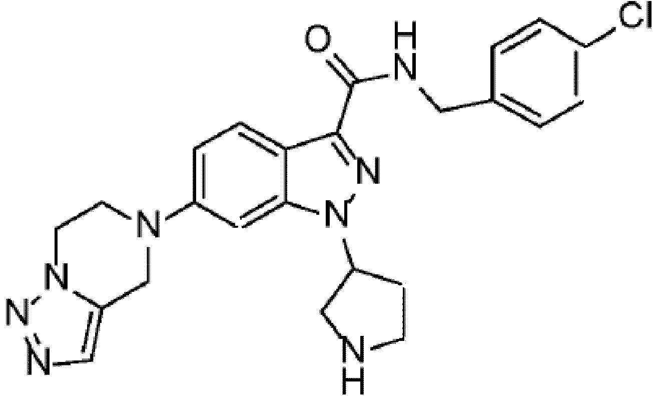 |
| 5 | 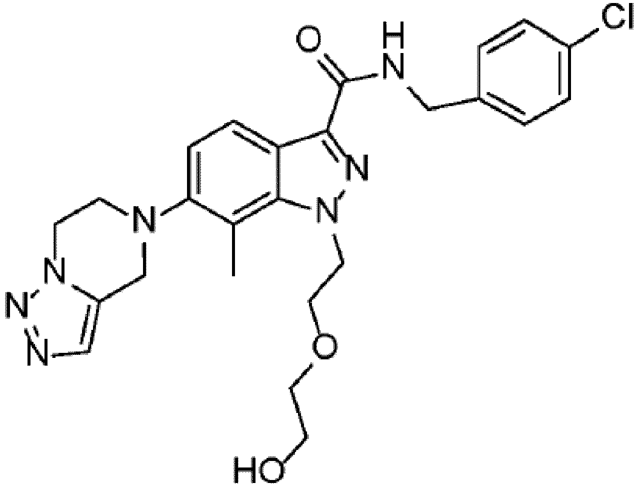 |
| 6 | 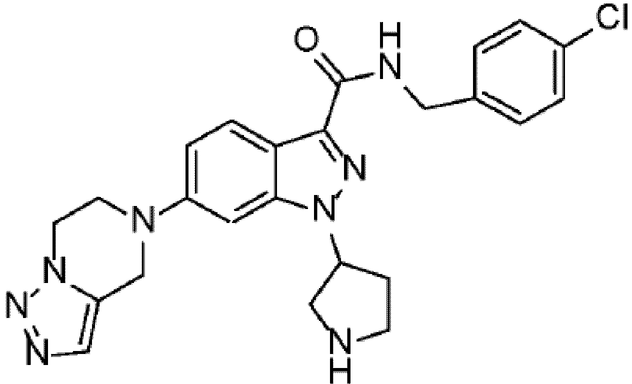 |
| 14 | 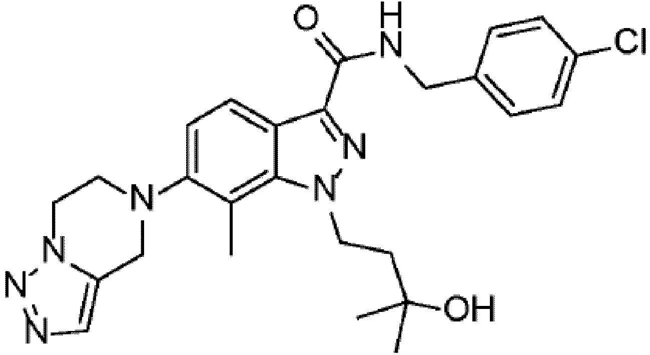 |
| 27 | 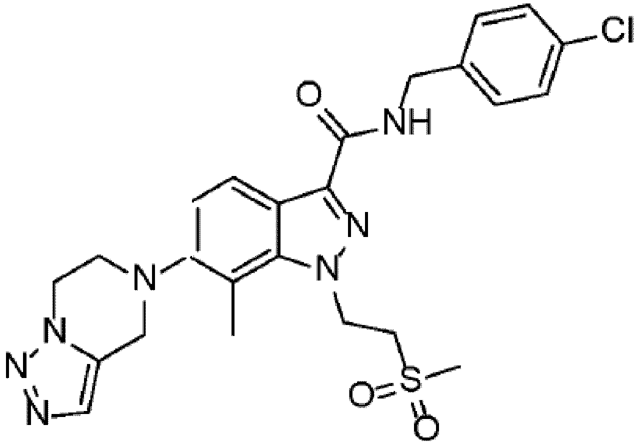 |
| 32 | 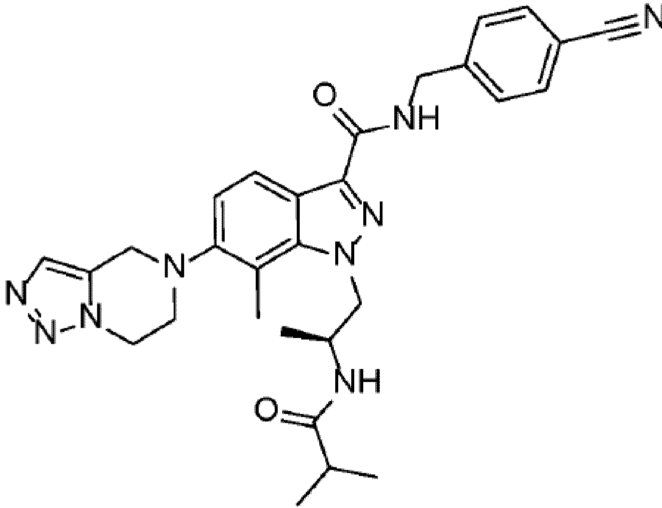 |
| 2 | 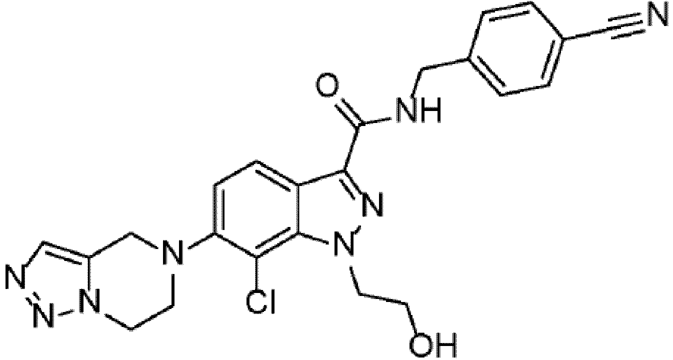 |
| 3 | 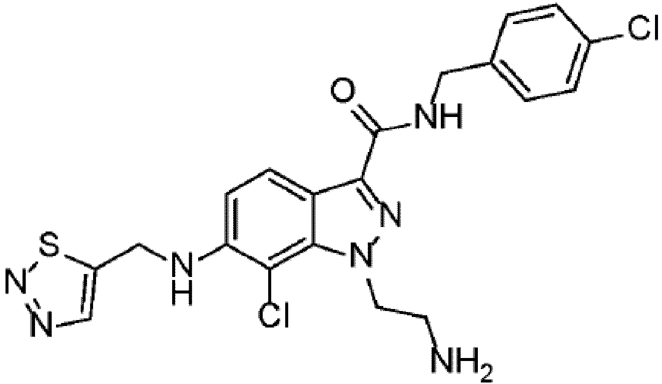 |
| 7 | 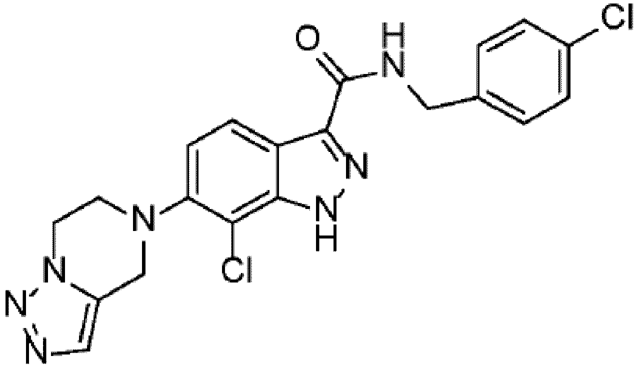 |
| 8 | 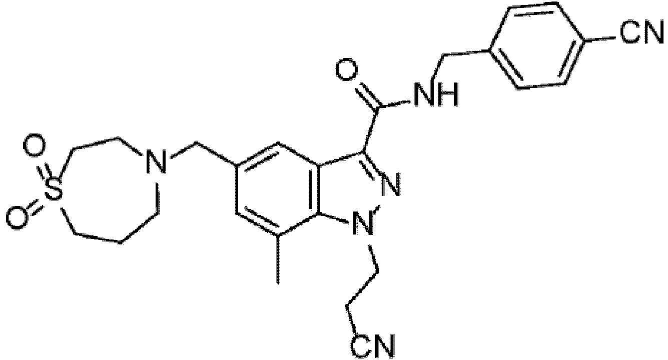 |
| 10 | 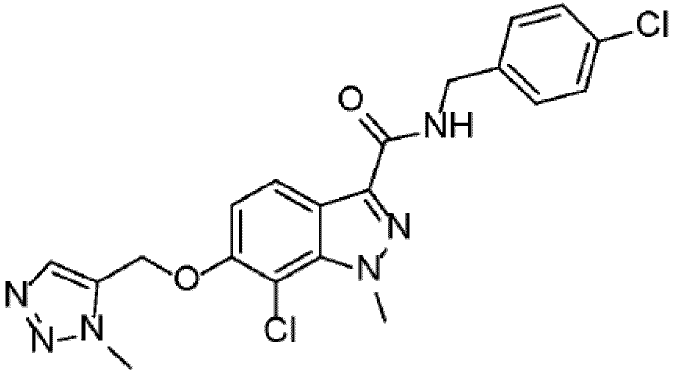 |
| 11 | 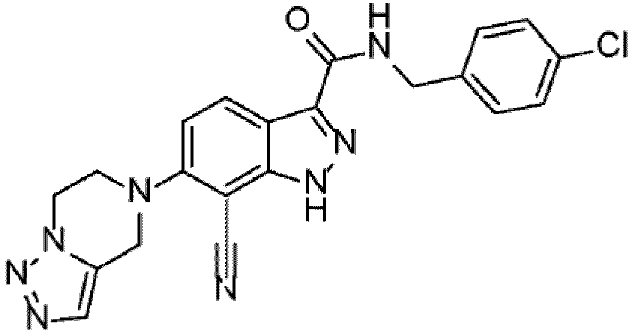 |
| 12 | 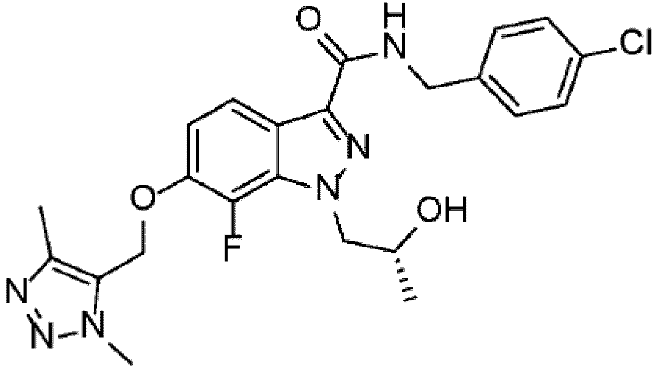 |
| 13 | 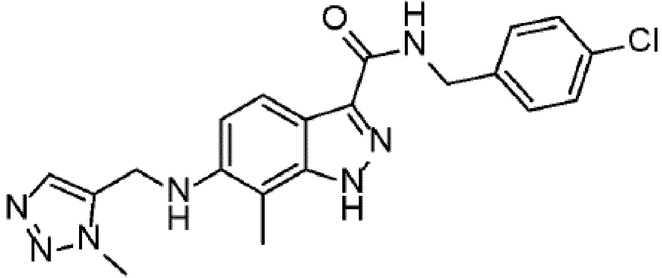 |
| 15 | 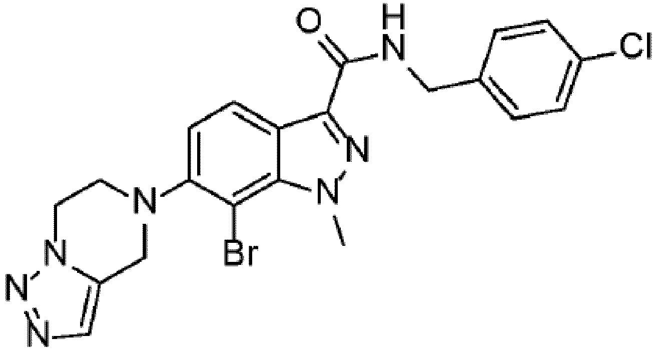 |
| 16 | 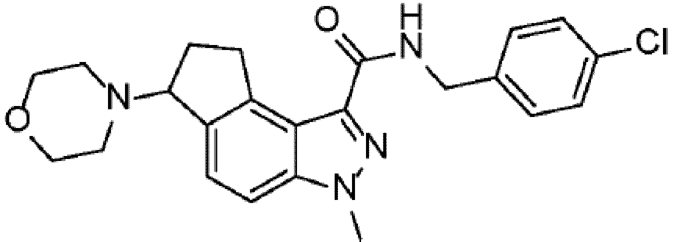 |
| 17 | 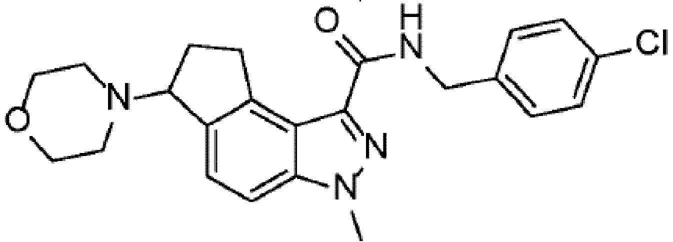 |
| 18 | 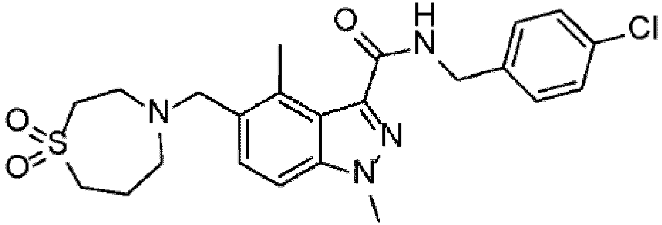 |
| 19 | 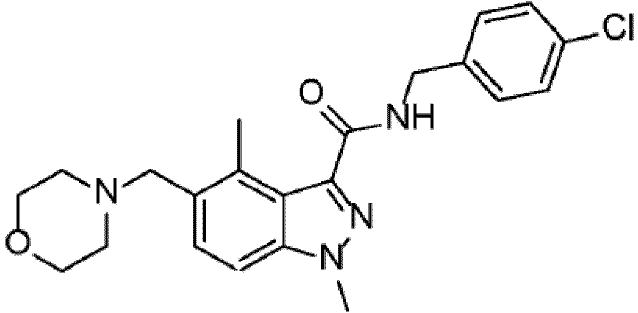 |
| 20 | 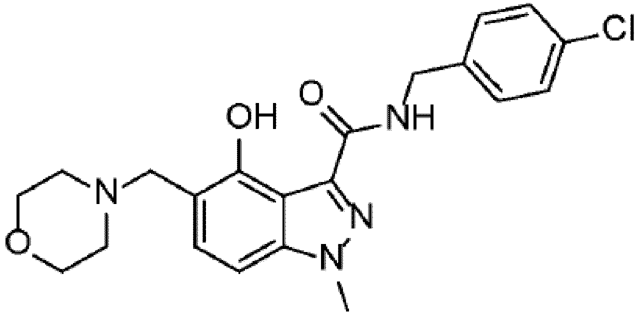 |
| 21 | 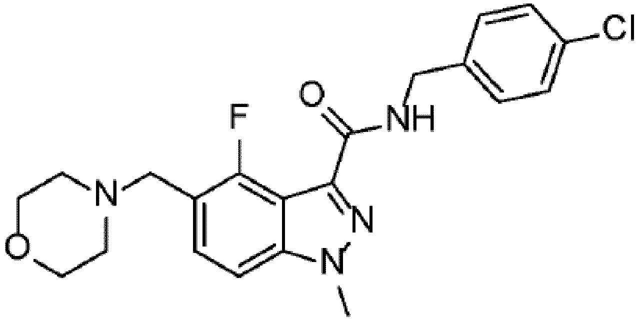 |
| 22 | 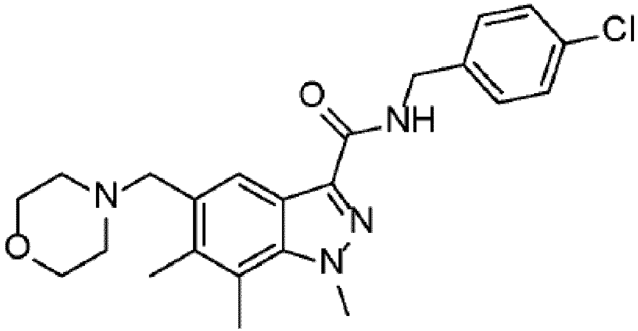 |
| 28 | 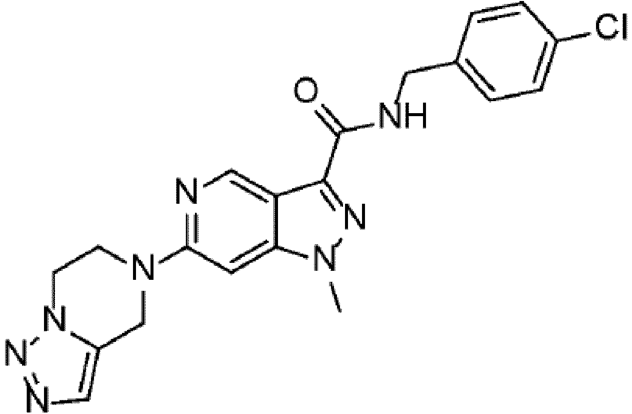 |
| 29 | 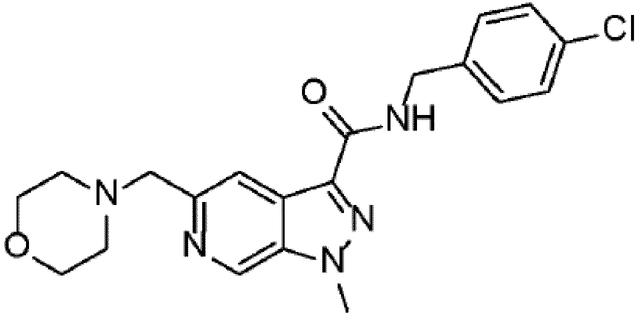 |
| 30 | 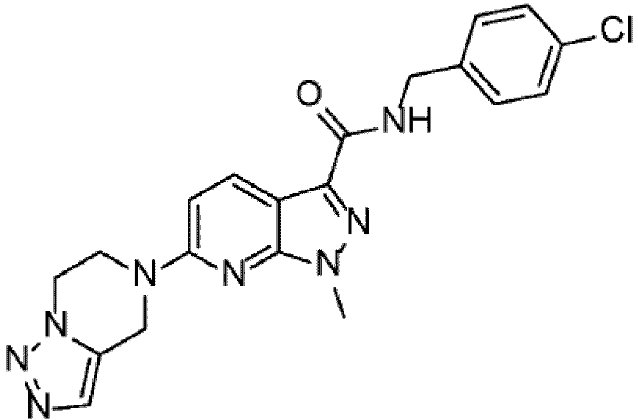 |
| 31 | 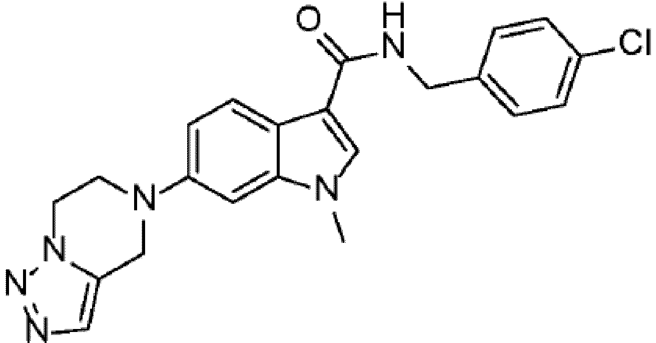 |
| 33 | 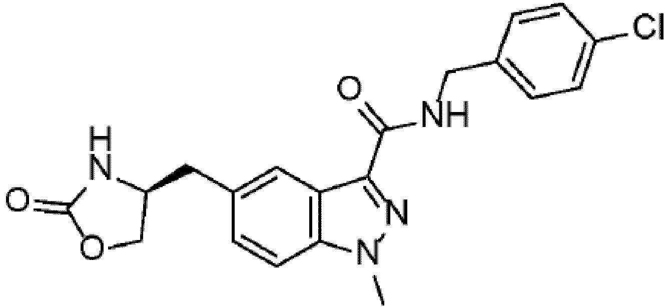 |
| 34 | 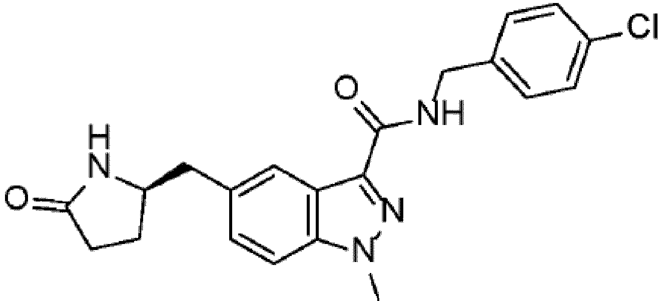 |
| 23 | 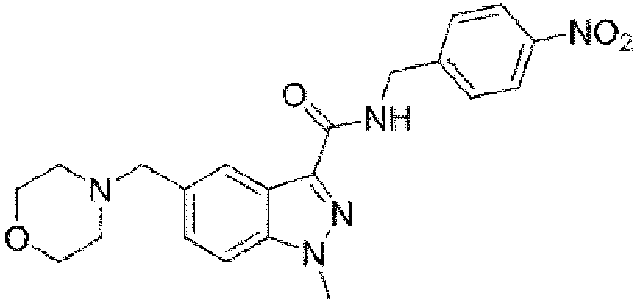 |
| 24 | 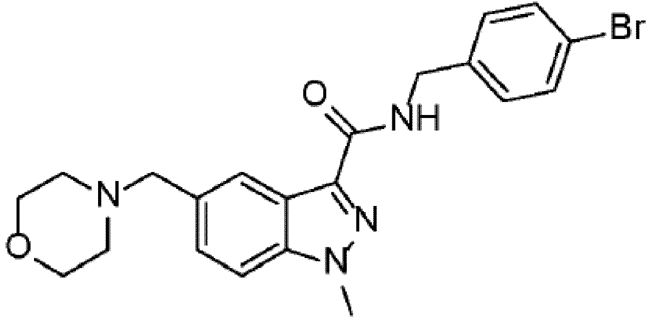 |
| 25 | 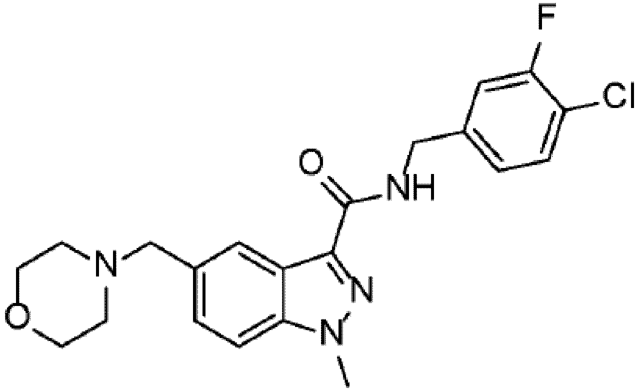 |
| 26 | 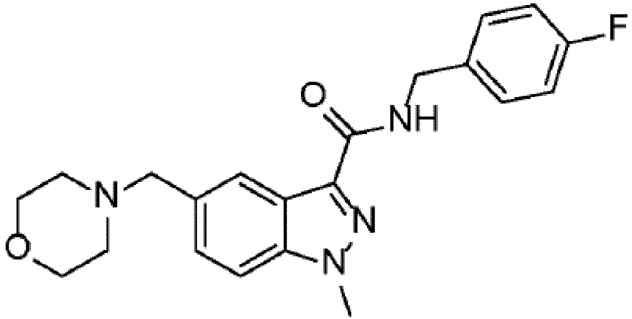 |
ou sel pharmaceutiquement acceptable de celui-ci.

9. Composition pharmaceutique comprenant le composé selon l'une quelconque des revendications 1 à 8, ou un sel pharmaceutiquement acceptable correspondant, et un véhicule pharmaceutiquement acceptable.

10. Composition pharmaceutique selon la revendication 9, comprenant en outre un ou plusieurs agents thérapeutiques supplémentaires, dans laquelle lesdits agents thérapeutiques supplémentaires sont choisis parmi les agents anti-herpès et les immunomodulateurs.

11. Composé selon l'une quelconque des revendications 1 à 8, ou sel pharmaceutiquement acceptable de celui-ci, pour une utilisation dans un traitement d'une infection par un virus de l'herpès.

12. Combinaison comprenant un composé selon l'une quelconque des revendications 1 à 8, ou un sel pharmaceutiquement acceptable de celui-ci et un ou plusieurs agents thérapeutiques supplémentaires choisis parmi les agents anti-herpès et les immunomodulateurs, pour une utilisation dans le traitement ou la prévention d'une infection par le virus de l'herpès.

13. Composé selon l'une quelconque des revendications 1 à 8, ou sel pharmaceutiquement acceptable de celui-ci pour une utilisation en thérapie.
